# EUROPEAN PATENT APPLICATION

(11) **EP 4 722 249 A1**
(43) Date of publication of application: **08.04.2026**
(21) Application number: 24815906.3
(22) Date of filing: 31.05.2024
(51) Int. Cl.: C07K 16/28, A61K 39/00

(54) **ANTI-CD200R1 ANTIBODY AND USE THEREOF**

(30) Priority: 02.06.2023 KR 20230071439
(71) Applicant: AIMED BIO INC., Seoul, 05835 (KR)
(72) Inventor: KIM, Hye-Jin, Seoul 05835 (KR); MIN, Byeongkwi, Seoul 05835 (KR); KIM, Jongbin, Seoul 05835 (KR); LEE, Sunghyun, Seoul 05835 (KR); CHO, Minjung, Seoul 05835 (KR); KANG, Sunghyun, Seoul 05835 (KR); YOO, Minyoung, Seoul 05835 (KR)
(74) Representative: Mewburn Ellis LLP
(86) International application number: PCT/KR2024/007469
(87) International publication number: WO 2024/248532

(57) **Abstract**

The present invention relates to an anti-CD200R1 antibody or an antigen-binding fragment thereof; a nucleic acid encoding same; an expression vector comprising the nucleic acid; a host cell having the nucleic acid or the expression vector introduced thereinto; a method for preparing the antibody or an antigen-binding fragment thereof; a bi- or multi-specific antibody comprising the antibody or an antigen-binding fragment thereof; a chimeric antigen receptor (CAR) comprising the antibody or an antigen-binding fragment thereof; an immune cell expressing the antibody or an antigen-binding fragment thereof or a chimeric antigen receptor comprising same; an immunosuppressive composition comprising the antibody or an antigen-binding fragment thereof, and a pharmaceutical use.

## Description

### [Technical Field]

The present invention relates to an anti-CD200R1 antibody or an antigen-binding fragment thereof, a nucleic acid encoding the same, an expression vector containing the nucleic acid, a host cell having the nucleic acid or the expression vector introduced thereinto, a method of preparing the antibody or antigen-binding fragment thereof, a bispecific or multispecific antibody including the antibody or antigen-binding fragment thereof, a chimeric antigen receptor (CAR) including the antibody or antigen-binding fragment thereof, an immune cell expressing the antibody or antigen-binding fragment thereof or a chimeric antigen receptor containing the same, an immunosuppressive composition containing the antibody or antigen-binding fragment thereof, and a pharmaceutical use thereof.

### [Background Art]

CD200R1 is known to be a receptor that can bind precisely due to its structural similarity to the CD200 protein which is one of the type 1 cell membrane glycoproteins. CD200R, which is a member of the Ig superfamily (IgSF), is a checkpoint receptor that suppresses immune cell activation and is composed of two IgSF domains, one transmembrane domain, and a cytoplasmic domain. CD200R1 and CD200R2 are two known isoforms of human CD200R, and CD200R1, CD200R2, CD200R3, and CD200R4 are four known isoforms of mouse CD200R. *In vivo,* CD200 ligand binds only to the N-terminal Ig V-type domain of CD200R1 and induces a significant immunosuppressive response (Int J Physiol Pahtophysiol Pharmacol 2019;11(6):297-309). In particular, CD200R1 is known to be more effective in signaling than other CD200R isoforms due to the long cytoplasmic domain thereof.

When CD200R1 is activated, phosphorylation of the Dok2 adaptor protein occurs, inducing an intracellular signaling cascade that suppresses target cell proliferation and immune responses. CD200R1 is known to be highly expressed in myeloid-derived mononuclear cells, such as macrophages, dendritic cells, and mast cells. Furthermore, CD200R1 is also expressed in large amounts in immune cells, such as eosinophils and neutrophils, which induce innate immunity. Therefore, CD200R1 has potential as a therapeutic agent for diseases such as atopic dermatitis with allergic pathology and sinusitis with nasal polyps. Furthermore, analysis of human genome data has reported a direct link between the CD200R1 and Dok2 protein genes and the heritability of atopic dermatitis (J Allergy Clin Immunol, Volume 145, Number 4). Furthermore, the CD200R1 pathway may serve as a potential target for neuroinflammatory diseases such as multiple sclerosis, stroke, and Parkinson's disease.

Anti-CD200R1 antibodies targeting CD200R1 are divided into agonists, which suppress immune responses, and antagonists, which activate immune responses. First, as the anti-CD200R1 signaling antagonist, 23ME-00610 (23AndMe) monoclonal antibody has reached Phase 1/2 clinical trials, demonstrating its efficacy in various cancers, including clear cell renal cell carcinoma, epithelial ovarian cancer, fallopian tube cancer, primary peritoneal cancer, and neuroendocrine tumors (Clinicaltrials, NCT05199272). Drug development is underway based on a mechanism of action that maximizes the anticancer effect of T cells by blocking signal transduction that suppresses the immune response.

Meanwhile, anti-CD200R1 agonists can enhance the immunosuppressive effects of CD200, which are present in conventional tissues in the body. WO 2015/057906 discloses that the CD200R agonist antibody (H2RM147, Janssen) competitively binds to human CD200. The CD200R agonist antibody (I-4P, Eli Lilly), developed to overcome this limitation, is reported to bind to a different domain of the CD200R1 protein. Therefore, each antibody has its own limitations.

Against this technical background, as a result of extensive efforts to overcome the limitations of conventional CD200R1 agonist antibodies and develop anti-CD200R1 agonist antibodies with superior efficacy, the present inventors developed an antibody that binds to the same domain as human CD200 while non-competitively binding to the same using various unique screening technologies owned by the applicant, and found that the antibody exhibits superior immune suppression and inflammatory response suppression effects than the conventional CD200R1 antibody. Based on this finding, the present invention has been completed.

### [Disclosure]

Therefore, it is one object of the present invention to provide a novel antibody specifically binding to CD200R1 (CD200 receptor 1) or an antigen-binding fragment thereof and a use thereof.

It is another object of the present invention to provide a nucleic acid encoding the antibody or antigen-binding fragment thereof.

It is another object of the present invention to provide a recombinant expression vector including the nucleic acid, and a cell into which the nucleic acid or expression vector is introduced.

It is another object of the present invention to provide a method of preparing an antibody specifically binding to CD200R1 or an antigen-binding fragment thereof.

It is another object of the present invention to provide a bi- or multi-specific antibody including the antibody or an antigen-binding fragment thereof.

It is another object of the present invention to provide a chimeric antigen receptor (CAR) comprising the scFv of the antibody as an antigen-binding domain of an extracellular domain, an immune cell into which the CAR is introduced, and a use thereof.

It is another object of the present invention to provide an immunosuppressive composition containing the antibody or antigen-binding fragment thereof, the bispecific or multispecific antibody, the chimeric antigen receptor, or the immune cell.

It is another object of the present invention to provide a composition for use in preventing or treating immune diseases or inflammatory diseases containing the antibody or antigen-binding fragment thereof, the bispecific or multispecific antibody, the chimeric antigen receptor, or the immune cell.

In accordance with an aspect of the present invention, the above and other objects can be accomplished by the provision of an antibody specifically binding to CD200R1 or an antigen-binding fragment thereof including:
a heavy chain CDR1 including at least one amino acid sequence selected from the group consisting of SEQ ID NOs: 1 to 8, SEQ ID NOs: 100 to 103, and SEQ ID NO: 158;
a heavy chain CDR2 including at least one amino acid sequence selected from the group consisting of SEQ ID NOs: 9 to 21, SEQ ID NOs: 104 to 109, and SEQ ID NO: 159;
a heavy chain CDR3 including at least one amino acid sequence selected from the group consisting of SEQ ID NOs: 22 to 35, SEQ ID NOs: 110 to 115, SEQ ID NO: 160, and SEQ ID NOs: 172 and 173;
a light chain CDR1 including at least one amino acid sequence selected from the group consisting of SEQ ID NOs: 36 to 47, SEQ ID NOs: 116 to 122, SEQ ID NO: 161, SEQ ID NO: 192, and SEQ ID NO: 193;
a light chain CDR2 including at least one amino acid sequence selected from the group consisting of SEQ ID NOs: 48 to 58, SEQ ID NOs: 123 to 127, SEQ ID NO: 162, and SEQ ID NO: 194; and
a light chain CDR3 including at least one amino acid sequence selected from the group consisting of SEQ ID NOs: 59 to 70, SEQ ID NOs: 128 to 134, SEQ ID NO: 163, SEQ ID NOs: 174 to 183, SEQ ID NO: 195, and SEQ ID NO: 196.

In accordance with another aspect of the present invention, there is provided a nucleic acid encoding the antibody or an antigen-binding fragment thereof.

In accordance with another aspect of the present invention, there is provided a recombinant expression vector containing the nucleic acid.

In accordance with another aspect of the present invention, there is provided a host cell transfected with the recombinant expression vector.

In accordance with another aspect of the present invention, there is provided a method of producing an antibody specifically binding to CD200R1 or an antigen-binding fragment thereof, the method including culturing a host cell to produce an antibody or an antigen-binding fragment thereof, and isolating and purifying the produced antibody or fragment.

In accordance with another aspect of the present invention, there is provided a bispecific/multispecific antibody including the antibody or antigen-binding fragment thereof.

In accordance with another aspect of the present invention, there is provided a chimeric antigen receptor (CAR) including the antigen-binding fragment.

In accordance with another aspect of the present invention, there is provided an immune cell including the chimeric antigen receptor (CAR).

In accordance with another aspect of the present invention, there is provided a composition containing the antibody or antigen-binding fragment thereof, the bispecific or multispecific antibody, the chimeric antigen receptor, or the immune cell.

In accordance with another aspect of the present invention, there is provided a composition for use in preventing or treating an immune or inflammatory disease, containing the antibody or antigen-binding fragment thereof, the bispecific or multispecific antibody, the chimeric antigen receptor, and/or the immune cell.

In accordance with another aspect of the present invention, there is provided the use of the antibody or antigen-binding fragment thereof, the bispecific or multispecific antibody, the chimeric antigen receptor, or the immune cell for the prevention or treatment of an immune disease or inflammatory disease.

In accordance with another aspect of the present invention, there is provided the use of the antibody or antigen-binding fragment thereof, the bispecific or multispecific antibody, the chimeric antigen receptor, or the immune cell for the preparation of a drug for preventing or treating an immune disease or inflammatory disease.

In accordance with another aspect of the present invention, there is provided a method for preventing or treating an immune disease or inflammatory disease, including administering the antibody or antigen-binding fragment thereof, the bispecific or multispecific antibody, the chimeric antigen receptor, and/or the immune cell to a subject.

### [Description of Drawings]

FIG. 1 shows the results of screening for human CD200R1-binding antibodies using phage display.
FIG. 2 shows the results of ELISA to determine cross-reactivity of clones identified through phage display to human and mouse CD200R1.
FIG. 3 shows the results of ELISA to determine competitive binding of clones identified through phage display to CD200 protein.
FIG. 4 shows the results of screening for human CD200R1-binding antibodies using hybridomas.
FIG. 5 is a schematic diagram illustrating humanization engineering of a P001017 antibody.
FIG. 6 shows the results of ELISA to determine human CD200R1-binding ability of humanized antibody clones of the P001017 antibody.
FIG. 7 shows the results of an in silico humanness analysis of P001018 antibody which is the humanized antibody of P001017 antibody.
FIG. 8 shows the results of an ELISA human CD200R1 binding assay of affinity-improved variants of the humanized antibody P001018.
FIG. 9 shows the results of identification of the cell-binding ability of affinity-improved mutants of the humanized antibody P001018 in HH cells.
FIG. 10 shows the results of ELISA of human CD200R1 and mouse CD200R1 binding ability of P001017, P001018 (humanized antibody), and the affinity-improved P001018v26 in IgG1 and IgG4 forms.
FIG. 11 shows the results of ELISA to determine whether the clones identified in the present invention cross-link with the immune activating receptors human CD200RLa and monkey CD200RLa.
FIG. 12 shows the differential binding of antibodies identified in the present invention to the human-mouse HM hybrid domain and the mouse-human MH hybrid domain.
FIG. 13 shows the results of a cell binding assay using the clones identified in the present invention in the IgG1 form in the CD200R-overexpressing cell lines Jurkat-CD200R and HH.
FIG. 14 shows the results of a cell binding assay using the CD200R-overexpressing cell lines Jurkat-CD200R and HH, respectively, after modifying the clones identified in the present invention with IgG4 and LALAPG mutations.
FIG. 15 shows the results of ELISA of P001009 and mutants subjected to antibody amino acid optimization and analysis of binding ability thereof to human, mouse, and monkey CD200R1.
FIG. 16 shows the results of SPR analysis of P001009 and mutants subjected to antibody amino acid optimization, and analysis of binding ability thereof to human and monkey CD200R1.
FIG. 17 shows the results of cell binding assays of P001009 and mutants subjected to antibody amino acid optimization.
FIG. 18 is a microscopic image of cells treated with LPS to induce a cellular inflammatory response to analyze the inflammatory cytokine suppression capacity in U937-CD200R1 overexpressing cells. FIG. 18A shows U937-CD200R1 cells without LPS treatment, and FIG. 18B shows U937-CD200R1 cells treated with 1 µg/ml LPS.
FIG. 19 shows the results of examining the inflammatory cytokine suppression capacity of the P001009 and P001009v1 antibodies in a U937-CD200R1 overexpressing cell line.
FIG. 20 shows the results of analysis of the degree of Dok2 protein phosphorylation induced by the P001009 and P001009v1 antibodies in a Jurkat-CD200R1 overexpressing cell line.
FIG. 21 shows an *ex vivo* screening flowchart using nasal polyp patient samples (top), and the results of establishing a cytokine suppression evaluation system using a positive control drug.
FIG. 22 shows P001009v1 exhibits superior cytokine suppression compared to conventional therapeutic agents in three patient samples.
FIG. 23 shows the results of evaluation of the anti-inflammatory efficacy of P001009v1 in an animal model of multiple sclerosis.
FIG. 24 shows the results of evaluation of the anti-inflammatory efficacy of P001009v1 in a contact dermatitis model.

### [Best Mode]

Unless defined otherwise, all technical and scientific terms used herein have the same meanings as those appreciated by those skilled in the field to which the present invention pertains. In general, the nomenclature used herein is well-known in the art and is ordinarily used.

### Anti-CD200R1 antibody

In one aspect, the present invention is directed to an antibody specifically binding to CD200R1 or an antigen-binding fragment thereof including:
a heavy chain CDR1 including at least one amino acid sequence selected from the group consisting of SEQ ID NOs: 1 to 8, SEQ ID NOs: 100 to 103, and SEQ ID NO: 158;
a heavy chain CDR2 including at least one amino acid sequence selected from the group consisting of SEQ ID NOs: 9 to 21, SEQ ID NOs: 104 to 109, and SEQ ID NO: 159;
a heavy chain CDR3 including at least one amino acid sequence selected from the group consisting of SEQ ID NOs: 22 to 35, SEQ ID NOs: 110 to 115, SEQ ID NO: 160, and SEQ ID NOs: 172 and 173;
a light chain CDR1 including at least one amino acid sequence selected from the group consisting of SEQ ID NOs: 36 to 47, SEQ ID NOs: 116 to 122, SEQ ID NO: 161, SEQ ID NO: 192, and SEQ ID NO: 193;
a light chain CDR2 including at least one amino acid sequence selected from the group consisting of SEQ ID NOs: 48 to 58, SEQ ID NOs: 123 to 127, SEQ ID NO: 162, and SEQ ID NO: 194; and
a light chain CDR3 including at least one amino acid sequence selected from the group consisting of SEQ ID NOs: 59 to 70, SEQ ID NOs: 128 to 134, SEQ ID NO: 163, SEQ ID NOs: 174 to 183, SEQ ID NO: 195, and SEQ ID NO: 196.

As used herein, the term "antibody" refers to an anti-CD200R1 antibody that specifically binds to CD200R1. The scope of the present invention includes not only a complete antibody specifically binding to CD200R1 but also an antigen-binding fragment of the antibody molecule.

The term "complete antibody" refers to a structure having two full-length light chains and two full-length heavy chains, wherein each light chain is linked to a corresponding heavy chain by a disulfide bond.

As used herein, the term "heavy chain" encompasses both a full-length heavy chain, which includes a variable domain (VH), containing an amino acid sequence having a variable region sequence sufficient for imparting specificity to an antigen and three constant domains (CH1, CH2 and CH3), and a fragment thereof. As used herein, the term "light chain" encompasses both a full-length light chain, which includes a variable domain (VL) containing an amino acid sequence having a variable region sequence sufficient for imparting specificity to an antigen and a constant domain (CL), and a fragment thereof.

The complete antibody includes subtypes of IgA, IgD, IgE, IgM and IgG, in particular IgG includes IgG1, IgG2, IgG3 and IgG4. The heavy chain constant region has gamma (γ), mu (µ), alpha (α), delta (δ) and epsilon (ε) types, and is subclassified into gamma 1 (γ1), gamma 2 (γ2), gamma 3 (γ3), gamma 4 (γ4), alpha 1 (α1) and alpha 2 (α2). The light-chain constant region has kappa (κ) and lambda (λ) types.

The antigen-binding fragment of an antibody or antibody fragment is a fragment that has antigen-binding ability and includes Fab, F(ab'), F(ab')₂, Fv and the like. Among the antibody fragments, Fab refers to a structure including a variable region of each of the heavy chain and the light chain, the constant region of the light chain, and the first constant domain (CH1) of the heavy chain, each having one antigen-binding site. Fab' is different from Fab in that it further includes a hinge region including at least one cysteine residue at the C-terminus of the CH1 domain of the heavy chain. F(ab')₂ is created by a disulfide bond between cysteine residues in the hinge region of Fab'.

Fv is the minimal antibody fragment having only a heavy-chain variable region and a light-chain variable region. Two-chain Fv is a fragment in which the variable region of the heavy chain and the variable region of the light chain are linked by a non-covalent bond, and single-chain Fv (scFv) is a fragment in which the variable region of the heavy chain and the variable region of the light chain are generally linked by a covalent bond via a peptide linker therebetween, or are directly linked at the C-terminal, forming a dimer-shaped structure, like the two-chain Fv. Such antibody fragments may be obtained using proteases (*e.g.,* Fab can be obtained by restriction-cleaving the complete antibody with papain, and the F(ab')₂ fragment can be obtained by restriction-cleaving the complete antibody with pepsin), and may be produced using genetic recombination techniques.

The "Fv" fragment is an antibody fragment containing complete antibody recognition and binding sites. Such a region includes a dimer that consists of one heavy chain variable domain and one light chain variable domain substantially bound to each other.

A "Fab" fragment contains a variable domain and a constant domain of the light chain and a variable domain and a first constant domain CH1 of the heavy chain. A F(ab')₂ antibody fragment generally includes a pair of Fab fragments covalently linked via cysteine of a hinge region present at the C-terminal of the Fab' fragment.

The "single chain Fv (scFv)" antibody fragment has a single polypeptide chain including VH and VL domains of the antibody. The scFv may further include a polypeptide linker between the VH domain and the VL domain in order for the scFv to form a desired structure for antigen binding.

In an embodiment, the antibody of the present invention includes, but is not limited to, monoclonal antibodies, multispecific antibodies, human antibodies, humanized antibodies, chimeric antibodies, single-chain Fvs (scFvs), Fab fragments, F(ab')₂ fragments, disulfide-bond Fvs (sdFVs), anti-idiotypic (anti-Id) antibodies, epitope-binding fragments of such antibodies, and the like.

The heavy chain constant region may be selected from gamma (γ), mu (µ), alpha (α), delta (δ) and epsilon (ε) types, and is, for example, gamma 1 (IgG1), gamma 2 (IgG2), gamma 3 (IgG3), or gamma 4 (IgG4). The light chain constant region may have kappa (κ) and lambda (λ) types.

"Monoclonal antibody" refers to an identical antibody, which is obtained from a population of substantially homogeneous antibodies, that is, each antibody constituting the population, excluding possible naturally occurring mutations that may be present in a minor amount. Monoclonal antibodies are highly specific and are induced against a single antigenic site. Unlike conventional (polyclonal) antibody preparations that typically include different antibodies directed against different determinants (epitopes), each monoclonal antibody is directed against a single determinant on the antigen.

The term "epitope" refers to a protein determinant to which an antibody can specifically bind. Epitopes usually consist of a group of chemically active surface molecules, such as amino acid or sugar side chains, and generally have not only specific three-dimensional structural characteristics but also specific charge characteristics. Three-dimensional epitopes are distinguished from non-three-dimensional epitopes in that the bond to the former is broken in the presence of a denatured solvent, while the bond to the latter is not broken.

The non-human (*e.g*., murine) antibody of the "humanized" form is a chimeric antibody containing a minimal sequence derived from non-human immunoglobulin. In most cases, the humanized antibody is a human immunoglobulin (receptor antibody) in which a residue from the hypervariable region of a receptor is replaced with a residue from the hypervariable region of a non-human species (donor antibody) such as a mouse, rat, rabbit or non-human primate having the desired specificity, affinity and ability.

As used herein, the term "human antibody" refers to a molecule derived from human immunoglobulin, in which all of the amino acid sequences constituting the antibody including a complementarity-determining region and a structural region are composed of human immunoglobulins.

The human antibody includes not only a "chimeric" antibody (immunoglobulin) in which the heavy chain and/or light chain portions are derived from a certain species, or are identical or homologous to the corresponding sequences in an antibody belonging to a certain antibody class or subclass, but the remaining chain(s) are derived from another species or are identical or homologous to the corresponding sequences in an antibody belonging to another antibody class or subclass, but also a fragment of the antibody that exhibits the desired biological activity.

As used herein, the term "variable domain" of the antibody refers to light chain and heavy chain regions of an antibody molecule including the amino acid sequence of a complementarity-determining region (CDR; i.e., CDR1, CDR2, and CDR3) and a framework region (FR). VH refers to a variable domain of the heavy chain. VL refers to a variable domain of the light chain.

The term "complementarity-determining region" (CDR; i.e., CDR1, CDR2, and CDR3) refers to an amino acid residue of the antibody variable domain that is necessary for antigen binding. Each variable domain typically has three CDR regions, identified as CDR1, CDR2, and CDR3.

In the present invention, the antibody or antigen-binding fragment thereof may include a heavy chain variable region selected from the group consisting of:
a heavy chain variable region including a heavy chain CDR1 of SEQ ID NO: 1, a heavy chain CDR2 of SEQ ID NO: 9, and a heavy chain CDR3 of SEQ ID NO: 22;
a heavy chain variable region including a heavy chain CDR1 of SEQ ID NO: 2, a heavy chain CDR2 of SEQ ID NO: 10, and a heavy chain CDR3 of SEQ ID NO: 23;
a heavy chain variable region including a heavy chain CDR1 of SEQ ID NO: 3, a heavy chain CDR2 of SEQ ID NO: 11, and a heavy chain CDR3 of SEQ ID NO: 24;
a heavy chain variable region including a heavy chain CDR1 of SEQ ID NO: 4, a heavy chain CDR2 of SEQ ID NO: 12, and a heavy chain CDR3 of SEQ ID NO: 25;
a heavy chain variable region including a heavy chain CDR1 of SEQ ID NO: 5, a heavy chain CDR2 of SEQ ID NO: 13, and a heavy chain CDR3 of SEQ ID NO: 26;
a heavy chain variable region including a heavy chain CDR1 of SEQ ID NO: 3, a heavy chain CDR2 of SEQ ID NO: 14, and a heavy chain CDR3 of SEQ ID NO: 27;
a heavy chain variable region including a heavy chain CDR1 of SEQ ID NO: 6, a heavy chain CDR2 of SEQ ID NO: 15, and a heavy chain CDR3 of SEQ ID NO: 28;
a heavy chain variable region including a heavy chain CDR1 of SEQ ID NO: 7, a heavy chain CDR2 of SEQ ID NO: 16, and a heavy chain CDR3 of SEQ ID NO: 29;
a heavy chain variable region including a heavy chain CDR1 of SEQ ID NO: 7, a heavy chain CDR2 of SEQ ID NO: 16, and a heavy chain CDR3 of SEQ ID NO: 30;
a heavy chain variable region including a heavy chain CDR1 of SEQ ID NO: 8, a heavy chain CDR2 of SEQ ID NO: 17, and a heavy chain CDR3 of SEQ ID NO: 31;
a heavy chain variable region including a heavy chain CDR1 of SEQ ID NO: 4, a heavy chain CDR2 of SEQ ID NO: 18, and a heavy chain CDR3 of SEQ ID NO: 32;
a heavy chain variable region including a heavy chain CDR1 of SEQ ID NO: 5, a heavy chain CDR2 of SEQ ID NO: 19, and a heavy chain CDR3 of SEQ ID NO: 33;
a heavy chain variable region including a heavy chain CDR1 of SEQ ID NO: 5, a heavy chain CDR2 of SEQ ID NO: 20, and a heavy chain CDR3 of SEQ ID NO: 34;
a heavy chain variable region including a heavy chain CDR1 of SEQ ID NO: 5, a heavy chain CDR2 of SEQ ID NO: 21, and a heavy chain CDR3 of SEQ ID NO: 35;
a heavy chain variable region including a heavy chain CDR1 of SEQ ID NO: 100, a heavy chain CDR2 of SEQ ID NO: 104, and a heavy chain CDR3 of SEQ ID NO: 110;
a heavy chain variable region including a heavy chain CDR1 of SEQ ID NO: 101, a heavy chain CDR2 of SEQ ID NO: 105, and a heavy chain CDR3 of SEQ ID NO: 111;
a heavy chain variable region including a heavy chain CDR1 of SEQ ID NO: 100, a heavy chain CDR2 of SEQ ID NO: 106, and a heavy chain CDR3 of SEQ ID NO: 112;
a heavy chain variable region including a heavy chain CDR1 of SEQ ID NO: 102, a heavy chain CDR2 of SEQ ID NO: 107, and a heavy chain CDR3 of SEQ ID NO: 113;
a heavy chain variable region including a heavy chain CDR1 of SEQ ID NO: 100, a heavy chain CDR2 of SEQ ID NO: 106, and a heavy chain CDR3 of SEQ ID NO: 110;
a heavy chain variable region including a heavy chain CDR1 of SEQ ID NO: 103, a heavy chain CDR2 of SEQ ID NO: 108, and a heavy chain CDR3 of SEQ ID NO: 114;
a heavy chain variable region including the heavy chain CDR1 of SEQ ID NO: 100, the heavy chain CDR2 of SEQ ID NO: 109, and a heavy chain CDR3 of SEQ ID NO: 115;
a heavy chain variable region including a heavy chain CDR1 of SEQ ID NO: 158, a heavy chain CDR2 of SEQ ID NO: 159, and a heavy chain CDR3 of SEQ ID NO: 160;
a heavy chain variable region including the heavy chain CDR1 of SEQ ID NO: 158, the heavy chain CDR2 of SEQ ID NO: 159, and a heavy chain CDR3 of SEQ ID NO: 172; and
a heavy chain variable region including the heavy chain CDR1 of SEQ ID NO: 158, the heavy chain CDR2 of SEQ ID NO: 159, and a heavy chain CDR3 of SEQ ID NO: 173.

In the present invention, the antibody or antigen-binding fragment thereof may include a light chain variable region selected from the group consisting of:
a light chain variable region including a light chain CDR1 of SEQ ID NO: 36, a light chain CDR2 of SEQ ID NO: 48, and a light chain CDR3 of SEQ ID NO: 59;
a light chain variable region including a light chain CDR1 of SEQ ID NO: 37, a light chain CDR2 of SEQ ID NO: 49, and a light chain CDR3 of SEQ ID NO: 60;
a light chain variable region including a light chain CDR1 of SEQ ID NO: 38, a light chain CDR2 of SEQ ID NO: 50, and a light chain CDR3 of SEQ ID NO: 61;
a light chain variable region including a light chain CDR1 of SEQ ID NO: 37, a light chain CDR2 of SEQ ID NO: 51, and a light chain CDR3 of SEQ ID NO: 62;
a light chain variable region including a light chain CDR1 of SEQ ID NO: 39, a light chain CDR2 of SEQ ID NO: 51, and a light chain CDR3 of SEQ ID NO: 62;
a light chain variable region including a light chain CDR1 of SEQ ID NO: 40, a light chain CDR2 of SEQ ID NO: 52, and a light chain CDR3 of SEQ ID NO: 63;
a light chain variable region including a light chain CDR1 of SEQ ID NO: 41, a light chain CDR2 of SEQ ID NO: 53, and a light chain CDR3 of SEQ ID NO: 64;
a light chain variable region including a light chain CDR1 of SEQ ID NO: 42, a light chain CDR2 of SEQ ID NO: 53, and a light chain CDR3 of SEQ ID NO: 65;
a light chain variable region including a light chain CDR1 of SEQ ID NO: 43, a light chain CDR2 of SEQ ID NO: 54, and a light chain CDR3 of SEQ ID NO: 66;
a light chain variable region including a light chain CDR1 of SEQ ID NO: 44, a light chain CDR2 of SEQ ID NO: 55, and a light chain CDR3 of SEQ ID NO: 65;
a light chain variable region including a light chain CDR1 of SEQ ID NO: 45, a light chain CDR2 of SEQ ID NO: 56, and a light chain CDR3 of SEQ ID NO: 67;
a light chain variable region including a light chain CDR1 of SEQ ID NO: 46, a light chain CDR2 of SEQ ID NO: 53, and a light chain CDR3 of SEQ ID NO: 68;
a light chain variable region including a light chain CDR1 of SEQ ID NO: 39, a light chain CDR2 of SEQ ID NO: 57, and a light chain CDR3 of SEQ ID NO: 69;
a light chain variable region including a light chain CDR1 of SEQ ID NO: 47, a light chain CDR2 of SEQ ID NO: 58, and a light chain CDR3 of SEQ ID NO: 70;
a light chain variable region including a light chain CDR1 of SEQ ID NO: 116, a light chain CDR2 of SEQ ID NO: 123, and a light chain CDR3 of SEQ ID NO: 128;
a light chain variable region including a light chain CDR1 of SEQ ID NO: 117, a light chain CDR2 of SEQ ID NO: 124, and a light chain CDR3 of SEQ ID NO: 129;
a light chain variable region including a light chain CDR1 of SEQ ID NO: 118, a light chain CDR2 of SEQ ID NO: 125, and a light chain CDR3 of SEQ ID NO: 130;
a light chain variable region including a light chain CDR1 of SEQ ID NO: 119, a light chain CDR2 of SEQ ID NO: 124, and a light chain CDR3 of SEQ ID NO: 131;
a light chain variable region including a light chain CDR1 of SEQ ID NO: 120, a light chain CDR2 of SEQ ID NO: 125, and a light chain CDR3 of SEQ ID NO: 132;
a light chain variable region including a light chain CDR1 of SEQ ID NO: 121, a light chain CDR2 of SEQ ID NO: 126, and a light chain CDR3 of SEQ ID NO: 133;
a light chain variable region including a light chain CDR1 of SEQ ID NO: 122, a light chain CDR2 of SEQ ID NO: 127, and a light chain CDR3 of SEQ ID NO: 134;
a light chain variable region including a light chain CDR1 of SEQ ID NO: 161, a light chain CDR2 of SEQ ID NO: 162, and a light chain CDR3 of SEQ ID NO: 163;
a light chain variable region including the light chain CDR1 of SEQ ID NO: 161, the light chain CDR2 of SEQ ID NO: 162, and a light chain CDR3 of SEQ ID NO: 174;
a light chain variable region including the light chain CDR1 of SEQ ID NO: 161, the light chain CDR2 of SEQ ID NO: 162, and a light chain CDR3 of SEQ ID NO: 175;
a light chain variable region including the light chain CDR1 of SEQ ID NO: 161, the light chain CDR2 of SEQ ID NO: 162, and a light chain CDR3 of SEQ ID NO: 176;
a light chain variable region including the light chain CDR1 of SEQ ID NO: 161, the light chain CDR2 of SEQ ID NO: 162, and a light chain CDR3 of SEQ ID NO: 177;
a light chain variable region including the light chain CDR1 of SEQ ID NO: 161, the light chain CDR2 of SEQ ID NO: 162, and a light chain CDR3 of SEQ ID NO: 178;
a light chain variable region including the light chain CDR1 of SEQ ID NO: 161, the light chain CDR2 of SEQ ID NO: 162, and a light chain CDR3 of SEQ ID NO: 179;
a light chain variable region including the light chain CDR1 of SEQ ID NO: 161, the light chain CDR2 of SEQ ID NO: 162, and a light chain CDR3 of SEQ ID NO: 180;
a light chain variable region including the light chain CDR1 of SEQ ID NO: 161, the light chain CDR2 of SEQ ID NO: 162, and a light chain CDR3 of SEQ ID NO: 181;
a light chain variable region including the light chain CDR1 of SEQ ID NO: 161, the light chain CDR2 of SEQ ID NO: 162, and a light chain CDR3 of SEQ ID NO: 182;
a light chain variable region including the light chain CDR1 of SEQ ID NO: 161, the light chain CDR2 of SEQ ID NO: 162, and a light chain CDR3 of SEQ ID NO: 183;
a light chain variable region including the light chain CDR1 of SEQ ID NO: 43, a light chain CDR2 of SEQ ID NO: 194, and a light chain CDR3 of SEQ ID NO: 195;
a light chain variable region including the light chain CDR1 of SEQ ID NO: 43, the light chain CDR2 of SEQ ID NO: 194, and a light chain CDR3 of SEQ ID NO: 196;
a light chain variable region including a light chain CDR1 of SEQ ID NO: 192, the light chain CDR2 of SEQ ID NO: 54, and the light chain CDR3 of SEQ ID NO: 66; and
a light chain variable region including a light chain CDR1 of SEQ ID NO: 193, the light chain CDR2 of SEQ ID NO: 54, and the light chain CDR3 of SEQ ID NO: 66.

In the present invention, the antibody or antigen-binding fragment thereof may include a heavy chain variable region and a light chain variable region selected from the group consisting of:
a heavy chain variable region including the heavy chain CDR1 of SEQ ID NO: 1, the heavy chain CDR2 of SEQ ID NO: 9, and the heavy chain CDR3 of SEQ ID NO: 22; and a light chain variable region including the light chain CDR1 of SEQ ID NO: 36, the light chain CDR2 of SEQ ID NO: 48, and the light chain CDR3 of SEQ ID NO: 59;
a heavy chain variable region including the heavy chain CDR1 of SEQ ID NO: 2, the heavy chain CDR2 of SEQ ID NO: 10, and the heavy chain CDR3 of SEQ ID NO: 23; and a light chain variable region including the light chain CDR1 of SEQ ID NO: 37, the light chain CDR2 of SEQ ID NO: 49, and the light chain CDR3 of SEQ ID NO: 60;
a heavy chain variable region including the heavy chain CDR1 of SEQ ID NO: 3, the heavy chain CDR2 of SEQ ID NO: 11, and the heavy chain CDR3 of SEQ ID NO: 24; and a light chain variable region including the light chain CDR1 of SEQ ID NO: 38, the light chain CDR2 of SEQ ID NO: 50, and the light chain CDR3 of SEQ ID NO: 61;
a heavy chain variable region including the heavy chain CDR1 of SEQ ID NO: 4, the heavy chain CDR2 of SEQ ID NO: 12, and the heavy chain CDR3 of SEQ ID NO: 25; and a light chain variable region including the light chain CDR1 of SEQ ID NO: 37, the light chain CDR2 of SEQ ID NO: 51, and the light chain CDR3 of SEQ ID NO: 62;
a heavy chain variable region including the heavy chain CDR1 of SEQ ID NO: 5, the heavy chain CDR2 of SEQ ID NO: 13, and the heavy chain CDR3 of SEQ ID NO: 26; and a light chain variable region including the light chain CDR1 of SEQ ID NO: 39, the light chain CDR2 of SEQ ID NO: 51, and the light chain CDR3 of SEQ ID NO: 62;
a heavy chain variable region including the heavy chain CDR1 of SEQ ID NO: 3, the heavy chain CDR2 of SEQ ID NO: 14, and the heavy chain CDR3 of SEQ ID NO: 27; and a light chain variable region including the light chain CDR1 of SEQ ID NO: 40, the light chain CDR2 of SEQ ID NO: 52, and the light chain CDR3 of SEQ ID NO: 63;
a heavy chain variable region including the heavy chain CDR1 of SEQ ID NO: 6, the heavy chain CDR2 of SEQ ID NO: 15, and the heavy chain CDR3 of SEQ ID NO: 28; and a light chain variable region including the light chain CDR1 of SEQ ID NO: 41, the light chain CDR2 of SEQ ID NO: 53, and the light chain CDR3 of SEQ ID NO: 64;
a heavy chain variable region including the heavy chain CDR1 of SEQ ID NO: 7, the heavy chain CDR2 of SEQ ID NO: 16, and the heavy chain CDR3 of SEQ ID NO: 29; and a light chain variable region including the light chain CDR1 of SEQ ID NO: 42, the light chain CDR2 of SEQ ID NO: 53, and the light chain CDR3 of SEQ ID NO: 65;
a heavy chain variable region including the heavy chain CDR1 of SEQ ID NO: 7, the heavy chain CDR2 of SEQ ID NO: 16, and the heavy chain CDR3 of SEQ ID NO: 30; and a light chain variable region including the light chain CDR1 of SEQ ID NO: 43, the light chain CDR2 of SEQ ID NO: 54, and the light chain CDR3 of SEQ ID NO: 66;
a heavy chain variable region including the heavy chain CDR1 of SEQ ID NO: 8, the heavy chain CDR2 of SEQ ID NO: 17, and the heavy chain CDR3 of SEQ ID NO: 31; and a light chain variable region including the light chain CDR1 of SEQ ID NO: 44, the light chain CDR2 of SEQ ID NO: 55, and the light chain CDR3 of SEQ ID NO: 65;
a heavy chain variable region including the heavy chain CDR1 of SEQ ID NO: 4, the heavy chain CDR2 of SEQ ID NO: 18, and the heavy chain CDR3 of SEQ ID NO: 32; and a light chain variable region including the light chain CDR1 of SEQ ID NO: 45, the light chain CDR2 of SEQ ID NO: 56, and the light chain CDR3 of SEQ ID NO: 67;
a heavy chain variable region including the heavy chain CDR1 of SEQ ID NO: 5, the heavy chain CDR2 of SEQ ID NO: 19, and the heavy chain CDR3 of SEQ ID NO: 33; and a light chain variable region including the light chain CDR1 of SEQ ID NO: 46, the light chain CDR2 of SEQ ID NO: 53, and the light chain CDR3 of SEQ ID NO: 68;
a heavy chain variable region including the heavy chain CDR1 of SEQ ID NO: 5, the heavy chain CDR2 of SEQ ID NO: 20, and the heavy chain CDR3 of SEQ ID NO: 34; and a light chain variable region including the light chain CDR1 of SEQ ID NO: 39, the light chain CDR2 of SEQ ID NO: 57, and the light chain CDR3 of SEQ ID NO: 69;
a heavy chain variable region including the heavy chain CDR1 of SEQ ID NO: 5, the heavy chain CDR2 of SEQ ID NO: 21, and the heavy chain CDR3 of SEQ ID NO: 35; and a light chain variable region including the light chain CDR1 of SEQ ID NO: 47, the light chain CDR2 of SEQ ID NO: 58, and the light chain CDR3 of SEQ ID NO: 70;
a heavy chain variable region including the heavy chain CDR1 of SEQ ID NO: 100, the heavy chain CDR2 of SEQ ID NO: 104, and the heavy chain CDR3 of SEQ ID NO: 110; and a light chain variable region including the light chain CDR1 of SEQ ID NO: 116, the light chain CDR2 of SEQ ID NO: 123, and the light chain CDR3 of SEQ ID NO: 128;
a heavy chain variable region including the heavy chain CDR1 of SEQ ID NO: 101, the heavy chain CDR2 of SEQ ID NO: 105, and the heavy chain CDR3 of SEQ ID NO: 111; and a light chain variable region including the light chain CDR1 of SEQ ID NO: 117, the light chain CDR2 of SEQ ID NO: 124, and the light chain CDR3 of SEQ ID NO: 129;
a heavy chain variable region including the heavy chain CDR1 of SEQ ID NO: 100, the heavy chain CDR2 of SEQ ID NO: 106, and the heavy chain CDR3 of SEQ ID NO: 112; and a light chain variable region including the light chain CDR1 of SEQ ID NO: 118, the light chain CDR2 of SEQ ID NO: 125, and the light chain CDR3 of SEQ ID NO: 130;
a heavy chain variable region including a heavy chain CDR1 of SEQ ID NO: 102, a heavy chain CDR2 of SEQ ID NO: 107, and a heavy chain CDR3 of SEQ ID NO: 113; and a light chain variable region including a light chain CDR1 of SEQ ID NO: 119, a light chain CDR2 of SEQ ID NO: 124, and a light chain CDR3 of SEQ ID NO: 131;
a heavy chain variable region including a heavy chain CDR1 of SEQ ID NO: 100, a heavy chain CDR2 of SEQ ID NO: 106, and a heavy chain CDR3 of SEQ ID NO: 110; and a light chain variable region including a light chain CDR1 of SEQ ID NO: 120, a light chain CDR2 of SEQ ID NO: 125, and a light chain CDR3 of SEQ ID NO: 132;
a heavy chain variable region including a heavy chain CDR1 of SEQ ID NO: 103, a heavy chain CDR2 of SEQ ID NO: 108, and a heavy chain CDR3 of SEQ ID NO: 114; and a light chain variable region including a light chain CDR1 of SEQ ID NO: 121, a light chain CDR2 of SEQ ID NO: 126, and a light chain CDR3 of SEQ ID NO: 133;
a heavy chain variable region including a heavy chain CDR1 of SEQ ID NO: 100, a heavy chain CDR2 of SEQ ID NO: 109, and a heavy chain CDR3 of SEQ ID NO: 115; and a light chain variable region including a light chain CDR1 of SEQ ID NO: 122, a light chain CDR2 of SEQ ID NO: 127, and a light chain CDR3 of SEQ ID NO: 134;
a heavy chain variable region including the heavy chain CDR1 of SEQ ID NO: 158, the heavy chain CDR2 of SEQ ID NO: 159, and the heavy chain CDR3 of SEQ ID NO: 160; and a light chain variable region including the light chain CDR1 of SEQ ID NO: 161, the light chain CDR2 of SEQ ID NO: 162, and the light chain CDR3 of SEQ ID NO: 163;
a heavy chain variable region including the heavy chain CDR1 of SEQ ID NO: 158, the heavy chain CDR2 of SEQ ID NO: 159, and the heavy chain CDR3 of SEQ ID NO: 172; and a light chain variable region including the light chain CDR1 of SEQ ID NO: 161, a light chain CDR2 of SEQ ID NO: 162, and the light chain CDR3 of SEQ ID NO: 163;
a heavy chain variable region including the heavy chain CDR1 of SEQ ID NO: 158, the heavy chain CDR2 of SEQ ID NO: 159, and the heavy chain CDR3 of SEQ ID NO: 173; and a light chain variable region including the light chain CDR1 of SEQ ID NO: 161, the light chain CDR2 of SEQ ID NO: 162, and the light chain CDR3 of SEQ ID NO: 163;
a heavy chain variable region including the heavy chain CDR1 of SEQ ID NO: 158, the heavy chain CDR2 of SEQ ID NO: 159, and the heavy chain CDR3 of SEQ ID NO: 160; and a light chain variable region including the light chain CDR1 of SEQ ID NO: 161, the light chain CDR2 of SEQ ID NO: 162, and the light chain CDR3 of SEQ ID NO: 174;
a heavy chain variable region including the heavy chain CDR1 of SEQ ID NO: 158, the heavy chain CDR2 of SEQ ID NO: 159, and the heavy chain CDR3 of SEQ ID NO: 160; and a light chain variable region including the light chain CDR1 of SEQ ID NO: 161, the light chain CDR2 of SEQ ID NO: 162, and the light chain CDR3 of SEQ ID NO: 175;
a heavy chain variable region including the heavy chain CDR1 of SEQ ID NO: 158, the heavy chain CDR2 of SEQ ID NO: 159, and the heavy chain CDR3 of SEQ ID NO: 160; and a light chain variable region including the light chain CDR1 of SEQ ID NO: 161, the light chain CDR2 of SEQ ID NO: 162, and the light chain CDR3 of SEQ ID NO: 176;
a heavy chain variable region including the heavy chain CDR1 of SEQ ID NO: 158, the heavy chain CDR2 of SEQ ID NO: 159, and the heavy chain CDR3 of SEQ ID NO: 160; and a light chain variable region including the light chain CDR1 of SEQ ID NO: 161, the light chain CDR2 of SEQ ID NO: 162, and the light chain CDR3 of SEQ ID NO: 177;
a heavy chain variable region including the heavy chain CDR1 of SEQ ID NO: 158, the heavy chain CDR2 of SEQ ID NO: 159, and the heavy chain CDR3 of SEQ ID NO: 160; and a light chain variable region including the light chain CDR1 of SEQ ID NO: 161, the light chain CDR2 of SEQ ID NO: 162, and the light chain CDR3 of SEQ ID NO: 178;
a heavy chain variable region including the heavy chain CDR1 of SEQ ID NO: 158, the heavy chain CDR2 of SEQ ID NO: 159, and the heavy chain CDR3 of SEQ ID NO: 160; and a light chain variable region including the light chain CDR1 of SEQ ID NO: 161, the light chain CDR2 of SEQ ID NO: 162, and the light chain CDR3 of SEQ ID NO: 179;
a heavy chain variable region including the heavy chain CDR1 of SEQ ID NO: 158, the heavy chain CDR2 of SEQ ID NO: 159, and the heavy chain CDR3 of SEQ ID NO: 160; and a light chain variable region including the light chain CDR1 of SEQ ID NO: 161, the light chain CDR2 of SEQ ID NO: 162, and the light chain CDR3 of SEQ ID NO: 180;
a heavy chain variable region including the heavy chain CDR1 of SEQ ID NO: 158, the heavy chain CDR2 of SEQ ID NO: 159, and the heavy chain CDR3 of SEQ ID NO: 173; and a light chain variable region including the light chain CDR1 of SEQ ID NO: 161, the light chain CDR2 of SEQ ID NO: 162, and the light chain CDR3 of SEQ ID NO: 174;
a heavy chain variable region including the heavy chain CDR1 of SEQ ID NO: 158, the heavy chain CDR2 of SEQ ID NO: 159, and the heavy chain CDR3 of SEQ ID NO: 173; and a light chain variable region including the light chain CDR1 of SEQ ID NO: 161, the light chain CDR2 of SEQ ID NO: 162, and the light chain CDR3 of SEQ ID NO: 178;
a heavy chain variable region including the heavy chain CDR1 of SEQ ID NO: 158, the heavy chain CDR2 of SEQ ID NO: 159, and the heavy chain CDR3 of SEQ ID NO: 160; and a light chain variable region including the light chain CDR1 of SEQ ID NO: 161, the light chain CDR2 of SEQ ID NO: 162, and the light chain CDR3 of SEQ ID NO: 181;
a heavy chain variable region including the heavy chain CDR1 of SEQ ID NO: 158, the heavy chain CDR2 of SEQ ID NO: 159, and the heavy chain CDR3 of SEQ ID NO: 173; and a light chain variable region including the light chain CDR1 of SEQ ID NO: 161, the light chain CDR2 of SEQ ID NO: 162, and the light chain CDR3 of SEQ ID NO: 182;
a heavy chain variable region including the heavy chain CDR1 of SEQ ID NO: 158, the heavy chain CDR2 of SEQ ID NO: 159, and the heavy chain CDR3 of SEQ ID NO: 173; and a light chain variable region including the light chain CDR1 of SEQ ID NO: 161, the light chain CDR2 of SEQ ID NO: 162, and the light chain CDR3 of SEQ ID NO: 183;
a heavy chain variable region including the heavy chain CDR1 of SEQ ID NO: 7, the heavy chain CDR2 of SEQ ID NO: 16, and the heavy chain CDR3 of SEQ ID NO: 30; and a light chain variable region including the light chain CDR1 of SEQ ID NO: 43, the light chain CDR2 of SEQ ID NO: 194, and the light chain CDR3 of SEQ ID NO: 195;
a heavy chain variable region including the heavy chain CDR1 of SEQ ID NO: 7, the heavy chain CDR2 of SEQ ID NO: 16, and the heavy chain CDR3 of SEQ ID NO: 30; and a light chain variable region including the light chain CDR1 of SEQ ID NO: 43, the light chain CDR2 of SEQ ID NO: 194, and the light chain CDR3 of SEQ ID NO: 196;
a heavy chain variable region including the heavy chain CDR1 of SEQ ID NO: 7, the heavy chain CDR2 of SEQ ID NO: 16, and the heavy chain CDR3 of SEQ ID NO: 30; and a light chain variable region including the light chain CDR1 of SEQ ID NO: 192, the light chain CDR2 of SEQ ID NO: 54, and the light chain CDR3 of SEQ ID NO: 66; and
a heavy chain variable region including the heavy chain CDR1 of SEQ ID NO: 7, the heavy chain CDR2 of SEQ ID NO: 16, and the heavy chain CDR3 of SEQ ID NO: 30; and a light chain variable region including the light chain CDR1 of SEQ ID NO: 193, the light chain CDR2 of SEQ ID NO: 54, and the light chain CDR3 of SEQ ID NO: 66.

The term "framework region" (FR) refers to a variable domain residue other than a CDR residue. Each variable domain typically has four FRs identified as FR1, FR2, FR3, and FR4.

The binding affinity of the anti-CD200R1 antibody to CD200R1 ranges from 10⁻⁵ M to 10⁻¹³ M. For example, the binding affinity of the anti-CD200R1 antibody to CD200R1 is 10⁻⁶ M to 10⁻¹³ M, 10⁻⁷ M to 10⁻¹³ M, 10⁻⁸ M to 10⁻¹³ M, 10⁻⁹ M to 10⁻¹³ M, 10⁻⁵ M to 10⁻¹¹ M, 10⁻⁶ M to 10⁻¹¹ M, 10⁻⁷ M to 10⁻¹¹ M, 10⁻⁸ M to 10⁻¹¹ M, 10⁻⁹ M to 10⁻¹¹ M, 10⁻¹⁰ M to 10⁻¹¹ M, 10⁻⁵ M to 10⁻¹⁰ M, 10⁻⁶ M to 10⁻¹⁰ M, 10⁻⁷ M to 10⁻¹⁰ M, 10⁻⁸ M to 10⁻¹⁰ M, 10⁻⁹ M to 10⁻¹⁰ M, 10⁻⁵ M to 10⁻⁹ M, 10⁻⁶ M to 10⁻⁹ M, 10⁻⁷ M to 10⁻⁹ M, 10⁻⁸ M to 10⁻⁹ M, 10⁻⁵ M to 10⁻⁸ M, 10⁻⁶ M to 10⁻⁸ M, 10⁻⁷ M to 10⁻⁸ M, 10⁻⁵ M to 10⁻⁷ M, 10⁻⁶ M to 10⁻⁷ M, or 10⁻⁵ M to 10⁻⁶ M.

In the present invention, the antibody binding to CD200R1 or antigen-binding fragment thereof may include a heavy chain variable region including at least one amino acid sequence selected from the group consisting of SEQ ID NOs: 71 to 85, SEQ ID NOs: 135 to 141, SEQ ID NOs: 149 to 153, SEQ ID NO: 184, and SEQ ID NO: 185.

In the present invention, the antibody binding to CD200R1 or antigen-binding fragment thereof may include a light chain variable region including at least one amino acid sequence selected from the group consisting of SEQ ID NOs: 86 to 99, SEQ ID NOs: 142 to 148, SEQ ID NOs: 154 to 157, SEQ ID NOs: 186 to 191, and SEQ ID NOs: 197 to 200.

According to one embodiment of the present invention, the antibody or an antigen-binding fragment thereof may include:
- a heavy chain variable region of SEQ ID NO: 71 and a light chain variable region of SEQ ID NO: 86;
- a heavy chain variable region of SEQ ID NO: 72 and a light chain variable region of SEQ ID NO: 87;
- a heavy chain variable region of SEQ ID NO: 73 and a light chain variable region of SEQ ID NO: 88;
- a heavy chain variable region of SEQ ID NO: 74 and a light chain variable region of SEQ ID NO: 89;
- a heavy chain variable region of SEQ ID NO: 75 and a light chain variable region of SEQ ID NO: 90;
- a heavy chain variable region of SEQ ID NO: 76 and a light chain variable region of SEQ ID NO: 91;
- a heavy chain variable region of SEQ ID NO: 77 and a light chain variable region of SEQ ID NO: 92;
- a heavy chain variable region of SEQ ID NO: 78 and a light chain variable region of SEQ ID NO: 93;
- a heavy chain variable region of SEQ ID NO: 79 and a light chain variable region of SEQ ID NO: 94;
   a heavy chain variable region of SEQ ID NO: 80 and a light chain variable region of SEQ ID NO: 95;
- a heavy chain variable region of SEQ ID NO: 81 and a light chain variable region of SEQ ID NO: 96;
- a heavy chain variable region of SEQ ID NO: 82 and a light chain variable region of SEQ ID NO: 97;
- a heavy chain variable region of SEQ ID NO: 83 and a light chain variable region of SEQ ID NO: 98;
- a heavy chain variable region of SEQ ID NO: 84 and a light chain variable region of SEQ ID NO: 94;
- a heavy chain variable region of SEQ ID NO: 85 and a light chain variable region of SEQ ID NO: 99;
- a heavy chain variable region of SEQ ID NO: 135 and a light chain variable region of SEQ ID NO: 142;
- a heavy chain variable region of SEQ ID NO: 136 and a light chain variable region of SEQ ID NO: 143;
- a heavy chain variable region of SEQ ID NO: 137 and a light chain variable region of SEQ ID NO: 144;
- a heavy chain variable region of SEQ ID NO: 138 and a light chain variable region of SEQ ID NO: 145;
- a heavy chain variable region of SEQ ID NO: 139 and a light chain variable region of SEQ ID NO: 146;
- a heavy chain variable region of SEQ ID NO: 140 and a light chain variable region of SEQ ID NO: 147;
- a heavy chain variable region of SEQ ID NO: 141 and a light chain variable region of SEQ ID NO: 148;
- a heavy chain variable region of SEQ ID NO: 150 and a light chain variable region of SEQ ID NO: 155;
- a heavy chain variable region of SEQ ID NO: 151 and a light chain variable region of SEQ ID NO: 155;
- a heavy chain variable region of SEQ ID NO: 152 and a light chain variable region of SEQ ID NO: 157;
- a heavy chain variable region of SEQ ID NO: 153 and a light chain variable region of SEQ ID NO: 157;
- a heavy chain variable region of SEQ ID NO: 151 and a light chain variable region of SEQ ID NO: 157;
- a heavy chain variable region of SEQ ID NO: 152 and a light chain variable region of SEQ ID NO: 155;
- a heavy chain variable region of SEQ ID NO: 184 and a light chain variable region of SEQ ID NO: 155;
   a heavy chain variable region of SEQ ID NO: 150 and a light chain variable region of SEQ ID NO: 186;
- a heavy chain variable region of SEQ ID NO: 150 and a light chain variable region of SEQ ID NO: 187;

a heavy chain variable region of SEQ ID NO: 150 and a light chain variable region of SEQ ID NO: 188;
a heavy chain variable region of SEQ ID NO: 150 and a light chain variable region of SEQ ID NO: 189;
a heavy chain variable region of SEQ ID NO: 185 and a light chain variable region of SEQ ID NO: 155;
a heavy chain variable region of SEQ ID NO: 150 and a light chain variable region of SEQ ID NO: 190;
a heavy chain variable region of SEQ ID NO: 185 and a light chain variable region of SEQ ID NO: 186;
a heavy chain variable region of SEQ ID NO: 185 and a light chain variable region of SEQ ID NO: 187;
a heavy chain variable region of SEQ ID NO: 150 and a light chain variable region of SEQ ID NO: 191;
a heavy chain variable region of SEQ ID NO: 185 and a light chain variable region of SEQ ID NO: 190;
a heavy chain variable region of SEQ ID NO: 185 and a light chain variable region of SEQ ID NO: 191;
a heavy chain variable region of SEQ ID NO: 79 and a light chain variable region of SEQ ID NO: 197;
a heavy chain variable region of SEQ ID NO: 79 and a light chain variable region of SEQ ID NO: 198;
a heavy chain variable region of SEQ ID NO: 79 and a light chain variable region of SEQ ID NO: 199; or
a heavy chain variable region of SEQ ID NO: 79 and a light chain variable region of SEQ ID NO: 200.

### scFv

An scFv is an antibody fragment, consisting of a single polypeptide chain including the VH and VL domains of an antibody. The scFv may further include a polypeptide linker between the VH and VL domains, enabling the scFv to form the desired structure for antigen binding.

In one embodiment, in a single-chain Fv (scFv) including the VH and VL domains of an antibody, the VH and VL domains may be linked via a linker. A heavy chain variable region including at least one amino acid sequence selected from the group consisting of SEQ ID NOs: 71 to 85, SEQ ID NOs: 135 to 141, SEQ ID NOs: 149 to 153, SEQ ID NOs: 184, and SEQ ID NOs: 185 may be linked to a light chain variable region including at least one amino acid sequence selected from the group consisting of SEQ ID NOs: 86 to 99, SEQ ID NOs: 142 to 148, SEQ ID NOs: 154 to 157, SEQ ID NOs: 186 to 191, and SEQ ID NOs: 197 to 200 via a linker.

In a specific embodiment of the present invention, the antibody or antigen-binding fragment thereof may include:
- a heavy chain variable region of SEQ ID NO: 71 and a light chain variable region of SEQ ID NO: 86;
- a heavy chain variable region of SEQ ID NO: 72 and a light chain variable region of SEQ ID NO: 87;
- a heavy chain variable region of SEQ ID NO: 73 and a light chain variable region of SEQ ID NO: 88;
- a heavy chain variable region of SEQ ID NO: 74 and a light chain variable region of SEQ ID NO: 89;
- a heavy chain variable region of SEQ ID NO: 75 and a light chain variable region of SEQ ID NO: 90;
- a heavy chain variable region of SEQ ID NO: 76 and a light chain variable region of SEQ ID NO: 91;
- a heavy chain variable region of SEQ ID NO: 77 and a light chain variable region of SEQ ID NO: 92;
- a heavy chain variable region of SEQ ID NO: 78 and a light chain variable region of SEQ ID NO: 93;
- a heavy chain variable region of SEQ ID NO: 79 and a light chain variable region of SEQ ID NO: 94;
- a heavy chain variable region of SEQ ID NO: 80 and a light chain variable region of SEQ ID NO: 95;
- a heavy chain variable region of SEQ ID NO: 81 and a light chain variable region of SEQ ID NO: 96;
- a heavy chain variable region of SEQ ID NO: 82 and a light chain variable region of SEQ ID NO: 97;
- a heavy chain variable region of SEQ ID NO: 83 and a light chain variable region of SEQ ID NO: 98;
- a heavy chain variable region of SEQ ID NO: 84 and a light chain variable region of SEQ ID NO: 94;
- a heavy chain variable region of SEQ ID NO: 85 and a light chain variable region of SEQ ID NO: 99;
- a heavy chain variable region of SEQ ID NO: 135 and a light chain variable region of SEQ ID NO: 142;
- a heavy chain variable region of SEQ ID NO: 136 and a light chain variable region of SEQ ID NO: 143;
- a heavy chain variable region of SEQ ID NO: 137 and a light chain variable region of SEQ ID NO: 144;
- a heavy chain variable region of SEQ ID NO: 138 and a light chain variable region of SEQ ID NO: 145;
- a heavy chain variable region of SEQ ID NO: 139 and a light chain variable region of SEQ ID NO: 146;
- a heavy chain variable region of SEQ ID NO: 140 and a light chain variable region of SEQ ID NO: 147;
- a heavy chain variable region of SEQ ID NO: 141 and a light chain variable region of SEQ ID NO: 148;
- a heavy chain variable region of SEQ ID NO: 150 and a light chain variable region of SEQ ID NO: 155;
- a heavy chain variable region of SEQ ID NO: 151 and a light chain variable region of SEQ ID NO: 155;
- a heavy chain variable region of SEQ ID NO: 152 and a light chain variable region of SEQ ID NO: 157;
- a heavy chain variable region of SEQ ID NO: 153 and a light chain variable region of SEQ ID NO: 157;
- a heavy chain variable region of SEQ ID NO: 151 and a light chain variable region of SEQ ID NO: 157;
- a heavy chain variable region of SEQ ID NO: 152 and a light chain variable region of SEQ ID NO: 155;
- a heavy chain variable region of SEQ ID NO: 184 and a light chain variable region of SEQ ID NO: 155;
- a heavy chain variable region of SEQ ID NO: 150 and a light chain variable region of SEQ ID NO: 186;
- a heavy chain variable region of SEQ ID NO: 150 and a light chain variable region of SEQ ID NO: 187;
- a heavy chain variable region of SEQ ID NO: 150 and a light chain variable region of SEQ ID NO: 188;
- a heavy chain variable region of SEQ ID NO: 150 and a light chain variable region of SEQ ID NO: 189;
- a heavy chain variable region of SEQ ID NO: 185 and a light chain variable region of SEQ ID NO: 155;
- a heavy chain variable region of SEQ ID NO: 150 and a light chain variable region of SEQ ID NO: 190;
- a heavy chain variable region of SEQ ID NO: 185 and a light chain variable region of SEQ ID NO: 186;
- a heavy chain variable region of SEQ ID NO: 185 and a light chain variable region of SEQ ID NO: 187;
- a heavy chain variable region of SEQ ID NO: 150 and a light chain variable region of SEQ ID NO: 191;
- a heavy chain variable region of SEQ ID NO: 185 and a light chain variable region of SEQ ID NO: 190;
- a heavy chain variable region of SEQ ID NO: 185 and a light chain variable region of SEQ ID NO: 191;
- a heavy chain variable region of SEQ ID NO: 79 and a light chain variable region of SEQ ID NO: 197;
- a heavy chain variable region of SEQ ID NO: 79 and a light chain variable region of SEQ ID NO: 198;
- a heavy chain variable region of SEQ ID NO: 79 and a light chain variable region of SEQ ID NO: 199; or
- a heavy chain variable region of SEQ ID NO: 79 and a light chain variable region of SEQ ID NO: 200.

In another embodiment, the scFv may include the amino acid sequence of SEQ ID NOs: 164 to 171.

The linker may be a peptide linker and may have a length of about 10-25 amino acids. For example, the linker may include, but is not limited to, hydrophilic amino acids such as glycine and/or serine.

Specifically, the linker may include, for example, (GS)ₙ, (GGS)ₙ, (GSGGS)ₙ or (GnS)ₘ (wherein n and m are each 1 to 10), but the linker may be, for example, (GₙS)ₘ (wherein n and m are each 1 to 10). Specifically, the linker may include GGGGS, for example, GGGGSGGGGSGGGGS of SEQ ID NO: 200 including three repeat units (GGGGS).

"Phage display" is a technique for displaying a mutant polypeptide as a fusion protein with at least a part of a coat protein on the surface of the particle of a phage, for example a fibrous phage. The usefulness of phage display is to rapidly and efficiently classify sequences that bind to target antigens with high affinity in large libraries of randomized protein mutants. Displaying peptides and protein libraries on phages has been used to screen millions of polypeptides in order to identify polypeptides with specific binding properties.

Phage display technology has offered a powerful tool for producing and screening novel proteins that bind to specific ligands (e.g., antigens). Using phage display technology, large libraries of protein mutants may be generated, and sequences binding with high affinity to target antigens may be rapidly classified. The nucleic acid encoding mutant polypeptides is fused with a nucleic acid sequence encoding a viral coat protein, e.g. a gene III or gene VIII protein. A monovalent phage display system, in which a nucleic acid sequence encoding a protein or polypeptide is fused with a nucleic acid sequence encoding a part of a gene III protein, has been developed. In the monovalent display system, a fused gene is expressed at a low level, and a wild-type gene III protein is also expressed, and thus particle infectivity is maintained.

It is important to demonstrate the expression of peptides on the fibrous phage surface and the expression of functional antibody fragments in the peripheral cytoplasm of *E. coli* for the development of antibody phage display libraries. Libraries of antibody- or antigen-binding polypeptides are produced through a number of methods, for example, methods of modifying a single gene by inserting a random DNA sequence or cloning a related gene sequence. Libraries can be screened regarding the expression of antibody- or antigen-binding proteins having desired characteristics.

Phage display technology has several advantages over conventional hybridomas and recombinant methods for producing antibodies having desired characteristics. This technique provides the generation of large antibody libraries with a variety of sequences within a short time without using animals. The production of hybridomas and the production of humanized antibodies may require a production time of several months. In addition, since no immunity is required, the phage antibody libraries can generate antibodies against antigens that are toxic or have low antigenicity. The phage antibody libraries can also be used to produce and identify novel therapeutic antibodies.

Techniques for generating human antibodies from immunized or non-immunized human germline sequences or naive B cell Ig repertoires using phage display libraries may be used. Naive or non-immunogenic antigen-binding libraries may be produced using various lymphatic tissues.

Techniques for identifying and separating high-affinity antibodies from phage display libraries are important for the separation of new therapeutic antibodies. The separation of high-affinity antibodies from libraries depends on the size of the libraries, the production efficiency in bacterial cells, and the variety of libraries. The size of the libraries is reduced by improper folding of the antibody- or antigen-binding protein and inefficient production due to the presence of the stop codon. Expression in bacterial cells may be inhibited by improper folding of the antibody- or antigen-binding domain. The expression may be improved by alternately mutating residues on the surface of the variable/constant interfaces or the selected CDR residues. The sequence of the framework region is an element that enables proper folding when generating antibody phage libraries in bacterial cells.

It is important to generate various libraries of antibody or antigen-binding proteins in the separation of high-affinity antibodies. CDR3 regions have often been found to participate in antigen binding. Since the CDR3 region in the heavy chain varies considerably with regard to size, sequence and structural/dimensional morphology, various libraries can be produced using the same.

In addition, diversity may be created by randomizing the CDR regions of variable heavy and light chains using all 20 amino acids at each position. The use of all 20 amino acids results in the production of antibody sequences having increased diversity and an increased chance of identifying new antibodies.

The antibody or antibody fragment of the present invention may include the sequence of the anti-CD200R1 antibody mentioned herein as well as biological equivalents thereto, as long as it can specifically recognize CD200R1. For example, additional variations may be made to the amino acid sequence of the antibody in order to further improve the binding affinity and/or other biological properties of the antibody. Such variations include, for example, deletion, insertion and/or substitution of the amino acid sequence residues of the antibody. Such amino acid variations are based on the relative similarity of amino-acid side-chain substituents, such as the hydrophobicity, hydrophilicity, charge, and size thereof. It can be seen through analysis of the size, shape and type of amino-acid side-chain substituents that all of arginine, lysine, and histidine are positively charged residues; alanine, glycine, and serine have similar sizes; and phenylalanine, tryptophan, and tyrosine have similar shapes. Thus, based on these considerations, arginine, lysine, and histidine; alanine, glycine, and serine; and phenylalanine, tryptophan, and tyrosine are considered to be biologically functional equivalents.

Taking into consideration variations having biologically equivalent activity, the antibody or a nucleotide molecule encoding the same according to the present invention is interpreted to include a sequence having substantial identity with the sequence set forth in the sequence number. The term "substantial identity" means that a sequence has a identity of at least 90%, most preferably a identity of at least 95%, at least 96%, at least 97%, at least 98%, or at least 99%, when aligning the sequence of the present invention with any other sequence so as to correspond to each other as closely as possible and analyzing the aligned sequence using algorithms commonly used in the art. Alignment methods for sequence comparison are well-known in the art. The NCBI Basic Local Alignment Search Tool (BLAST) is accessible through NCBI or the like, and can be used in conjunction with sequence analysis programs such as BLASTP, BLASM, BLASTX, TBLASTN and TBLASTX over the Internet. BLAST is available at www.ncbi.nlm.nih.gov/BLAST/. A method of comparing sequence identity using this program can be found at www.ncbi.nlm.nih.gov/BLAST/blast_help.html.

Based on this, the antibody or antigen-binding fragment thereof according to the present invention can have identity of 90%, 91%, 92%, 93%, 94%, 95%, 96%, 97%, 98%, 99%, or more with the sequences disclosed herein or the entirety thereof. Identity can be determined through sequence comparison and/or alignment by methods known in the art. For example, the percentage sequence identity of the nucleic acid or protein according to the present invention can be determined using a sequence comparison algorithm (*i.e.,* BLAST or BLAST 2.0), manual alignment, or visual inspection.

In another aspect, the present invention is directed to a nucleic acid encoding the antibody or an antigen-binding fragment thereof. By isolating the nucleic acid encoding the antibody or antigen-binding fragment thereof according to the present invention, an antibody or antigen-binding fragment thereof can be produced in a recombinant manner.

The term "nucleic acid" is intended to encompass both DNA (gDNA and cDNA) and RNA molecules, and a nucleotide, which is the basic constituent unit of nucleic acids, includes naturally derived nucleotides as well as analogues thereof, in which sugar or base moieties are modified. The sequence of the nucleic acid encoding heavy- and light-chain variable regions of the present invention can vary. Such variation includes addition, deletion, or non-conservative or conservative substitution of nucleotides.

The DNA encoding the antibody can be easily separated or synthesized using conventional procedures (for example, using an oligonucleotide probe capable of specifically binding to DNA encoding heavy and light chains of the antibody). The nucleic acid is isolated and inserted into a replicable vector, followed by further cloning (amplification of DNA) or further expression. Based thereon, in another aspect, the present invention is directed to a recombinant expression vector including the nucleic acid.

As used herein, the term "vector" refers to a means for expressing target genes in host cells, and includes plasmid vectors, cosmid vectors, and viral vectors such as bacteriophage vectors, adenovirus vectors, retroviral vectors, and adeno-associated viral vectors. Vector components generally include, but are not limited to, one or more of the following components: signal sequences, replication origins, one or more antibiotic resistance marker genes, enhancer elements, promoters, and transcription termination sequences. The nucleic acid encoding the antibody is operably linked to promoters, transcription termination sequences or the like.

The term "operably linked" means functional linkage between a nucleic acid expression regulation sequence (e.g., an array of the binding site of the promoter, signal sequence or transcription regulator) and another nucleic acid sequence, and enables the regulation sequence to regulate the transcription and/or translation of the other nucleic acid sequence.

When a prokaryotic cell is used as a host, it generally includes a potent promoter capable of conducting transcription (such as a tac promoter, lac promoter, lacUV5 promoter, lpp promoter, pLλ promoter, pRλ promoter, rac5 promoter, amp promoter, recA promoter, SP6 promoter, trp promoter, or T7 promoter), a ribosome-binding site for initiation of translation, and a transcription/translation termination sequence. In addition, for example, when a eukaryotic cell is used as a host, it includes a promoter derived from the genome of mammalian cells (e.g., a metallothionein promoter, a β-actin promoter, a human hemoglobin promoter or a human muscle creatine promoter), or a promoter derived from a mammalian virus (e.g., an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, or Rous sarcoma virus (RSV) promoter), and generally has a polyadenylation sequence as a transcription termination sequence.

Optionally, the vector may be fused with another sequence in order to facilitate purification of the antibody expressed thereby. The sequence to be fused therewith may include, for example, glutathione S-transferase (Pharmacia, USA), a maltose-binding protein (NEB, USA), FLAG (IBI, USA), 6x His (hexahistidine; Quiagen, USA) and the like.

The vector includes antibiotic resistance genes commonly used in the art as selectable markers, and examples thereof include genes conferring resistance to ampicillin, gentamycin, carbenicillin, chloramphenicol, streptomycin, kanamycin, geneticin, neomycin, and tetracycline.

In another aspect, the present invention is directed to a cell transformed with the recombinant expression vector. The cell used to produce the antibody of the present invention may be a prokaryote, yeast, or higher eukaryotic cell, but is not limited thereto.

Prokaryotic host cells such as Escherichia coli, strains of the genus Bacillus, such as Bacillus subtilis and Bacillus thuringiensis, Streptomyces spp., Pseudomonas spp. (for example, Pseudomonas putida), Proteus mirabilis and Staphylococcus spp. (for example, Staphylococcus carnosus) may be used.

Examples of animal cells include, but are not limited to, COS-7, BHK, CHO, CHOK1, DXB-11, DG-44, CHO/- DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

In another aspect, the present invention is directed to a method of producing an antibody specifically binding to CD200R1 or an antigen-binding fragment thereof, including culturing a host cell to produce an antibody, and isolating and purifying the produced antibody.

The host cells may be cultured in various media. Any commercially available medium may be used as a culture medium without limitation. All other essential supplements well-known to those skilled in the art may be included in appropriate concentrations. Culture conditions such as temperature and pH are those that are conventionally used with host cells selected for expression, as will be apparent to those skilled in the art.

The recovery of the antibody or antigen-binding fragment thereof may be carried out, for example, by centrifugation or ultrafiltration to remove impurities from and purify the resulting product using, for example, affinity chromatography. Other additional purification techniques, such as anion or cation exchange chromatography, hydrophobic interaction chromatography, and hydroxyapatite (HA) chromatography, may be used.

### Bispecific or multispecific antibodies

In another aspect, the present invention is directed to a bispecific or multispecific antibody including the antibody or antigen-binding fragment thereof.

The bispecific antibody refers to an antibody that has binding or antagonistic activity to one or more targets, and has a structure in which antibodies having binding or antagonistic abilities to two different targets bind to each other, or has a structure in which an antibody having binding ability to one target binds to a substance having antagonistic abilities to another target.

The multispecific antibody refers to an antibody that has binding specificity for at least three different antigens. The multispecific antibody may include an antibody that targets at least three targets, such as a trispecific antibody, a tetraspecific antibody, or an antibody that targets five or more targets.

The antibody belonging to the bispecific or multispecific antibody may be categorized into ScFv-based antibodies, Fab-based antibodies, and IgG-based antibodies. Bispecific or multispecific antibodies may simultaneously inhibit or amplify two or more signals and thus be more effective, compared to when a single signal is suppressed/amplified, and enables low dose administration and suppression/amplification of two or more signals in the same time and space, compared to when each signal is treated with a separate inhibitor.

Methods of producing bispecific or multispecific antibodies are well known. Traditionally, recombinant production of bispecific antibodies is based on the co-expression of two or more immunoglobulin heavy/light chain pairs, where the two or more heavy chains possess different specificities.

In the bispecific or multispecific antibodies based on scFv, the VL and VH of different scFvs may be combined with each other to produce a hybrid scFv in a heterodimeric form thereby to produce a diabody, different scFvs may be linked to each other to produce a tandem ScFv, the CH1 and CL of Fab may be expressed at the terminals of each scFv to produce a heterodimeric miniantibody, and some amino acids in the CH3 domain, the homodimeric domain of Fc, may be replaced to change the same into a "knob-into-hole" type heterodimeric structure, and these modified CH3 domains may be expressed at the terminals of different scFvs to produce a minibody in the form of a heterodimeric scFv.

For Fab-based bispecific or multispecific antibodies, individual Fabs targeting specific antigens may be combined using disulfide bonds or mediators to produce heterodimeric Fabs. By expressing scFvs targeting different antigens at the heavy or light chain ends of a specific Fab, the antigen-binding valency may be increased to two. By placing a hinge region between the Fab and scFv, a homodimeric antibody with four antigen-binding valencies may be produced. Furthermore, bispecific bibodies having three antigen-binding valencies may be produced by fusing scFvs targeting different antigens to the light and heavy chain ends of the Fab, trispecific bibodies having three antigen-binding valencies may be produced by chemically conjugating three different Fabs. Fab-based bispecific or multispecific antibodies may be produced by chemically conjugating three different Fabs.

For IgG-based bispecific or multispecific antibodies, a method known by Trion Pharma involves crossbreeding mouse and rat hybridomas to produce hybrid hybridomas, also known as "quadromas". Furthermore, bispecific antibodies may be produced in the so-called "holes and knob", wherein the light chain is shared while one or more amino acids in the CH3 homodimeric domain of the Fc are modified for different heavy chains to produce a heterodimeric form. In addition to heterodimeric bispecific antibodies, two different scFvs may be fused to the constant domains of the IgG light and heavy chains, instead of the variable domains of the IgG light and heavy chain, to produce homodimeric (scFv)4-IgG. Furthermore, ImClone reported the production of a bispecific antibody based on IMC-1C11, a chimeric monoclonal antibody targeting human VEGFR-2, by fusing only the single variable domain of mouse platelet-derived growth factor receptor-a to the light chain amino terminus of the antibody. Furthermore, antibodies with multiple antigen-binding affinities for CD20 may be produced through the so-called "dock and lock (DNL)", which uses the dimerization and docking domain (DDD) of the protein kinase A (PKA) R subunit and the anchoring domain of PKA.

A wide variety of recombinant antibody formats have been developed, including bivalent, trivalent, or tetravalent bispecific or multispecific antibodies. For example, the recombinant antibody formats also include bivalent, trivalent, or tetravalent antibodies disclosed in International Patent Application Publication Nos. WO 2001/077342, WO 2009/080251, WO 2009/080252, WO 2009/080253, WO 2009/080254, WO 2010/112193, WO 2010/115589, WO 2010/136172, WO 2010/145792, WO 2010/145793, and WO 2011/117330. Bivalent, trivalent, or tetravalent antibodies mean the presence of two or more binding domains, three or more binding domains, or four or more binding domains, respectively, in the antibody molecule.

In specific embodiments, the bispecific or multispecific antibody of the present invention may include the anti-CD200R1 antibody or antigen-binding fragment, specifically in the form of a complete IgG antibody or fragment thereof, such as a single-chain Fv, V_{H} domain, and/or V_{L} domain, Fab, or (Fab)₂.

Furthermore, the bispecific or multispecific antibody of the present invention may include the antibody targeting CD200R1 and an antibody binding to a different target, for example, an antibody targeting one or more selected from the group consisting of IL-6, TNF-α, IL-4, IL-5, TSLP, and IFN-γ, specifically in the form of a complete IgG antibody or fragment thereof, such as single-chain Fv, V_{H} domain, and/or V_{L} domain, Fab, or (Fab)₂.

The bispecific or multispecific antibodies of the present invention can achieve additional binding specificities induced or mediated by targets other than CD200R1.

For example, the bispecific antibody of the present invention may simultaneously target CD200R1 and one or more selected from the group consisting of IL-6, TNF-α, IL-4, IL-5, TSLP, and IFN-γ.

For example, the multispecific antibody of the present invention may simultaneously target CD200R1 and two or more selected from the group consisting of IL-6, TNF-α, IL-4, IL-5, TSLP, and IFN-γ.

In the present invention, the antibody or antigen-binding fragment thereof may specifically bind to CD200R1, and more preferably, specifically bind to human CD200R1.

In the present invention, the antibody or antigen-binding fragment may not cross-link to an immune-activating CD200R, such as CD200RLa.

In the present invention, the antibody or antigen-binding fragment may specifically bind to human or primate CD200R1 and not cross-link to mouse CD200R1.

In the present invention, the antibody or antigen-binding fragment may non-competitively bind to CD200, an endogenous ligand of CD200R1.

In the present invention, the antibody or antigen-binding fragment may bind to CD200R1 and act as an agonist or antagonist.

In the present invention, the antibody or antigen-binding fragment may preferably bind to CD200R1 and act as an agonist.

In the present invention, the antibody or antigen-binding fragment may activate the downstream signaling pathway of CD200-CD200R1, thereby effectively suppressing the immune response.

### Chimeric antigen receptor (CAR)

In another aspect, the present invention is directed to a chimeric antigen receptor (CAR) including the antigen-binding fragment.

The chimeric antigen receptor (CAR) is a synthetic construct designed to induce an immune response (immune activation or suppression) against a target antigen and a cell expressing the antigen. The CAR may include an extracellular domain including the antigen-binding fragment of the present invention, a transmembrane domain, and an intracellular signaling domain. The CAR may further include a hinge domain and/or a co-stimulatory domain.

The CAR may include the CDRs, light chain, and/or heavy chain of an anti-CD200R1 antibody of the present invention as the antigen recognition site of the extracellular domain.

In one embodiment, the antigen-binding fragment of the extracellular domain may be an scFv. In an scFv including the VH and VL domains of an antibody, the VH and VL domains may be linked via a linker.

The linker may be a peptide linker and may be approximately 10-25 amino acids in length. For example, the linker may include a hydrophilic amino acid such as glycine and/or serine.

The linker may include, for example, (GS)ₙ, (GGS)ₙ, (GSGGS)ₙ, or (GₙS)ₘ (where n and m are each 1 to 10), but the linker may be, for example, (GₙS)ₘ (where n and m are each 1 to 10). Specifically, the linker may include GGGGS and may, for example, be GGGGSGGGGSGGGGS of SEQ ID NO: 200 including three repeat units.

In the present invention, the hinge domain may be selected from, for example, the extracellular domain of CD8, CD4, or CD28, the Fc region of an IgG1 antibody, or the extracellular domain of any TLR receptor known to those skilled in the art.

The transmembrane domain may be derived from a natural or synthetic source. When the source is natural, the domain may be derived from any membrane-bound protein or transmembrane protein. The transmembrane domain may include the alpha, beta, or zeta chain of T-cell receptor, CD2, CD28, CD3 epsilon, CD45, CD4, CD5, CD8, CD9, CD16, CD22, CD33, CD37, CD64, CD80, CD86, CD134, CD137, CD154, or ICOS. When the transmembrane domain is synthesized, it may contain hydrophobic residues such as leucine and valine, or peptides containing phenylalanine, tryptophan, and valine at each terminus. A short oligo- or polypeptide linker, 2 to 10 amino acids long, may form a bond between the transmembrane domain and the cytoplasmic signaling domain of the CAR. A glycine-serine peptide may be used as the linker.

The intracellular signaling domain may induce activation of the immune activation or suppression function of an immune cell modified to express the CAR. The signaling domain may include a truncated fragment of the intracellular signaling domain sufficient to transduce an immune suppression signal.

The signaling domain may induce immune activation or suppression function of an immune cell modified to express the CAR. The signaling domain may include a truncated fragment of an intracellular signaling domain sufficient to transduce an immunosuppressive signal.

For example, the signaling domain regulates primary activation of the TCR complex in a stimulatory or inhibitory manner. A stimulatory primary cytoplasmic signaling sequence may contain a signaling motif known as an immunoreceptor tyrosine-based activation motif, or ITAM. Examples of ITAMs containing a primary cytoplasmic signaling sequence include TCR zeta, FcR gamma, FcR beta, CD3 gamma, CD3 delta, CD3 epsilon, CD5, CD22, CD79a, CD79b, and CD66d.

In some cases, the cytoplasmic domain of the CAR may include a CD3 zeta chain portion and a costimulatory signaling domain. The costimulatory signaling domain refers to a portion of a CAR that includes the intracellular domain of a costimulatory molecule. For example, the costimulatory signaling domain may include ligands that specifically bind to CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, and CD83.

For another example, the signaling domain may include an Fc signaling domain. The Fc signaling domain may be any one of the Fc-alpha, Fc-gamma, Fc-epsilon, Fc-mu, and Fc-delta receptors. For example, an Fc receptor signaling domain may include amino acids involved in interaction with Src (e.g., Fgr, Fyn, Hck, Lyn, Yes, and Src) and one or more ITAM domains from a ZAP-70 family kinase (see, e.g., Sanchez-Mejorada et al. (1998) J. Leukocyte Biol. 63:531; Garcia-Garcia et al. (2002) J. Leukocyte Biol. 72:1092). In some embodiments, an Fc receptor signaling domain may, for example, include at least one ITAM domain selected from Fc-alpha, Fc-gamma, Fc-epsilon, Fc-mu, and Fc-delta receptors, or an ITAM domain substantially identical thereto.

The cytoplasmic signaling sequence within the cytoplasmic signaling portion of the CAR may be linked via a peptide linker containing 2 to 10 amino acids, for example, a glycine-serine sequence.

In the present invention, the nucleic acid encoding the CAR may be introduced to be expressed in immune cells, preferably regulatory T cells.

The type of each domain of the CAR for use in the production of CAR-Treg may be easily selected from those known in the art (e.g., Cellular Immunology, 104220, etc.).

In another aspect, the present invention is directed to an immune cell containing the chimeric antigen receptor (CAR) introduced thereinto.

The immune cells may be selected depending on the desired therapeutic effect. For example, when the antibody or antigen-binding moiety of the present invention has an inhibitory effect for CD200R1, the immune cell may be selected from the group consisting of immune-activated cells, such as T cells, NK cells, cytokine-induced killer cells (CIKs), activated cytotoxic T lymphocytes (CTLs), macrophages, tumor-infiltrating lymphocytes (TILs), and dendritic cells. When the antibody or antigen-binding moiety of the present invention has an agonistic effect for CD200R1, the immune cell may be a regulatory T cell, but is not limited thereto.

### Composition for use in treatment

In another aspect, the present invention is directed to a composition for use in preventing or treating a disease, containing the antibody or antigen-binding fragment thereof, the bispecific or multispecific antibody including the antibody or antigen-binding fragment thereof, the chimeric antigen receptor including the antibody or antigen-binding fragment thereof, and/or the immune cell containing the chimeric antigen receptor.

In the present invention, when the antibody or antigen-binding fragment thereof, a bispecific or multispecific antibody containing the antibody or antigen-binding fragment thereof, or the antibody or antigen-binding fragment thereof exhibits an antagonist effect, the disease may be a disease for which a therapeutic effect is expected through immune activation, such as an infectious disease or cancer, but is not limited thereto.

In the present invention, when the antibody or antigen-binding fragment thereof, a bispecific or multispecific antibody including the antibody or antigen-binding fragment thereof, or the antibody or antigen-binding fragment thereof exhibits an agonist effect, the disease may be a disease for which a therapeutic effect is expected through immune suppression, such as, an immune disease or an inflammatory disease, but is not limited thereto.

As used herein, the term "immune disease" refers to a disease that may be directly caused by an abnormality in the immune system, and may be selected from the group consisting of dermatitis, allergies, rhinitis, gout, ankylosing spondylitis, rheumatic fever, lupus, fibromyalgia, tendonitis, type 1 diabetes, scleroderma, neurodegenerative disease, type 2 diabetes, silicosis, atherosclerosis, vitiligo, conjunctivitis, and autoimmune disease, but is not limited thereto.

As used herein, the term "autoimmune disease" refers to a disease that occurs when immune cells in an organism recognize the organism's own tissues or cells, rather than an external invading antigen, as an antigen and attack the same. The autoimmune disease may include at least one selected from the group consisting of rheumatoid arthritis, systemic sclerosis (scleroderma), insulin-dependent juvenile diabetes mellitus caused by pancreatic islet cell antibodies, alopecia areata, psoriasis, pemphigus, asthma, aphthous stomatitis, chronic thyroiditis, some forms of acquired aplastic anemia, primary biliary cirrhosis, ulcerative colitis, Behçet's disease, Crohn's disease, silicosis, asbestosis, IgA nephropathy, post-streptococcal glomerulonephritis, Sjögren's syndrome, Guillain-Barré syndrome, dermatomyositis, polymyositis, multiple sclerosis, autoimmune hemolytic anemia, autoimmune encephalomyelitis, myasthenia gravis, Graves' disease (Graves' hyperthyroidism), polyarteritis nodosa, ankylosing spondylitis, fibrositis, temporal arteritis, Wilson's disease, fanconi syndrome, multiple myeloma, and systemic lupus erythematosus, but is not limited thereto.

As used herein, the term "inflammatory disease" is a generic term for a disease accompanied by inflammation as a main main cause and/or lesion, particularly one selected from the group consisting of edema, allergies, asthma, conjunctivitis, periodontitis, rhinitis, otitis media, sore throat, tonsillitis, pneumonia, gastric ulcer, gastritis, Crohn's disease, colitis, hemorrhoids, gout, ankylosing spondylitis, rheumatic fever, lupus, fibromyalgia, psoriatic arthritis, osteoarthritis, rheumatoid arthritis, periarthritis of the shoulder, tendinitis, tenosynovitis, myositis, hepatitis, cystitis, nephritis, Sjogren's syndrome, severe myasthenia gravis, and multiple sclerosis, but is not limited thereto.

As used herein, the term "prevention" or "prophylactic" refers to any action that can suppress or delay the onset of an immune disease or inflammatory disease by administration of the pharmaceutical composition according to the present invention.

As used herein, the term "treatment" or "therapeutic" refers to any action that can ameliorate or beneficially alter the symptoms of an immune disease or inflammatory disease by administration of the pharmaceutical composition according to the present invention.

The pharmaceutical composition according to the present invention may be administered orally or parenterally. The parenteral administration may be intravenous injection, subcutaneous injection, intramuscular injection, intraperitoneal injection, endothelial administration, topical administration, intranasal administration, pulmonary administration, rectal administration, or the like.

Upon oral administration, since proteins or peptides are digested, an oral composition should be coated with an active drug or formulated so as to protect the same from degradation in the stomach. In addition, the pharmaceutical composition may be administered using any device capable of delivering the active substance to target cells.

The suitable dose of the pharmaceutical composition according to the present invention may vary depending on factors such as the formulation method, administration method, and age, body weight, gender, pathological conditions, diet, administration time, administration route, excretion rate, and responsiveness of the patient, and a general physician of ordinary skill can easily determine and prescribe a dose effective for the desired treatment or prevention.

The pharmaceutical composition according to the present invention may be prepared into a unit dose form, or may be incorporated into a multi-dose container through formulation using a pharmaceutically acceptable carrier and/or excipient according to a method that can be easily implemented by those skilled in the art to which the present invention pertains. Here, the formulation may be in the form of a solution, a suspension or an emulsion in an oil or aqueous medium, or may be in the form of an extract, a powder, a suppository, a granule, a tablet, or a capsule. The composition may further contain a dispersant or a stabilizer.

### Treatment method

In another aspect, the present invention is directed to a composition for use in or treating an immune or inflammatory disease, containing the antibody or antigen-binding fragment thereof, the bispecific or multispecific antibody containing the antibody or antigen-binding fragment thereof, the chimeric antigen receptor containing the antibody or antigen-binding fragment thereof, and/or the immune cell containing the chimeric antigen receptor.

In another aspect, the present invention is directed to a method of treating an immune disease or inflammatory disease, including administering the antibody or antigen-binding fragment thereof, the bispecific or multispecific antibody, the chimeric antigen receptor, and/or the immune cell to a subject.

In another aspect, the present invention is directed to the use of the antibody or antigen-binding fragment thereof, the bispecific or multispecific antibody including the antibody or antigen-binding fragment thereof, the chimeric antigen receptor, or the immune cell containing the chimeric antigen receptor for the prevention or treatment of an immune disease or inflammatory disease.

In another aspect, the present invention is directed to the use of the antibody or antigen-binding fragment thereof, the bispecific or multispecific antibody including the antibody or antigen-binding fragment thereof, the chimeric antigen receptor containing the antibody or antigen-binding fragment thereof, or the immune cell containing the chimeric antigen receptor for the preparation of a drug for preventing or treating an immune disease or inflammatory disease.

### Combination therapy

In another aspect, the present invention is directed to a use for combination therapy of the antibody or antigen-binding fragment thereof, the bispecific or multispecific antibody containing the antibody or antigen-binding fragment thereof, the chimeric antigen receptor, or the immune cell containing the chimeric antigen receptor.

The antibody or antigen-binding fragment thereof, the bispecific or multispecific antibody including the antibody or antigen-binding fragment thereof, the chimeric antigen receptor, or the immune cell containing the chimeric antigen receptor may be administer in combination with another active ingredient.

Examples of various drugs that may be used for immune or inflammatory diseases are well known in the art, and those skilled in the art can easily select and use drugs for combined administration based on this knowledge.

### Example

Hereinafter, the present invention will be described in more detail with reference to examples. However, it will be obvious to those skilled in the art that these examples are provided only for illustration of the present invention, and should not be construed as limiting the scope of the present invention.

### Example 1: Preparation of Phage Library (scFv)

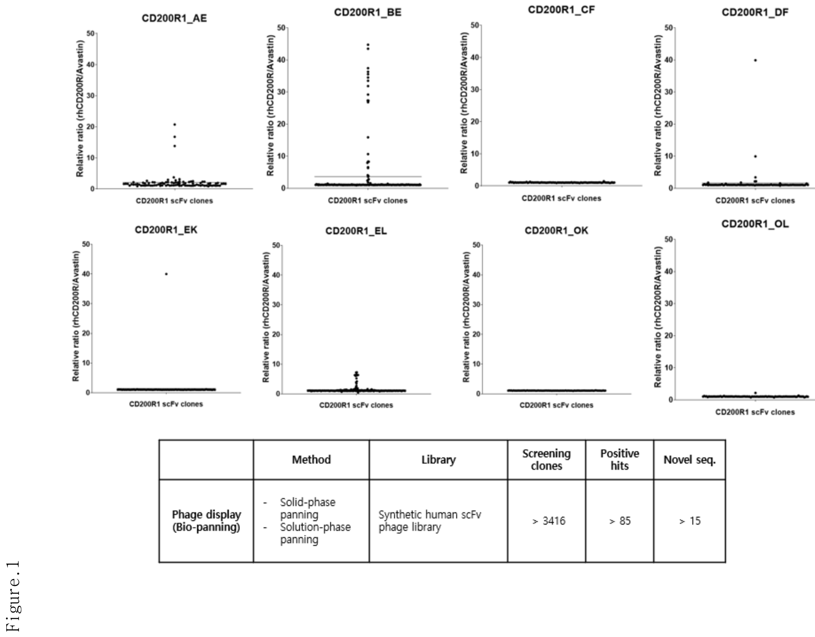

To recover a phage form from a previously constructed phagemid vector carrying human-derived synthetic scFv library genes, six sublibraries sample*((1)Bai, Xuelian & Kim, Jihye & Kang, Seungmin &* Kim, Wankyu & Shim, Hyunbo. (2015). A Novel Human scFv Library with Non-Combinatorial Synthetic CDR Diversity. PloS one. 10. e0141045. 10.1371/journal.pone.0141045*., (2)* Yang, Hye & Kang, Kyung & TCW, Julia & Shim, Hyunbo. (2009). Construction of a large synthetic human scFv library with six diversified CDRs and high functional diversity. Molecules and cells. 27. 225-35. 10.1007/s10059-009-0028-9*.)* were cultured in 400 ml of culture medium (SB/ampicillin/2% glucose) for 2 hours. When the absorbance at OD₆₀₀ reached 0.5 to 0.7, the supernatant was removed by centrifugation at 5,000 g for 20 minutes, and 10¹² pfu (plaque forming unit) of helper phage (VCSM13) was added to 400 ml of secondary culture medium (SB/ampicillin) and re-cultured for 1 hour. Then, kanamycin antibiotic (antibiotic gene introduced into helper phage) was added at a concentration of 70 *µ*g/mℓ and the mixture was shaken and cultured at 30°C and 250 rpm for 16 hours to produce a phage library outside the host cells. The culture product was then centrifuged, and PEG 8000 (polyethylene glycol 8000) and NaCl were added to the supernatant, followed by stirring at 4°C for 2 hours to precipitate the recombinant phage. The pellet was centrifuged at 12,000 g and at 4°C for 30 minutes, and PBS was added to the precipitated phage to suspend the precipitated phage. PEG 8000 (polyethylene glycol 8000) and NaCl were then added and the pellet was centrifuged at 15,000 g and at 4°C for 30 minutes. PBS was then added to the precipitated phage pellet to recover the phage library. The concentration of the amplified sublibrary was calculated by diluting the recovered phage, infecting TG1 cells therewith, and culturing the result on LB/ampicillin solid culture medium. The number of colonies generated was calculated (FIG. 1).

### Example 2: Screening of anti-CD200R1-specific antibodies (scFv) using biopanning

Phage display panning was performed for screening human antibodies that specifically bind to CD200R1.

Biopanning was performed using the CD200R1-Fc protein as follows. The recombinant antigen used herein was recombinant human CD200R1 Fc chimera protein, CF (R&D systems, 3414-CD).

antigen-immobilized Biopanning: 1 mL each of human CD200R1 Fc protein and negative Fc protein were coated separately in 5 mL immunotubes at 4 °C for 16 hours, and blocked with 3% skim milk. After emptying the tube, the library (approximately 2.0 x 10¹³ pfu) was added to the negative Fc protein-coated tube, followed by incubation at room temperature for 1 hour. This is a step of removing phages that bind to proteins other than human CD200R1 from the antibody phage library, to prevent nonspecific binding to a target other than CD200R1.

Phages that did not bind to the negative Fc protein were recovered and allowed to bind to a human CD200R1 Fc-coated plate for 1 hour. The result was washed 3 to 9 times with PBST (Phosphate-buffered saline-0.05% Tween 20) to remove nonspecific binding and human CD200R1-specific phage antibodies were recovered using IgG Elution Buffer (Thermo Scientific, 21028, Pierce^{™} IgG Elution Buffer, pH 2.0). The panning was performed four times. The results of antigen-fixed biopanning for rounds 1 to 4 are shown in Tables 1 to 5.

**[Table 1]**

| Round 1 | Input (cfu/mL) | Output (cfu/mL) | Recovery rate (Output/Input) |
|---|---|---|---|
| Library1 | 4.28E+12 | 3.60E+06 | 8.42E-07 |
| Library2 | 1.33E+12 | 2.57E+06 | 1.93E-06 |
| Library3 | 2.14E+12 | 1.14E+07 | 5.32E-06 |
| Library4 | 1.34E+13 | 5.75E+06 | 4.29E-07 |
| Library5 | 2.54E+13 | 2.23E+07 | 8.77E-07 |
| Library6 | 9.11E+12 | 4.48E+06 | 4.91E-07 |
| Library7 | 1.26E+13 | 9.91E+06 | 7.85E-07 |
| Library8 | 1.13E+13 | 1.63E+07 | 1.45E-06 |

**[Table 2]**

| Round 2 | Input | Output | Recovery rate |
|---|---|---|---|
| | (cfu/mL) | (cfu/mL) | (Output/Input) |
| Library1 | 2.84E+12 | 2.70E+04 | 9.51E-09 |
| Library2 | 4.20E+12 | 4.10E+04 | 9.76E-09 |
| Library3 | 5.00E+12 | 8.00E+04 | 1.60E-08 |
| Library4 | 6.46E+12 | 1.90E+05 | 2.94E-08 |
| Library5 | 6.07E+12 | 3.35E+04 | 5.52E-09 |
| Library6 | 1.97E+12 | 1.20E+04 | 6.10E-09 |
| Library7 | 5.67E+12 | 4.85E+04 | 8.55E-09 |
| Library8 | 3.39E+12 | 2.55E+04 | 7.52E-09 |

**[Table 3]**

| Round 3 | Input (cfu/mL) | Output (cfu/mL) | Recovery rate (Output/Input) |
|---|---|---|---|
| Library1 | 3.55E+12 | 2.36E+05 | 6.63E-08 |
| Library2 | 2.41E+12 | 2.00E+04 | 8.31E-09 |
| Library3 | 2.61E+12 | 5.35E+04 | 2.05E-08 |
| Library4 | 2.89E+12 | 3.04E+05 | 1.05E-07 |
| Library5 | 2.48E+12 | 1.07E+05 | 4.32E-08 |
| Library6 | 5.57E+12 | 5.90E+04 | 1.06E-08 |
| Library7 | 2.87E+12 | 9.65E+05 | 3.37E-07 |
| Library8 | 3.51E+12 | 1.29E+05 | 3.68E-08 |

**[Table 4]**

| Round 4 | Input (cfu/mL) | Output (cfu/mL) | Recovery rate (Output/Input) |
|---|---|---|---|
| Library1 | 2.39E+12 | 4.03E+06 | 1.69E-06 |
| Library2 | 4.02E+12 | 2.43E+06 | 6.05E-07 |
| Library3 | 1.75E+12 | 3.28E+06 | 1.87E-06 |
| Library4 | 1.08E+12 | 1.12E+06 | 1.03E-06 |
| Library5 | 1.54E+12 | 2.70E+04 | 1.76E-08 |
| Library6 | 1.44E+12 | 2.10E+05 | 1.46E-07 |
| Library7 | 2.21E+12 | 5.77E+06 | 2.61E-06 |
| Library8 | 3.33E+12 | 2.40E+06 | 7.19E-07 |

**[Table 5]**

| Recovery rate (Output/I nput) | Libra ry1 | Libra ry2 | Libra ry3 | Libra ry4 | Libra ry5 | Libra ry6 | Libra ry7 | Libra ry8 |
|---|---|---|---|---|---|---|---|---|
| Round 1 | 8.42E -07 | 1.93E -06 | 5.32E -06 | 4.29E -07 | 8.77E -07 | 4.91E -07 | 7.85E -07 | 1.45E -06 |
| Round 2 | 9.51E -09 | 9.76E -09 | 1.60E -08 | 2.94E -08 | 5.52E -09 | 6.10E -09 | 8.55E -09 | 7.52E -09 |
| Round 3 | 6.63E -08 | 8.31E -09 | 2.05E -08 | 1.05E -07 | 4.32E -08 | 1.06E -08 | 3.37E -07 | 3.68E -08 |
| Round 4 | 1.69E -06 | 6.05E -07 | 1.87E -06 | 1.03E -06 | 1.76E -08 | 1.46E -07 | 2.61E -06 | 7.19E -07 |

### Example 3: Affinity ELISA screening and sequence analysis of anti-CD200R1-specific antibody (scFv)

ScFv screening was performed to select monoclonal antibodies that specifically bind to CD200R1 from the phages recovered after the fourth round of panning. Colonies from the final round were harvested and inoculated into 96-well plates containing 200 µL of SB/ampicillin culture medium and incubated at 37°C for 3 to 4 hours. To induce scFv-pIII protein expression, each well was treated with 1 mM IPTG (Isopropyl β-D-1-thiogalactopyranoside) and incubated at 30°C for 16 hours. The cultured plates were centrifuged at 4,000 rpm for 15 minutes to remove the supernatant and 40 µl of TES (50 mM Tris, 1 mM EDTA, 20% Sucrose, pH 8.0) solution was added to each well to recover phage particles by lysing the cells for 30 minutes at room temperature. Then, the cells were treated with 60 µl of 0.2XTES solution and incubated at 4°C for 2 hours to lyse the cells. The plates were then centrifuged at 4,000 rpm for 15 minutes to recover the supernatant.

The supernatant was added to each well of a 96-well plate coated with human CD200R1 (R&D systems, 3414-CD) and negative Fc protein to induce binding for 2 hours at room temperature, and then the result was washed four times with 0.05% PBST and distilled water. Then, the antibody was bound to the HA tag using an HRP-conjugated anti-HA antibody for 1 hour at room temperature, and then washed four times with PBST and distilled water.

The color was developed with TMB Substrate solution (Thermo Scientific, 34029, 1-Step^{™} Ultra TMB-ELISA Substrate Solution). The color reaction was stopped with a stop solution (Invitrogen, SS04, ELISA Stop Solution). The absorbance was measured at an optical density of 450 nm. 15 antibody clones that bound only to human CD200R1 but not to the negative Fc protein were screened using ELISA. The CDR sequences of each antibody are shown in Tables 6 and 7, and the amino acid sequences of the heavy and light chain variable regions are shown in Tables 8 and 9.

**[Table 6]**

| | CDR H1 | | CDR H2 | | CDR H3 | |
|---|---|---|---|---|---|---|
| | Sequenc e | SEQ ID NO. | Sequenc e | SEQ ID NO. | Sequenc e | SEQ ID NO. |
| P001001 | **SYDMH** | 1 | RISFTGS TSYNPSL KS | 9 | RDTWWEG YFDL | 22 |
| P001002 | **SYAMS** | 2 | WISYDSS SKYYADS VKG | 10 | RGVVQPY LWTEFDY | 23 |
| P001003 | **DYAMS** | 3 | AISHGGS SKYYADS VKG | 11 | RGPEVML EFDY | 24 |
| P001004 | **DYYMS** | 4 | GIYPGDG SIYYADS VKG | 12 | KGLDLYE PMAFDY | 25 |
| P001005 | **GYDMS** | 5 | QIYPGNG SKYYADS VKG | 13 | KDLQTWT SRRFDY | 26 |
| P001006 | **DYAMS** | 3 | ISYGNGN TYYADSV KG | 14 | KDPWQTA INDFDY | 27 |
| P001007 | **NYDMS** | 6 | ISYDNGN TYYADSV KG | 15 | RNRAWFD Y | 28 |
| P001008 | **SYDMS** | 7 | ISHGGGS IYYADSV KG | 16 | RDAPALM HSLFDY | 29 |
| P001009 | **SYDMS** | 7 | ISHGGGS IYYADSV KG | 16 | RDAPAYS APLFDY | 30 |
| P001010 | **NYAMS** | 8 | ISPGGGS IYYADSV KG | 17 | RDPGQMF WPLFDY | 31 |
| P001011 | **DYYMS** | 4 | ISSGGGS TYYADSV KG | 18 | RVRGTCN GTLCYYS NAMDV | 32 |
| P001012 | **GYDMS** | 5 | ISSSGGN KYYADSV KG | 19 | RAIRPCI SAPCYYD DAMDV | 33 |
| P001013 | **GYDMS** | 5 | IPPGDGS TYYADSV KG | 20 | KHLVSDH PPSQYSD DGMDV | 34 |
| P001015 | **SYDMS** | 7 | ISHGGGS IYYADSV KG | 16 | RDAPAYS APLFDY | 30 |
| P001016 | **GYDMS** | 5 | ISYDGGS KYYADSV KG | 21 | KDGYPLG AYYFDY | 35 |

**[Table 7]**

| | CDR L1 | | CDR L2 | | CDR L3 | |
|---|---|---|---|---|---|---|
| | Sequenc e | SEQ ID NO. | Sequenc e | SEQ ID NO. | Sequenc e | SEQ ID NO. |
| P001001 | CSGSSSN IGNNHVS | 36 | **DTDRLTS** | 48 | GTWDSSL SAYV | 59 |
| P001002 | CSGSSSN IGSNYVN | 37 | **SDSHRPS** | 49 | GSWDDSL SGYV | 60 |
| P001003 | CTGSSSN IGNNAVT | 38 | **SDNHRPS** | 50 | AAWDSSL SGYV | 61 |
| P001004 | CSGSSSN IGSNYVN | 37 | **SNSHRPS** | 51 | GTWDDSL NGYV | 62 |
| P001005 | CSGSSSN IGNNDVN | 39 | **SNSHRPS** | 51 | GTWDDSL NGYV | 62 |
| P001006 | CTGSSSN IGNNSVN | 40 | **TNSHRPS** | 52 | GTWDSSL SGYV | 63 |
| P001007 | CSGSSSN IGNNNVY | 41 | **DSQRPS** | 53 | AAWDASL NGYV | 64 |
| P001008 | CSGSSSN IGSNHVN | 42 | **DSQRPS** | 53 | GSWDASL NGYV | 65 |
| P001009 | CSGSSSN IGNNAVN | 43 | **DSNRPS** | 54 | AAWDDSL SGYV | 66 |
| P001010 | CTGSSSN IGSNNVS | 44 | **DDSHRPS** | 55 | GSWDASL NGYV | 65 |
| P001011 | CTGSSSN IGSNDVS | 45 | **DDNHRPS** | 56 | GTWDASL NAYV | 67 |
| P001012 | CTGSSSN IGSNYVS | 46 | **DSQRPS** | 53 | GSWDYSL SAYV | 68 |
| P001013 | CSGSSSN IGNNDVN | 39 | **NSQRPS** | 57 | GSWDYSL NGYV | 69 |
| P001015 | CSGSSSN IGNNAVN | 43 | **DSNRPS** | 54 | AAWDDSL SGYV | 66 |
| P001016 | CSVSSSN IGSNYVS | 47 | **DSKRPS** | 58 | GTWDASL NGYV | 70 |

**[Table 8]**

| | VH Sequence | SEQ ID NO. |
|---|---|---|
| P001001 | EVQLLESGGGLVQPGGSLRLSCAASGFTFSSYDMHWVRQAPGKGL EWVSRISFTGSTSYNPSLKSRFTISRDNSKNTLYLQMNSLRAEDT AVYYCARDTWWEGYFDLWGQGTLVTVSS | 71 |
| P001002 | EVQLLESGGGLVQPGGSLRLSCAASGFTFSSYAMSWVRQAPGKGL EWVSWISYDSSSKYYADSVKGRFTISRDNSKNTLYLQMNSLRAED TAVYYCARGVVQPYLWTEFDYWGQGTLVTVSS | 72 |
| P001003 | EVQLLESGGGLVQPGGSLRLSCAASGFTFSDYAMSWVRQAPGKGL EWVSAISHGGSSKYYADSVKGRFTISRDNSKNTLYLQMNSLRAED TAVYYCARGPEVMLEFDYWGQGTLVTVSS | 73 |
| P001004 | EVQLLESGGGLVQPGGSLRLSCAASGFTFSDYYMSWVRQAPGKGL EWVSGIYPGDGSIYYADSVKGRFTISRDNSKNTLYLQMNSLRAED AAVYYCAKGLDLYEPMAFDYWGQGTLVTVSS | 74 |
| P001005 | EVQLLESGGGLVQPGGSLRLSCAASGFTFSGYDMSWVRQAPGKGL EWVSQIYPGNGSKYYADSVKGRFTISRDNSKNALYLQMNSLRAED TAVYYCAKDLQTWTSRRFDYWGQGTLVTVSS | 75 |
| P001006 | EVQLLESGGGLVQPGGSLRLSCAASGFTFSDYAMSWVRQAPGKGL EWVSGISYGNGNTYYADSVKGRFTISRDNSKNTLYLQMNSLRAED TAVYYCAKDPWQTAINDFDYWGQGTLVTVSS | 76 |
| P001007 | EVQLLESGGGLVQPGGSLRLSCAASGFTFSNYDMSWVRQAPGKGL EWVSAISYDNGNTYYADSVKGRFTISRDNSKNTLYLQMNSLRAED TAVYYCARNRAWFDYWGQGTLVTVSS | 77 |
| P001008 | EVQLLESGGGLVQPGGSLRLSCAASGFTFSSYDMSWVRQAPGKGL EWVSWISHGGGSIYYADSVKGRFTISRDNSKNTLYLQMNSLRAED TAVYYCARDAPALMHSLFDYWGQGTLVTVSS | 78 |
| P001009 | EVQLLESGGGLVQPGGSLRLSCAASGFTFSSYDMSWVRQAPGKGL EWVSWISHGGGSIYYADSVKGRFTISRDNSKNTLYLQMNSLRAED TAVYYCARDAPAYSAPLFDYWGQGTLVTVSS | 79 |
| P001010 | EVQLLESGGGLVQPGGSLRLSCAASGFTFSNYAMSWVRQAPGKGL EWVSSISPGGGSIYYADSVKGRFTISRDNSKNTLYLQMNSLRAED TAVYYCARDPGQMFWPLFDYWGQGTLVTVSS | 80 |
| P001011 | EVQLLESGGGLVQPGGSLRLSCAASGFTFSDYYMSWVRQAPGKGL EWVSWISSGGGSTYYADSVKGRFTISRDNSKNTLYLQMNSLGAED TAVYYCARVRGTCNGTLCYYSNAMDVWGQGTLVTVSS | 81 |
| P001012 | EVQLLESGGGLVQPGGSLRLSCAASGFTFSGYDMSWVRQAPGKGL EWVSGISSSGGNKYYADSVKGRFTISRDNSKNTLYLQMNSLRAED TAVYYCARAIRPCISAPCYYDDAMDVWGQGTLVTVSS | 82 |
| P001013 | EVQLLESGGGLVQPGGSLRLSCAASGFTFSGYDMSWVRQAPGKGL EWVSGIPPGDGSTYYADSVKGRFTISRDNSKNTLYLQMNSLRAED TAVYYCAKHLVSDHPPSQYSDDGMDVWGQGTLVTVSS | 83 |
| P001015 | EVQLLESGGGLVQPGGSLRLSCAASGFTFSSYDMSWVRQVPGKGL EWVSWISHGGGSIYYADSVKGRFTISRDNSKNTLYLQMNSLRAED TAVYYCARDAPAYSAPLFDYWGQGTLVTVSS | 84 |
| P001016 | EVQLLESGGGLVQPGGSLRLSCAASGFTFSGYDMSWVRQAPGKGL EWVSGISYDGGSKYYADSVKGRFTISRDNSKNTLYLQMNSLRAED TAVYYCAKDGYPLGAYYFDYWGQGTLVTVSS | 85 |

**[Table 9]**

| | VL Sequence | SEQ ID NO. |
|---|---|---|
| P001001 | QSVLTQPPSASGTPGQRVTISCSGSSSNIGNNHVSWYQQLPGTAP KLLIYDDTDRLTSVPNRFSGSKSGTSASLAISGLRSEDEADYYCG TWDSSLSAYVFGGGTKLTVL | 86 |
| P001002 | QSVLTQPPSASGTPGQRVTISCSGSSSNIGSNYVNWYQQLPGTAP KLLIYSDSHRPSGVPDRFSGSKSGTSASLAISGLRSEDEADYYCG SWDDSLSGYVFGGGTKLTVL | 87 |
| P001003 | QSVLTQPPSASGTPGQRVTISCTGSSSNIGNNAVTWYQQLPGTAP KLLIYSDNHRPSGVPDRFSGSKSGTSASLAISGLRSEDEADYYCA AWDSSLSGYVFGGGTKLTVL | 88 |
| P001004 | QSVLTQPPSASGTPGQRVTISCSGSSSNIGSNYVNWYQQLPGTAP KLLIYSNSHRPSGVPDRFSGSKSGTSASLAISGLRSEDGADYYCG TWDDSLNGYVFGGGTKLTVL | 89 |
| P001005 | QSVLTQPPSASGTPGQRVTISCSGSSSNIGNNDVNWYQQLPGTAP KLLIYSNSHRPSGVPDRFSGSKSGTSASLAISGLRSEDGADYYCG TWDDSLNGYVFGGGTKLTVL | 90 |
| P001006 | QSVLTQPPSASGTPGQRVTISCTGSSSNIGNNSVNWYQQLPGTAP KLLIYTNSHRPSEVPDRFSGSKSGTSASLAISGLRSEDEADYYCG TWDSSLSGYVFGGGTKLTVL | 91 |
| P001007 | QSVLTQPPSASGTPGQRVTISCSGSSSNIGNNNVYWYQQLPGTAP KLLIYYDSQRPSGVPDRFSGSKSGTSASLAISGLRSEDEADYYCA AWDASLNGYVFGGGTKLTVL | 92 |
| P001008 | QSVLTQPPSASGTPGQRVTISCSGSSSNIGSNHVNWYQQLPGTAP KLLIYYDSQRPSGVPDRFSGSKSGTSASLAISGLRSEDEADYYCG SWDASLNGYVFGGGTKLTVL | 93 |
| P001009 | QSVLTQPPSASGTPGQRVTISCSGSSSNIGNNAVNWYQQLPGTAP KLLIYYDSNRPSGVPDRFSGSKSGTSASLAISGLRSEDEADYYCA AWDDSLSGYVFGGGTKLTVL | 94 |
| P001010 | QSVLTQPPSASGTPGQRVTISCTGSSSNIGSNNVSWYQQLPGTAP KLLIYDDSHRPSGVPDRFSGSKSGTSASLAISGLRSEDEADYYCG SWDASLNGYVFGGGTKLTVL | 95 |
| P001011 | QSVLTQPPSASGTPGQRVTISCTGSSSNIGSNDVSWYQQLPGTAP KLLIYDDNHRPSGVPDRFSGSKSGTSASLAISGLRSEDEADYYCG TWDASLNAYVFGGGTKLTVL | 96 |
| P001012 | QSVLTQPPSASGTPGQRVTISCTGSSSNIGSNYVSWYRQLPGTAP KLLIYSDSQRPSGVPDRFSGSKSGTSASLAISGLRSEDEADYYCG SWDYSLSAYVFGGGTKLTVL | 97 |
| P001013 | QSVLTQPPSASGTPGQRVTISCSGSSSNIGNNDVNWYQQLPGTAP KLLIYYNSQRPSGVPDRFSGSKSGTSASLAISGLRSEDEADYYCG SWDYSLNGYVFGGGTKLTVL | 98 |
| P001015 | QSVLTQPPSASGTPGQRVTISCSGSSSNIGNNAVNWYQQLPGTAP KLLIYYDSNRPSGVPDRFSGSKSGTSASLAISGLRSEDEADYYCA AWDDSLSGYVFGGGTKLTVL | 94 |
| P001016 | QSVLTQPPSASGTPGQRVTISCSVSSSNIGSNYVSWYRQLPGTAP KLLIYDDSKRPSGVPDRFSGSKSGTSASLAISGLRSEDEADYYCG TWDASLNGYVFGGGTKLTVL | 99 |

### Example 4: CD200R1-specific binding ability analysis of anti-CD200R1 antibodies discovered by phage display (ELISA)

96-well plates were coated with human CD200R1 (R&D systems, 3414-CD) and mouse CD200R1 protein (R&D systems, 2554-CD) at a concentration of 2 µg/mL at 4°C for 16 hours and then were blocked with 3% skim milk. The result was treated with the antibodies at concentrations of 300 nM, 60 nM, 12 nM, 2.4 nM, 0.48 nM, 0.096 nM, and 0.0192 nM, and reacted at room temperature for 2 hours.

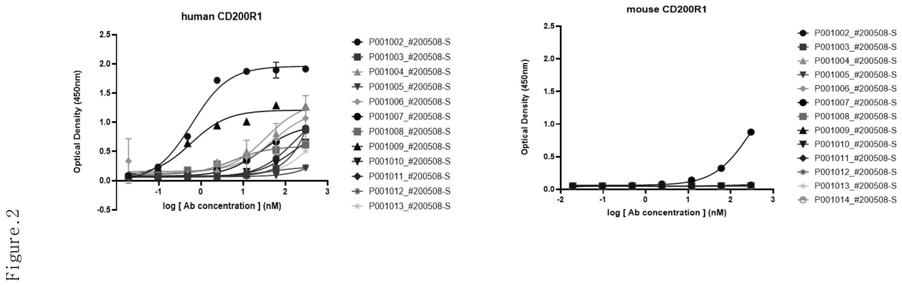

The result was washed with PBST and distilled water, the samples were bound with a goat-derived HRP-conjugated anti-human antibody (Invitrogen, 31482, Goat anti-Human IgG F(ab')2 Secondary Antibody, HRP) for 1 hour at room temperature, and then washed again with PBST and distilled water. Color was developed with a TMB substrate solution (Thermo Scientific, 34029, 1-Step^{™} Ultra TMB-ELISA Substrate Solution). The color reaction was stopped with stop solution (Invitrogen, SS04, ELISA Stop Solution). The absorbance was measured at an optical density of 450 nm. The anti-CD200R1 antibody of the present invention was found to not induce cross-reactivity of human CD200R1 and mouse CD200R1 (FIG. 2).

### Example 5: Competitive binding assay with human CD200 (ELISA)

96-well plates were coated with human CD200R1 (R&D systems, 3414-CD) recombinant protein at a concentration of 2 µg/mL at 4°C for 16 hours, and blocked with 3% skim milk for 1 hour. Then, the result was treated with antibodies at concentrations of 500 nM, 166.67 nM, 55.56 nM, 18.52 nM, 6.17 nM, 2.06 nM, and 0.69 nM, and incubated at room temperature for 30 minutes. RhCD200-Fc (R&D systems, 2724-CD) recombinant protein was then added to a final concentration of 1 µg/mL, to induce binding for 1 hour and 30 minutes at room temperature. The result was washed with PBST and distilled water, and then the samples were treated with human CD200 biotinylated antibody at a concentration of 0.5 µg/mL (R&D systems, BAF2724). The result was washed with PBST and distilled water, and the samples were bound with Pierce^{™} High Sensitivity Streptavidin-HRP (Thermo Scientific, 21130) at room temperature for 1 hour, and then washed again with PBST and distilled water. TMB substrate solution (Thermo Scientific, 34029, 1-Step^{™} Ultra TMB-ELISA substrate solution) was added to develop the color. The reaction was stopped with stop solution (Invitrogen, SS04, ELISA Stop Solution). The absorbance at 450 nm was measured to determine whether or not the identified clones competitively bound to CD200.

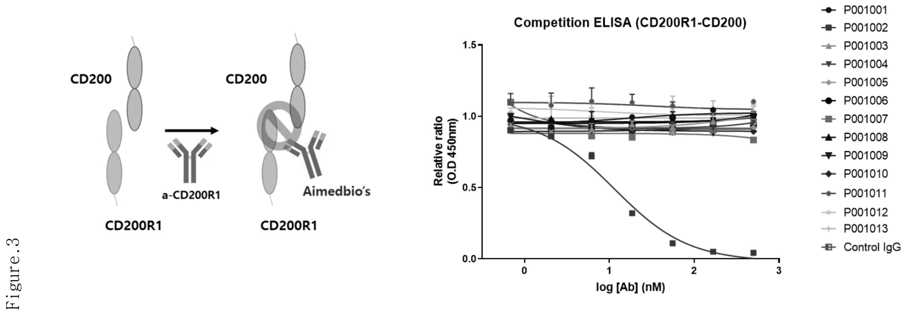

As shown in FIG. 3, with the exception of P001002, none of the substances identified in the present invention exhibited competitive binding. P001002 exhibited an IC₅₀ of 11.5 nM, demonstrating an antagonistic effect against CD200R1.

### Example 6: Screening of anti-CD200R1-specific antibodies using hybridomas

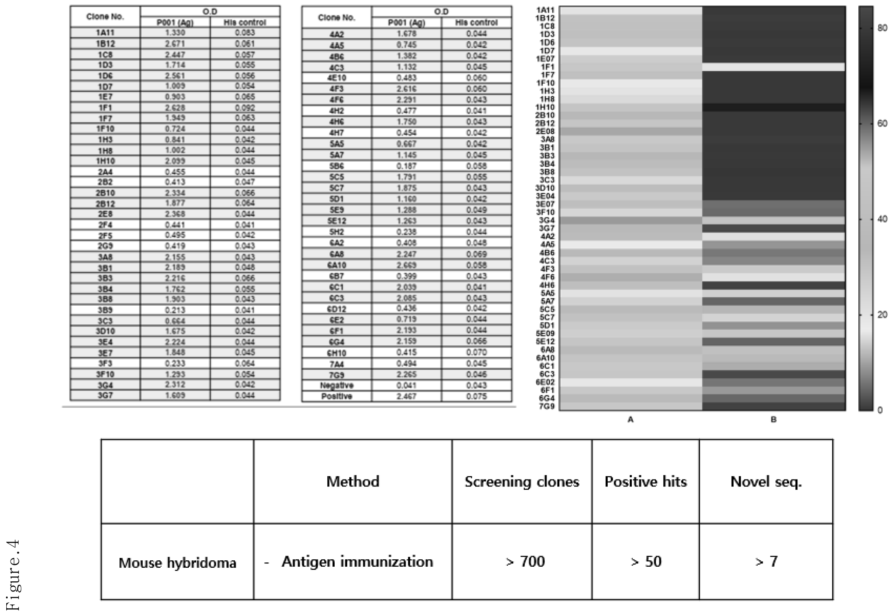

To screen for anti-CD200R1 antibodies using hybridoma technology, immunization was performed using the human CD200R1 recombinant protein (Abclon-MANI protocol). Five weeks after immunization, a pool of clones that bound to human CD200R1 was selected through fusion ELISA. Cloning was performed on each of the clones with high resulting values (OD values) of ELISA to ultimately select hybridoma clones that exhibited antigen binding (FIG. 4). The clones were 1F1, 1H10, 3G4, 5E9, 6A8, 6A10, and 6C3, and the ELISA binding to the antigen was analyzed as an ELISA O.D. of approximately 1.5 or more (negative antigen, 0.04 or less). Based on this, the following cloning was performed to obtain antibody sequences for the hybridoma clones screened through immunization. RNA was isolated using a commercially available RNA extraction kit (Invitrogen, 12183555, TRIzol^{™} Plus RNA Purification Kit). Single-stranded DNA was synthesized using a PCR cDNA synthesis kit (Takara, 634925, SMARTer^{®} PCR cDNA Synthesis Kit) and the following primers. SMART_F (SEQ ID NO: 201): 5'-AAG CAG TGG TAT CAA CGC AGA GTA CGC GGG-3', SMART_CDS_F (SEQ ID NO: 202): 5'-AAG CAG TGG TAT CAA CGC AGA GTA CTT TTT TTT TTT TTT TTT TTT TTT TTT TTT TVN-3' (V= A, C, G; N = A, C, G, T).

First-strand cDNA synthesis was performed using a synthesis kit (Invitrogen, 18091050, SuperScript^{™} IV First-Strand Synthesis System), and ss-cDNA synthesis was performed using Pfu-X DNA Polymerase (Solgent, SPX16-R250, Solg^{™} Pfu-X DNA Polymerase).

The base sequences of the antibody heavy and light chain variable regions were amplified using the heavy chain variable region primers Hybridoma_F (SEQ ID NO: 203) (5' - AAG CAG TGG TAT CAA CGC AGA GT -3') and mIgG2a_1 & mIgG2b_1 (SEQ ID NOs: 204 and 205) (5'- CAG GGG CCA GTG GAT AGA CCG ATG -3', 5'- CAG GGG CCA GTG GAT AGA CTG ATG -3'). The light chain variable region primers were Hybridoma_F (5'- AAG CAG TGG TAT CAA CGC AGA GT -3') and mckappa_1_R (SEQ ID NO: 206) (5'- GGA TGG TGG GAA GAT GGA TAC AGT TGG TG -3') or mckappa_2_R (SEQ ID NO: 207) (5'- TTA ACA CTC ATT CCT GTT GAA GCT CTT GAC -3'). The mixed sample was loaded into a PCR machine and then amplified. The resulting product was identified by agarose gel electrophoresis and purified using a gel extraction kit (Macherey-Nagel, 740609.50, NucleoSpin Gel and PCR Clean-up). The amplified PCR product was ligated into the Topo vector using a kit (Invitrogen, 45-0245, Zero Blunt^{™} TOPO^{™} PCR Cloning Kit, without competent cells). The heavy and light chain expression plasmids were electroporated into competent cells for transformation. The resulting colonies were collected, and the plasmids were purified using a miniprep kit (QIAGEN, 27104, QIAprep Spin Miniprep Kit).

The CDR sequences of each antibody are shown in Tables 10 and 11, and the amino acid sequences of the heavy and light chain variable regions are shown in Tables 12 and 13.

**[Table 10]**

| | CDR H1 | | CDR H2 | | CDR H3 | |
|---|---|---|---|---|---|---|
| | Sequence | SEQ ID NO. | Sequence | SEQ ID NO. | Sequence | SEQ ID NO. |
| 1F1 | SYWMN | 100 | RIDPADSEIH YNQKFKD | 104 | SGGWLPGFAY | 110 |
| 1H10 | SYWIN | 101 | DINPGSGSTN YNEKFRS | 105 | EDYYGSRFFY | 111 |
| 3G4 | SYWMN | 100 | RIDPSDSETH YNQKFKD | 106 | SGGWLPGFAY | 112 |
| 5E9 | SYWMQ | 102 | AIYPGDGDTR YTQKFKG | 107 | GGVTTLVPYF FDY | 113 |
| 6A8 | SYWMN | 100 | RIDPSDSETH YNQKFKD | 106 | SGGWLPGFAY | 110 |
| 6A10 | DYYLH | 103 | WIDPENGDTE YAPKFQG | 108 | GGSY | 114 |
| 6C3 | SYWMN | 100 | RIDPYDSETH YNQKFKD | 109 | VDGNHLFDY | 115 |

**[Table 11]**

| | CDR L1 | | CDR L2 | | CDR L3 | |
|---|---|---|---|---|---|---|
| | Sequence | SEQ ID NO. | Sequence | SEQ ID NO. | Sequence | SEQ ID NO. |
| 1F1 | SASSSLIYMH | 116 | DTSKLAS | 123 | FQGSGYPLMYT | 128 |
| 1H10 | RSSQSLVHSNG NTYLH | 117 | KVSNRFS | 124 | SQSTHVT | 129 |
| 3G4 | SARSSVSYMH | 118 | DTSKLAS | 125 | FQGSGDPLMYT | 130 |
| 5E9 | RSSQSIVHSNG NTYLE | 119 | KVSNRFS | 124 | FQGSHVPPT | 131 |
| 6A8 | SASSSLSYML | 120 | DTSKLAS | 125 | FQGSGNPLMYT | 132 |
| 6A10 | KSSQSLLNSGN QKNYLA | 121 | GASTRES | 126 | QNDHSYPYT | 133 |
| 6C3 | KASQDINSYLS | 122 | RANRLVD | 127 | LQYDEFPYT | 134 |

**[Table 12]**

| | VH Sequence | SEQ ID NO. |
|---|---|---|
| 1F1 | QVQLQQPGAEFVKPGAPVKLSCKASGYTFTSYWMNWVKQRPGRGLEW IGRIDPADSEIHYNQKFKDKATLTVDKSSSTAYIQFSSLTSEDSAVY YCARSGGWLPGFAYWGQGTLVTVSA | 135 |
| 1H10 | QVQLQQPGAELVKPGTSVKLSCKASGYNFTSYWINWVKLRPGQGLEW IGDINPGSGSTNYNEKFRSKATLTVGTSSSTAYMQLSSLASEDSALY YCAREDYYGSRFFYWGQGTLVTVSA | 136 |
| 3G4 | QVQLQQPGAELVKPGAPVKLSCKASGFTLTSYWMNWVKQRPGRGLEW IGRIDPSDSETHYNQKFKDKATLTVDKSSSTAYIQLTSLTSEDSAVY YCARSGGWLPGFAYWGQGTLVTVSA | 137 |
| 5E9 | QVQLQQSGAELARPGASVKLSCKASGYTFTSYWMQWVKQRPGQGLEW IGAIYPGDGDTRYTQKFKGKATLTADKSSSTAYMQLRSLASEDSAVF YCARGGVTTLVPYFFDYWGQGATLTVSS | 138 |
| 6A8 | QVQLQQPGAELVKPGAPVKLSCKASGYTFTSYWMNWVKQRPGRGLEW IGRIDPSDSETHYNQKFKDKATLTVDKSSSTAYIQLSSLTSEDSAVY YCARSGGWLPGFAYWGQGTLVTVSA | 139 |
| 6A10 | QLQQSGAELVRSGASVKLSCTASGFNIKDYYLHWIKQRPEQGLEWIG WIDPENGDTEYAPKFQGKATMTADTSSNTAYLQLISLTSEDAAVYYC FAGGSYWGQGTSVTVSS | 140 |
| 6C3 | QVQLQQPGAELVRPGASVKLSCKASAYTFTSYWMNWVKQRPEQGLEW IGRIDPYDSETHYNQKFKDKAILTVDKSSSTAYMQLSSLTSEDSAVY YCARVDGNHLFDYWGQGTTLTVSS | 141 |

**[Table 13]**

| | VL Sequence | SEQ ID NO. |
|---|---|---|
| 1F1 | ENVLTQSPAIMSASPGEKVTMTCSASSSLIYMHWYQQKSSTSPKLWI YDTSKLASGVPGRFSGSGSGNSYSLTINSMEAEDVATYYCFQGSGYP LMYTFGGGTKLEIK | 142 |
| 1H10 | DVVMTQTPLSLPVSLGDQASISCRSSQSLVHSNGNTYLHWYLQKPGQ SPKLLIYKVSNRFSGVPDRFSGSGSGTDFTLKISRVEAEDLGVYFCS QSTHVTFGAGTKLELK | 143 |
| 3G4 | ENVLTQSPAIMSASPGEKVTMTCSARSSVSYMHWYQQKSRTSPKLWI YDTSKLASGVPGRFSGSGSGNSYSLTISSMEAEDVATYYCFQGSGDP LMYTFGGGTKLEIK | 144 |
| 5E9 | DVLMTQTPLSLPVSLGDQASISCRSSQSIVHSNGNTYLEWYLQKPGQ SPKLLIYKVSNRFSGVPDRFSGSGSGTDFTLKISRVEAEDLGVYYCF QGSHVPPTFGGGTKLEIK | 145 |
| 6A8 | ENVLTQSPAIMSASPGEKVTMTCSASSSLSYMLWYQQKSSTSPKLWI YDTSKLASGVPGRFSGSGSGNSYSLTISSMEAEDVGTYYCFQGSGNP LMYTFGGGTKLEIK | 146 |
| 6A10 | DIVMTQSPSSLSVSAGEKVTMSCKSSQSLLNSGNQKNYLAWYQQKPG QPPKLLIYGASTRESGVPDRFTGSGSGTDFTLTISSVQAEDLAVYYC QNDHSYPYTFGGGTKLEIK | 147 |
| 6C3 | DIKMTQSPSSMYASLGERVTITCKASQDINSYLSWFQQKPGKSPKTL IYRANRLVDGVPSRFSGSGSGQDYSLTISSLEYEDMGIYYCLQYDEF PYTFGGGTKLEIK | 148 |

### Example 7: Humanization engineering of anti-CD200R1 antibody

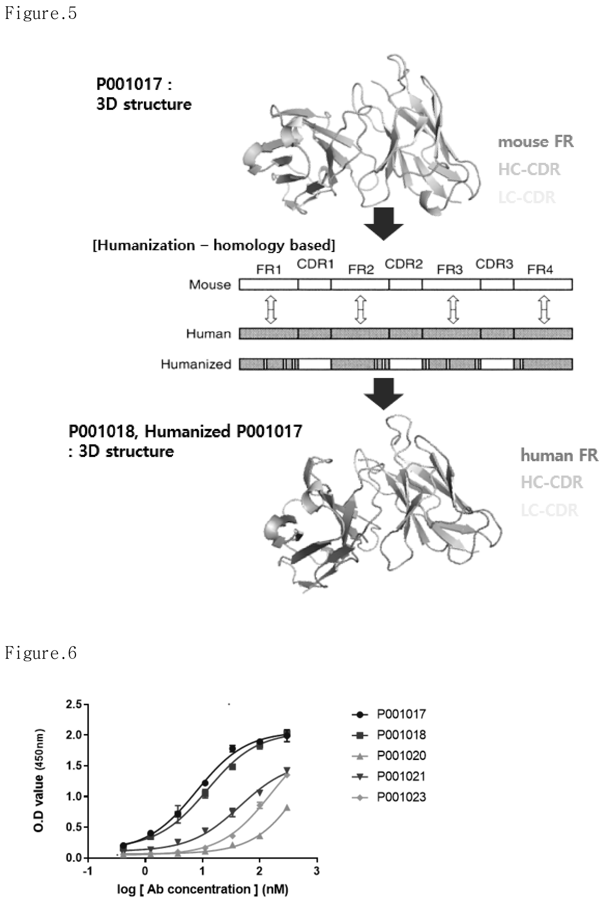

Humanization engineering was performed on the murine anti-CD200R1 antibody OX108 (P001017, NCBI database IDs: 3DGG_A and 3DGG_B) clone for development and improvement for therapeutic applications. The variable regions of the light and heavy chains of the P001017 clone were analyzed (Kabat numbering and Chothia numbering), and the CDRs of P001007 were segmented and grafted onto human FRs, which is a process called "CDR grafting". Furthermore, the clone was engineered using a homology-based framework (FR) or based on a human framework (FR) commonly incorporated into therapeutic antibodies (fixed framework) (FIG. 5).

Respective light and heavy chains are shown in Tables 14 and 15, and humanized antibody engineering was performed using each combination. Table 16 shows the sequences of antibodies discovered through humanized engineering, and Table 17 shows the CDR sequences of each antibody.

**[Table 14]**

| | VH Sequence | SEQ ID NO. |
|---|---|---|
| OX108 (P0010 17) | EVKLQQSGPELVKPGASVKMSCKASGYTFTSYVMHWVKQKPGQGLEW IGYINPYNDDTNYNEKFKGKATLTSDKSSSTAYMELSSLTSEDSAVY YCAREDYYGSRWGYWGQGTTLTVSS | 149 |
| OX108_ VH_hu1 | QVQLVQSGAEVKKPGASVKVSCKASGYTFTSYVMHWVRQAPGQGLEW MGYINPYNDDTNYNEKFKGRVTMTRDTSTSTVYMELSSLRSEDTAVY YCAREDYYGSRWGYWGQGTLVTVSS | 150 |
| OX108_ VH_hu2 | QVQLVQSGAEVKKPGASVKVSCKASGYTFTSYVMHWVRQAPGQGLEW IGYINPYNDDTNYNEKFKGRATLTSDKSTSTAYMELSSLRSEDTAVY YCAREDYYGSRWGYWGQGTLVTVSS | 151 |
| OX108 VH_hu3 | QVQLVESGGGVVQPGGSLRLSCAASGYTFTSYVMHWVRQAPGKGLEW VAYINPYNDDTNYNEKFKGRFTISRDNSKNTLYLQMNSLRAEDTAVY YCAKEDYYGSRWGYWGQGTLVTVSS | 152 |
| OX108_ VH_hu4 | EVQLVESGGGVVQPGGSLRLSCAASGYTFTSYVMHWVRQAPGKGLEW IGYINPYNDDTNYNEKFKGRATLSSDKSKNTAYLQMNSLRAEDTAVY YCAREDYYGSRWGYWGQGTLVTVSS | 153 |

**[Table 15]**

| | VL Sequence | SEQ ID NO. |
|---|---|---|
| OX108 (P0010 17) | DIVLTQSPASLAVSLGQRATISCRASKSVSTSGYSYMHWYQQKPGQP PKLLIYLASNLESGVPARFSGSGSGTDFTLNIHPVEEEDAATYYCQH SRELLTFGAGTKLELK | 154 |
| OX108_ VL_hu1 | DIVLTQSPASLAVSPGQRATITCRASKSVSTSGYSYMHWYQQKPGQP PKLLIYLASNLESGVPARFSGSGSGTDFTLTINPVEANDTANYYCQH SRELLTFGQGTKVEIK | 155 |
| OX108_ VL_hu2 | DIVLTQSPASLAVSPGQRATITCRASKSVSTSGYSYMHWYQQKPGQP PKLLIYLASNLESGVPARFSGSGSGTDFTLTINPVEANDTANYYCQH SRELLTFGAGTKVEIK | 156 |
| OX108_ VL_hu3 | DIQMTQSPSSLSASVGDRVTITCRASKSVSTSGYSYMHWYQQKPGKA PKLLIYLASNLESGVPSRFSGSGSGTDFTLTISSLQPEDFATYYCQH SRELLTFGQGTKVEIK | 157 |

**[Table 16]**

| Antibody name | Heavy chain | Light chain | Comments |
|---|---|---|---|
| huOX108.1 (P001018) | OX108_VH_hu1 (SEQ ID NO.: 150) | OX108_VL_hu1 (SEQ ID NO.: 155) | CDR-grafting only |
| huOX108.2 (P001019) | OX108_VH_hu2 (SEQ ID NO.: 151) | OX108_VL_hu1 (SEQ ID NO.: 155) | Humanization using homology based framework |
| huOX108.3 (P001020) | OX108_VH_hu3 (SEQ ID NO.: 152) | OX108_VL_hu3 (SEQ ID NO.: 157) | Humanization using fixed framework |
| huOX108. 4 (P001021) | OX108_VH_hu4 (SEQ ID NO.: 153) | OX108_VL_hu3 (SEQ ID NO.: 157) | Humanization using fixed framework |
| huOX108.5 (P001022) | OX108_VH_hu2 (SEQ ID NO.: 151) | OX108_VL_hu3 (SEQ ID NO.: 157) | HC (Homology based FR) / LC (Fixed FR) mix |
| huOX108. 6 (P001023) | OX108_VH_hu3 (SEQ ID NO.: 152) | OX108_VL_hu1 (SEQ ID NO.: 155) | HC (Fixed FR) / LC (Homology based FR) mix |

**[Table 17]**

| | | | | | | |
|---|---|---|---|---|---|---|
| | | | | | | |
| | CDR H1 | | CDR H2 | | CDR H3 | |
| | Sequenc e | SEQ ID NO. | Sequenc e | SEQ ID NO. | Sequenc e | SEQ ID NO. |
| P001017 ~ P001023 | SYVMH | 158 | YINPYND DTNYNEK FKG | 159 | EDYYGSR WGY | 160 |
| | CDR L1 | | CDR L2 | | CDR L3 | |
| | RASKSVS TSGYSYM H | 161 | LASNLES | 162 | QHSRELL T | 163 |

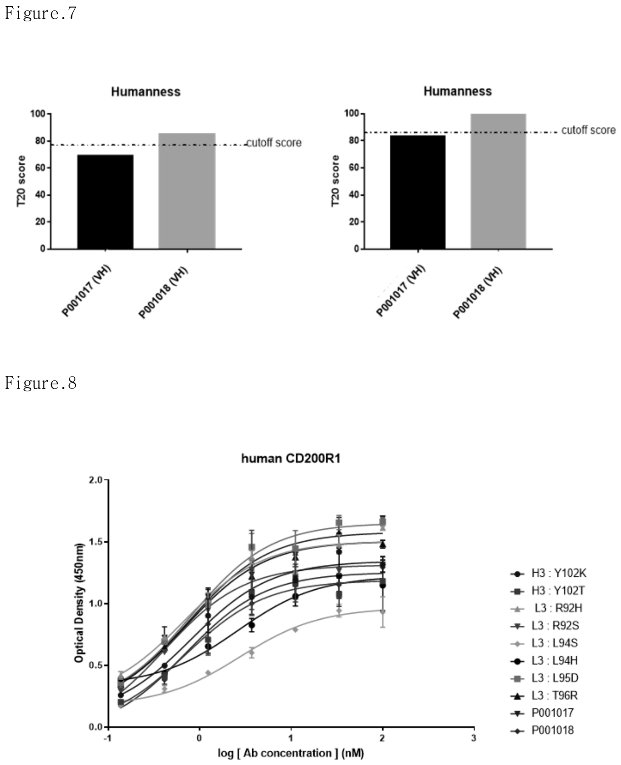

As shown in FIGS. 6 and 7, the binding ability of the humanized engineered antibodies to human CD200R1 was analyzed via ELISA. The P001018 clone exhibited similar specific binding ability to P001017 (the parent antibody). *In silico* humanness analysis showed that the humanization level satisfied the standard range, demonstrating reduction of the risk of immunogenicity or ADA.

### Example 8: Affinity engineering for anti-CD200R1 humanized antibodies

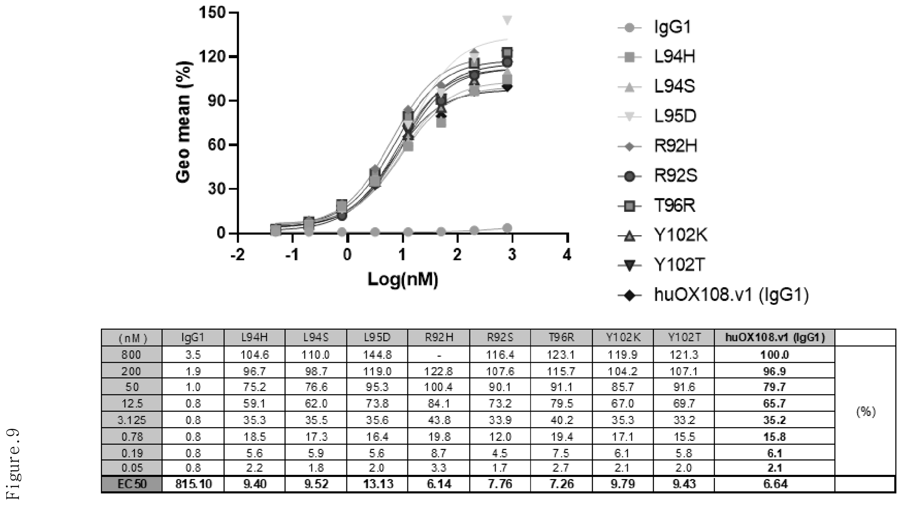

Affinity engineering for the humanized antibody P001018 was performed using phage display technology. A phagemid vector displaying the P001018 scFv fragment (heavy chain variable region; VH, light chain variable region; VL) on the surface of M13 bacteriophage was constructed and used as a library template. For affinity maturation, CDRs H1, H2, H3 and CDRs L1, L2, L3 were selected based on the Kabat numbering of the antibody, and a P001018 mutant library was constructed using primers containing the NNK degeneration codon for each CDR. For each library, the biopanning (antigen fixation panning) described above was performed 3 to 4 times, and based on this, antibodies with higher affinity than the P001018 clone were isolated through ELISA-based affinity screening. As shown in FIGS. 8 and 9, the major clones with enhanced affinity were identified as H3_Y102K, H3-Y102T, L3-R92H, L3-R92S, L3-L94S, L3-L94H, L3-L95D, and L3-T96R (Kabat numbering). These clones were identified through antigen binding assays (ELISA) and cell binding assays (FACS). The sequences and CDR sequences of ScFvs with each mutant sequence are shown in Tables 18 to 20.

**[Table 18]**

| | scFv Sequence | SEQ ID NO. |
|---|---|---|
| H3-Y102K | QVQLVQSGAEVKKPGASVKVSCKASGYTFTSYVMHWVRQAPGQGLEWM GYINPYNDDTNYNEKFKGRVTMTRDTSTSTVYMELSSLRSEDTAVYYC AREDYYGSRWGKWGQGTLVTVSSGGGGSGGGGSGGGGSDIVLTQSPAS LAVSPGQRATITCRASKSVSTSGYSYMHWYQQKPGQPPKLLIYLASNL ESGVPARFSGSGSGTDFTLTINPVEANDTANYYCQHSRELLTFGQGTK VEIK | 164 |
| H3-Y102T | QVQLVQSGAEVKKPGASVKVSCKASGYTFTSYVMHWVRQAPGQGLEWM GYINPYNDDTNYNEKFKGRVTMTRDTSTSTVYMELSSLRSEDTAVYYC AREDYYGSRWGTWGQGTLVTVSSGGGGSGGGGSGGGGSDIVLTQSPAS LAVSPGQRATITCRASKSVSTSGYSYMHWYQQKPGQPPKLLIYLASNL ESGVPARFSGSGSGTDFTLTINPVEANDTANYYCQHSRELLTFGQGTK VEIK | 165 |
| L3-R92H | QVQLVQSGAEVKKPGASVKVSCKASGYTFTSYVMHWVRQAPGQGLEWM GYINPYNDDTNYNEKFKGRVTMTRDTSTSTVYMELSSLRSEDTAVYYC AREDYYGSRWGYWGQGTLVTVSSGGGGSGGGGSGGGGSDIVLTQSPAS LAVSPGQRATITCRASKSVSTSGYSYMHWYQQKPGQPPKLLIYLASNL ESGVPARFSGSGSGTDFTLTINPVEANDTANYYCQHSHELLTFGQGTK VEIK | 166 |
| L3-R92S | QVQLVQSGAEVKKPGASVKVSCKASGYTFTSYVMHWVRQAPGQGLEWM GYINPYNDDTNYNEKFKGRVTMTRDTSTSTVYMELSSLRSEDTAVYYC AREDYYGSRWGYWGQGTLVTVSSGGGGSGGGGSGGGGSDIVLTQSPAS LAVSPGQRATITCRASKSVSTSGYSYMHWYQQKPGQPPKLLIYLASNL ESGVPARFSGSGSGTDFTLTINPVEANDTANYYCQHSSELLTFGQGTK VEIK | 167 |
| L3-L94S | QVQLVQSGAEVKKPGASVKVSCKASGYTFTSYVMHWVRQAPGQGLEWM GYINPYNDDTNYNEKFKGRVTMTRDTSTSTVYMELSSLRSEDTAVYYC AREDYYGSRWGYWGQGTLVTVSSGGGGSGGGGSGGGGSDIVLTQSPAS LAVSPGQRATITCRASKSVSTSGYSYMHWYQQKPGQPPKLLIYLASNL ESGVPARFSGSGSGTDFTLTINPVEANDTANYYCQHSRESLTFGQGTK VEIK | 168 |
| L3-L94H | QVQLVQSGAEVKKPGASVKVSCKASGYTFTSYVMHWVRQAPGQGLEWM GYINPYNDDTNYNEKFKGRVTMTRDTSTSTVYMELSSLRSEDTAVYYC AREDYYGSRWGYWGQGTLVTVSSGGGGSGGGGSGGGGSDIVLTQSPAS LAVSPGQRATITCRASKSVSTSGYSYMHWYQQKPGQPPKLLIYLASNL ESGVPARFSGSGSGTDFTLTINPVEANDTANYYCQHSREHLTFGQGTK VEIK | 169 |
| L3-L95D | QVQLVQSGAEVKKPGASVKVSCKASGYTFTSYVMHWVRQAPGQGLEWM GYINPYNDDTNYNEKFKGRVTMTRDTSTSTVYMELSSLRSEDTAVYYC AREDYYGSRWGYWGQGTLVTVSSGGGGSGGGGSGGGGSDIVLTQSPAS LAVSPGQRATITCRASKSVSTSGYSYMHWYQQKPGQPPKLLIYLASNL | 170 |
| | ESGVPARFSGSGSGTDFTLTINPVEANDTANYYCQHSRELDTFGQGTK VEIK | |
| L3-T96R | QVQLVQSGAEVKKPGASVKVSCKASGYTFTSYVMHWVRQAPGQGLEWM GYINPYNDDTNYNEKFKGRVTMTRDTSTSTVYMELSSLRSEDTAVYYC AREDYYGSRWGYWGQGTLVTVSSGGGGSGGGGSGGGGSDIVLTQSPAS LAVSPGQRATITCRASKSVSTSGYSYMHWYQQKPGQPPKLLIYLASNL ESGVPARFSGSGSGTDFTLTINPVEANDTANYYCQHSRELLRFGQGTK VEIK | 171 |

| | | |
|---|---|---|
| * Bold/underline indicates mutation location | | |

**[Table 19]**

| | CDR H1 | | CDR H2 | | CDR H3 | |
|---|---|---|---|---|---|---|
| | Sequence | SEQ ID NO. | Sequence | SEQ ID NO. | Sequence | SEQ ID NO. |
| H3-Y102 K | SYVMH | 158 | YINPYNDD TNYNEKFK G | 159 | EDYYGSRW GK | 172 |
| H3-Y102 T | SYVMH | 158 | YINPYNDD TNYNEKFK G | 159 | EDYYGSRW GT | 173 |
| L3-R92H | SYVMH | 158 | YINPYNDD TNYNEKFK G | 159 | EDYYGSRW GY | 160 |
| L3-R92S | SYVMH | 158 | YINPYNDD TNYNEKFK G | 159 | EDYYGSRW GY | 160 |
| L3-L94S | SYVMH | 158 | YINPYNDD TNYNEKFK G | 159 | EDYYGSRW GY | 160 |
| L3-L94H | SYVMH | 158 | YINPYNDD TNYNEKFK G | 159 | EDYYGSRW GY | 160 |
| L3-L95D | SYVMH | 158 | YINPYNDD TNYNEKFK G | 159 | EDYYGSRW GY | 160 |
| L3-T96R | SYVMH | 158 | YINPYNDD TNYNEKFK G | 159 | EDYYGSRW GY | 160 |

| | | | | | | |
|---|---|---|---|---|---|---|
| * Bold/underline indicates mutation location | | | | | | |

**[Table 20]**

| | CDR L1 | | CDR L2 | | CDR L3 | |
|---|---|---|---|---|---|---|
| | Sequence | SEQ ID NO. | Sequence | SEQ ID NO. | Sequence | SEQ ID NO. |
| H3-Y102 K | RASKSVST SGYSYMH | 161 | LASNLES | 162 | QHSRELLT | 163 |
| H3-Y102 T | RASKSVST SGYSYMH | 161 | LASNLES | 162 | QHSRELLT | 163 |
| L3-R92H | RASKSVST SGYSYMH | 161 | LASNLES | 162 | QHS**H**ELLT | 174 |
| L3-R92S | RASKSVST SGYSYMH | 161 | LASNLES | 162 | QHS**S**ELLT | 175 |
| L3-L94S | RASKSVST SGYSYMH | 161 | LASNLES | 162 | QHSRE**S**LT | 176 |
| L3-L94H | RASKSVST SGYSYMH | 161 | LASNLES | 162 | QHSRE**H**LT | 177 |
| L3-L95D | RASKSVSTS GYSYMH | 161 | LASNLES | 162 | QHSREL**D**T | 178 |
| L3-T96R | RASKSVSTS GYSYMH | 161 | LASNLES | 162 | QHSRELL**R** | 179 |

| | | | | | | |
|---|---|---|---|---|---|---|
| * Bold/underline indicates mutation location | | | | | | |

The obtained clones were produced as antibodies and the affinity thereof for human CD200R1 was evaluated by ELISA. Based thereon, variants with superior affinity were combined for secondary engineering (Table 21).

**[Table 21]**

| **Antibody name** | **Heavy chain** | **Light chain** |
|---|---|---|
| P001018v1 | H3-Y102K | - |
| P001018v2 | - | L3-R92H |
| P001018v3 | - | L3-L95D |
| P001018v4 | | L3-R92S |
| P001018v5 | | L3-T96R |
| P001018v6 | H3-Y102T | |
| P001018v23 | | L3-R92H |
| | | L3-L95D |
| P001018v26 | H3-Y102T | L3-R92H |
| P001018v36 | H3-Y102T | L3-L95D |
| | | L3-R92H |
| P001018v235 | | L3-L95D |
| | | L3-T96R |
| P001018v236 | H3-Y102T | L3-R92H |
| | | L3-L95D |
| | | L3-R92H |
| P001018v2356 | H3-Y102T | L3-L95D |
| | | L3-T96R |

The amino acid sequences and CDR sequences of the heavy and light chain variable regions of each antibody with improved affinity are shown in Tables 22 to 25.

**[Table 22]**

| | CDR H1 | | CDR H2 | | CDR H3 | |
|---|---|---|---|---|---|---|
| | Sequenc e | SEQ ID NO. | Sequenc e | SEQ ID NO. | Sequenc e | SEQ ID NO. |
| P001018 v1 | SYVMH | 158 | YINPYND DTNYNEK FKG | 159 | EDYYGSR WGK | 172 |
| P001018 v2 | SYVMH | 158 | YINPYND DTNYNEK FKG | 159 | EDYYGSR WGY | 160 |
| P001018 v3 | SYVMH | 158 | YINPYND DTNYNEK FKG | 159 | EDYYGSR WGY | 160 |
| P001018 v4 | SYVMH | 158 | YINPYND DTNYNEK FKG | 159 | EDYYGSR WGY | 160 |
| P001018 v5 | SYVMH | 158 | YINPYND DTNYNEK FKG | 159 | EDYYGSR WGY | 160 |
| P001018 v6 | SYVMH | 158 | YINPYND DTNYNEK FKG | 159 | EDYYGSR WGT | 173 |
| P001018 v23 | SYVMH | 158 | YINPYND DTNYNEK FKG | 159 | EDYYGSR WGY | 160 |
| P001018 v26 | SYVMH | 158 | YINPYND DTNYNEK FKG | 159 | EDYYGSR WGT | 173 |
| P001018 v36 | SYVMH | 158 | YINPYND DTNYNEK FKG | 159 | EDYYGSR WGT | 173 |
| P001018 v235 | SYVMH | 158 | YINPYND DTNYNEK FKG | 159 | EDYYGSR WGY | 160 |
| P001018 v236 | SYVMH | 158 | YINPYND DTNYNEK FKG | 159 | EDYYGSR WGT | 173 |
| P001018 v2356 | SYVMH | 158 | YINPYND DTNYNEK FKG | 159 | EDYYGSR WGT | 173 |

| | | | | | | |
|---|---|---|---|---|---|---|
| * Bold/underline indicates mutation location | | | | | | |

**[Table 23]**

| | CDR L1 | | CDR L2 | | CDR L3 | |
|---|---|---|---|---|---|---|
| | Sequenc e | SEQ ID NO. | Sequenc e | SEQ ID NO. | Sequenc e | SEQ ID NO. |
| P001018 v1 | RASKSVS TSGYSYM H | 161 | LASNLES | 162 | QHSRELL T | 163 |
| P001018 v2 | RASKSVS TSGYSYM H | 161 | LASNLES | 162 | QHSHELL T | 174 |
| P001018 v3 | RASKSVS TSGYSYM H | 161 | LASNLES | 162 | QHSRELDT | 178 |
| P001018 v4 | RASKSVS TSGYSYM H | 161 | LASNLES | 162 | QHSSELL T | 175 |
| P001018 v5 | RASKSVST SGYSYMH | 161 | LASNLES | 162 | QHSRELLR | 179 |
| P001018 v6 | RASKSVST SGYSYMH | 161 | LASNLES | 162 | QHSRELL T | 163 |
| P001018 v23 | RASKSVS TSGYSYM H | 161 | LASNLES | 162 | QHSHELD T | 180 |
| P001018 v26 | RASKSVS TSGYSYM H | 161 | LASNLES | 162 | QHSHELL T | 174 |
| P001018 v36 | RASKSVS TSGYSYM H | 161 | LASNLES | 162 | QHSRELDT | 178 |
| P001018 v235 | RASKSVS TSGYSYM H | 161 | LASNLES | 162 | QHSHELD R | 181 |
| P001018 v236 | RASKSVST SGYSYMH | 161 | LASNLES | 162 | QHSHELD T | 182 |
| P001018 v2356 | RASKSVST SGYSYMH | 161 | LASNLES | 162 | QHSHELD R | 183 |

| | | | | | | |
|---|---|---|---|---|---|---|
| * Bold/underline indicates mutation location | | | | | | |

**[Table 24]**

| | VH Sequence | SEQ ID NO. |
|---|---|---|
| P001018v1 | QVQLVQSGAEVKKPGASVKVSCKASGYTFTSYVMHWVRQAPGQGL EWMGYINPYNDDTNYNEKFKGRVTMTRDTSTSTVYMELSSLRSED TAVYYCAREDYYGSRWGKWGQGTLVTVSS | 184 |
| P001018v2 | QVQLVQSGAEVKKPGASVKVSCKASGYTFTSYVMHWVRQAPGQGL EWMGYINPYNDDTNYNEKFKGRVTMTRDTSTSTVYMELSSLRSED TAVYYCAREDYYGSRWGYWGQGTLVTVSS | 150 |
| P001018v3 | QVQLVQSGAEVKKPGASVKVSCKASGYTFTSYVMHWVRQAPGQGL EWMGYINPYNDDTNYNEKFKGRVTMTRDTSTSTVYMELSSLRSED TAVYYCAREDYYGSRWGYWGQGTLVTVSS | 150 |
| P001018v4 | QVQLVQSGAEVKKPGASVKVSCKASGYTFTSYVMHWVRQAPGQGL EWMGYINPYNDDTNYNEKFKGRVTMTRDTSTSTVYMELSSLRSED TAVYYCAREDYYGSRWGYWGQGTLVTVSS | 150 |
| P001018v5 | QVQLVQSGAEVKKPGASVKVSCKASGYTFTSYVMHWVRQAPGQGL EWMGYINPYNDDTNYNEKFKGRVTMTRDTSTSTVYMELSSLRSED TAVYYCAREDYYGSRWGYWGQGTLVTVSS | 150 |
| P001018v6 | QVQLVQSGAEVKKPGASVKVSCKASGYTFTSYVMHWVRQAPGQGL EWMGYINPYNDDTNYNEKFKGRVTMTRDTSTSTVYMELSSLRSED TAVYYCAREDYYGSRWGTWGQGTLVTVSS | 185 |
| P001018v2 3 | QVQLVQSGAEVKKPGASVKVSCKASGYTFTSYVMHWVRQAPGQGL EWMGYINPYNDDTNYNEKFKGRVTMTRDTSTSTVYMELSSLRSED TAVYYCAREDYYGSRWGYWGQGTLVTVSS | 150 |
| P001018v2 6 | QVQLVQSGAEVKKPGASVKVSCKASGYTFTSYVMHWVRQAPGQGL EWMGYINPYNDDTNYNEKFKGRVTMTRDTSTSTVYMELSSLRSED TAVYYCAREDYYGSRWGTWGQGTLVTVSS | 185 |
| P001018v3 6 | QVQLVQSGAEVKKPGASVKVSCKASGYTFTSYVMHWVRQAPGQGL EWMGYINPYNDDTNYNEKFKGRVTMTRDTSTSTVYMELSSLRSED TAVYYCAREDYYGSRWGTWGQGTLVTVSS | 185 |
| P001018v2 35 | QVQLVQSGAEVKKPGASVKVSCKASGYTFTSYVMHWVRQAPGQGL EWMGYINPYNDDTNYNEKFKGRVTMTRDTSTSTVYMELSSLRSED TAVYYCAREDYYGSRWGYWGQGTLVTVSS | 150 |
| P001018v2 36 | QVQLVQSGAEVKKPGASVKVSCKASGYTFTSYVMHWVRQAPGQGL EWMGYINPYNDDTNYNEKFKGRVTMTRDTSTSTVYMELSSLRSED TAVYYCAREDYYGSRWGTWGQGTLVTVSS | 185 |
| P001018v2 356 | QVQLVQSGAEVKKPGASVKVSCKASGYTFTSYVMHWVRQAPGQGL EWMGYINPYNDDTNYNEKFKGRVTMTRDTSTSTVYMELSSLRSED TAVYYCAREDYYGSRWGTWGQGTLVTVSS | 185 |

**[Table 25]**

| | VL Sequence | SEQ ID NO. |
|---|---|---|
| P001018v1 | DIVLTQSPASLAVSPGQRATITCRASKSVSTSGYSYMHWYQQKPG QPPKLLIYLASNLESGVPARFSGSGSGTDFTLTINPVEANDTANY YCQHSRELLTFGQGTKVEIK | 155 |
| P001018v2 | DIVLTQSPASLAVSPGQRATITCRASKSVSTSGYSYMHWYQQKPG QPPKLLIYLASNLESGVPARFSGSGSGTDFTLTINPVEANDTANY YCQHSHELLTFGQGTKVEIK | 186 |
| P001018v3 | DIVLTQSPASLAVSPGQRATITCRASKSVSTSGYSYMHWYQQKPG QPPKLLIYLASNLESGVPARFSGSGSGTDFTLTINPVEANDTANY YCQHSRELDTFGQGTKVEIK | 187 |
| P001018v4 | DIVLTQSPASLAVSPGQRATITCRASKSVSTSGYSYMHWYQQKPG QPPKLLIYLASNLESGVPARFSGSGSGTDFTLTINPVEANDTANY YCQHSSELLTFGQGTKVEIK | 188 |
| P001018v5 | DIVLTQSPASLAVSPGQRATITCRASKSVSTSGYSYMHWYQQKPG QPPKLLIYLASNLESGVPARFSGSGSGTDFTLTINPVEANDTANY YCQHSRELLRFGQGTKVEIK | 189 |
| P001018v6 | DIVLTQSPASLAVSPGQRATITCRASKSVSTSGYSYMHWYQQKPG QPPKLLIYLASNLESGVPARFSGSGSGTDFTLTINPVEANDTANY YCQHSRELLTFGQGTKVEIK | 155 |
| P001018v2 3 | DIVLTQSPASLAVSPGQRATITCRASKSVSTSGYSYMHWYQQKPG QPPKLLIYLASNLESGVPARFSGSGSGTDFTLTINPVEANDTANY YCQHSHELDTFGQGTKVEIK | 190 |
| P001018v2 6 | DIVLTQSPASLAVSPGQRATITCRASKSVSTSGYSYMHWYQQKPG QPPKLLIYLASNLESGVPARFSGSGSGTDFTLTINPVEANDTANY YCQHSHELLTFGQGTKVEIK | 186 |
| P001018v3 6 | DIVLTQSPASLAVSPGQRATITCRASKSVSTSGYSYMHWYQQKPG QPPKLLIYLASNLESGVPARFSGSGSGTDFTLTINPVEANDTANY YCQHSRELDTFGQGTKVEIK | 187 |
| P001018v2 35 | DIVLTQSPASLAVSPGQRATITCRASKSVSTSGYSYMHWYQQKPG QPPKLLIYLASNLESGVPARFSGSGSGTDFTLTINPVEANDTANY YCQHSHELDRFGQGTKVEIK | 191 |
| P001018v2 36 | DIVLTQSPASLAVSPGQRATITCRASKSVSTSGYSYMHWYQQKPG QPPKLLIYLASNLESGVPARFSGSGSGTDFTLTINPVEANDTANY YCQHSHELDTFGQGTKVEIK | 190 |
| P001018v2 356 | DIVLTQSPASLAVSPGQRATITCRASKSVSTSGYSYMHWYQQKPG QPPKLLIYLASNLESGVPARFSGSGSGTDFTLTINPVEANDTANY YCQHSHELDRFGQGTKVEIK | 191 |

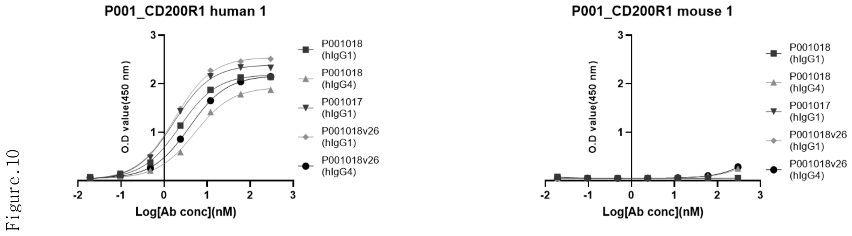

The secondary engineered antibodies were evaluated by ELISA in the same manner as in Examples described above. Finally, a variety of affinity-matured mutants were obtained by antibody sequence analysis. As shown in FIG. 10, the obtained P001018 mutants exhibited a significantly increased specific binding ability to human CD200R1 recombinant protein compared to the parent antibody.

### Example 9: Mammalian cell expression and purification of anti-CD200R1 antibody

Cloning and production processes were performed to produce all clones obtained in Examples described above into monoclonal antibodies in the form of immunoglobulin (IgG). To construct a heavy chain expression vector, DNA encoding the heavy chain, including the heavy chain variable region and constant region, was cloned into the pOptivec vector. To construct a light chain expression vector, DNA encoding the light chain, including the light chain variable region and light chain constant region, was cloned into the pcDNA3.3 vector.

The light and heavy chain expression vectors were used for protein expression and purification using transient transfection. Expi293F suspension cells (Gibco, A14527, Expi293F^{™} Cells) growing in suspension in Expi293 expression medium (Gibco, A1435101, Expi293^{™} Expression Medium) were transfected with a mixture of plasmids, Opti-MEMI (Gibco, 31985070, Opti-MEM^{™} I Reduced Serum Medium), and ExpiFectamine293 (Gibco, 100014995, ExpiFectamine^{™}293). After culturing for 5 days at 8% CO₂, 37°C, and 130 rpm, proteins were purified from the cell culture supernatant. The culture medium was passed through a column (GE healthcare, 17-5438-01, MabSelect^{™} SuRe) to allow the expressed antibody to bind to the column. After elution with IgG elution buffer (Thermo Scientific, 21028, Pierce^{™} IgG Elution Buffer, pH 2.0), the eluted antibody fraction was concentrated using an Amicon Ultra 30kDa tube (Merck Millipore, UFC903024, Amicon^{®} Ultra-15 Centrifugal Filter Unit) by exchanging the buffer with PBS (pH 7.4).

The purified anti-CD200R1 antibody was quantified using absorbance and extinction coefficient at 280 nm.

### Example 10: Human and monkey cross-reactivity ability analysis (ELISA) of CD200RLa isoform, immune-activating CD200R

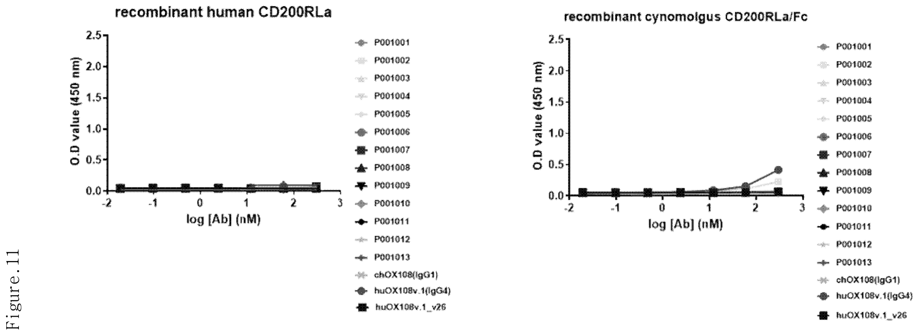

96-well plates were coated with human CD200RLa (Sino Biological, 11620-H08H) and monkey CD200RLa (Sino Biological, 11576-C02H) recombinant proteins at a concentration of 2 µg/mL at 4°C for 16 hours, and blocked with 3% skim milk for 1 hour. The plates were then treated with antibodies at concentrations of 300 nM, 60 nM, 12 nM, 2.4 nM, 0.48 nM, 0.096 nM, and 0.0192 nM, respectively, and incubated at room temperature for 2 hours. The result was washed with PBST and distilled water, and the samples were bound with a goat-derived HRP-conjugated anti-human antibody (Invitrogen, 31482, Goat anti-Human IgG F(ab')2 Secondary Antibody, HRP) for 1 hour at room temperature, and then washed again with PBST and distilled water. Color was developed with a TMB substrate solution (Thermo Scientific, 34029, 1-Step^{™} Ultra TMB-ELISA Substrate Solution). The color reaction was stopped with stop solution (Invitrogen, SS04, ELISA Stop Solution). Absorbance was measured at an optical density of 450 nm and the result showed that the identified clones lacked cross-reactivity ability to human CD200RLa and cyno CD200RLa (FIG. 11).

### Example 11: Analysis of CD200R1 binding domain of anti-CD200R1 antibodies using human-mouse CD200R1 hybrid protein (ELISA)

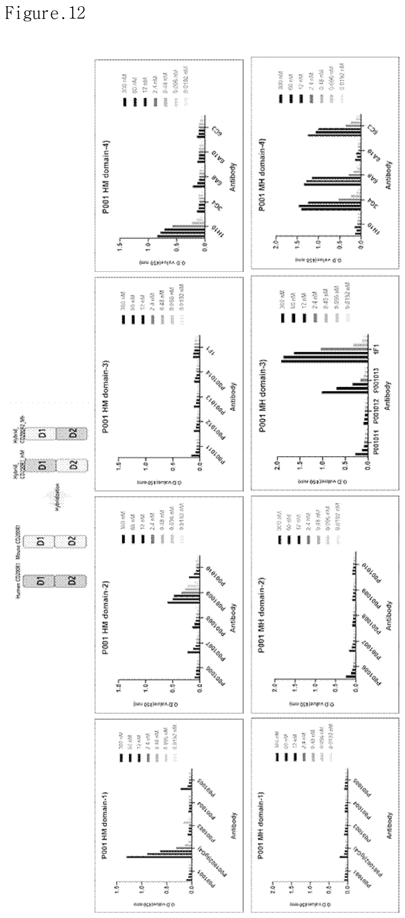

Recombinant proteins were produced from the extracellular Ig-like V-type domain and Ig-like C2-type domain of human CD200R1, each designed as human-mouse (HM hybrid domain) and mouse-human (MH hybrid domain) recombinant proteins. A 96-well plate was coated at a concentration of 1 µg/mL with each protein at 4°C for 16 hours and blocked with 3% skim milk. The result was treated with antibodies at concentrations of 300 nM, 60 nM, 12 nM, 2.4 nM, 0.48 nM, 0.096 nM, and 0.0192 nM, and incubated at room temperature for 2 hours. After washing with PBST and distilled water, the result was bound to a goat-derived HRP-conjugated anti-human antibody (Invitrogen, 31482, Goat anti-Human IgG F(ab')2 Secondary Antibody, HRP) for 1 hour at room temperature, and then washed again with PBST and distilled water. After color development with TMB substrate solution (Thermo Scientific, 34029, 1-Step^{™}Ultra TMB-ELISA Substrate Solution), the color reaction was stopped with a stop solution (Invitrogen, SS04, ELISA Stop Solution), and the absorbance was measured at an O.D. of 450 nm. The anti-CD200R1 antibody clones binding to each domain were analyzed through ELISA and the results are as shown in FIG. 12.

### Example 12: Binding ability of anti-CD200R1 antibodies selected using multifaceted antibody discovery technology to CD200R1-expressing cells

The binding ability of the antibodies identified using unique multifaceted antibody discovery technology was identified in HH (CRL-2105) cells, which endogenously express CD200R1, and Jurkat-CD200R1 cells (Source: DiscoverX), which were generated by intranuclear injection of CD200R1 cDNA.

HH and Jurkat-CD200R1 cells were maintained in RPMI 1640 medium supplemented with 10% heat-inactivated FBS and 1% Penicillin/Streptomycin (P/S). The cells were washed by centrifugation at 130g for 5 minutes. The cells were seeded onto 96-well round-bottom plates (SPL, #34296) at a density of 1x10⁵ cells/well in Flow cytometry staining buffer (Invitrogen, #00-4222-26) or FACS buffer. The cells were treated with all 16 substances and the positive control LY3454738 (US 2020/0087395, I-4P, Eli Lilly) at a concentration of 500 nM in the IgG1 form. After incubation for 1 hour at 4°C, the cells were treated with Alexa-647 Goat anti-human IgG (H+L) cross-adsorbed secondary antibody (Invitrogen, #A21445) and incubated at 4°C for an additional 30 minutes. After washing twice with FACS buffer, flow cytometry analysis was performed using a Novocyte Flow cytometer (Agilent).

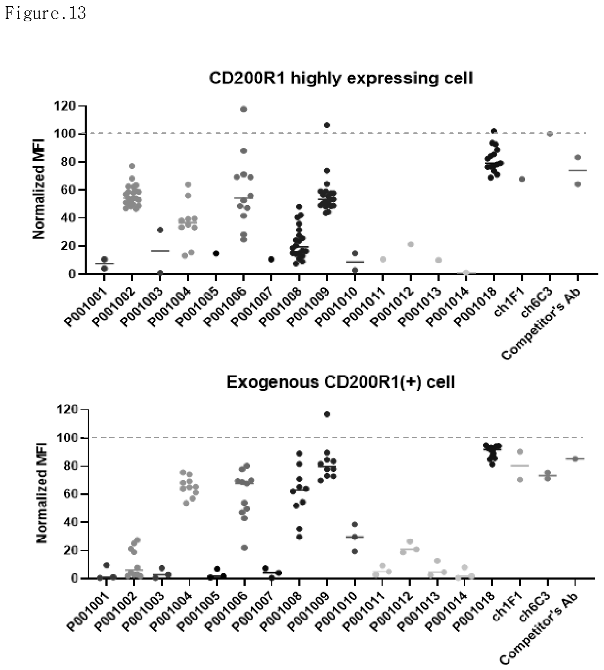

As shown in FIG. 13, mean fluorescence intensity (MFI) was obtained by measuring the fluorescence intensity of each antibody tagged with an Alexa-647 secondary antibody and normalizing the fluorescence expression of each antibody to the fluorescence expression of each antibody based on P001017 (chimeric OX108). In two cell lines, six active substances, P001002, P001004, P001006, P001008, P001009, and P001018, exhibited excellent binding ability of over 50%. Furthermore, among the seven compounds discovered using hybridoma technology, the ch1F1 and ch6C3 clones also exhibited significant binding ability to two cell lines.

### Example 13: Cell-binding ability of anti-CD200R1 antibodies in IgG4 and LALAPG mutants

Cell-binding ability was determined in the same manner as in Example 12. For the six active substances primarily selected in Example 12, the isotype was changed to IgG4 or the LALAPG gene was mutated, and binding activity was determined in the two cell lines mentioned above.

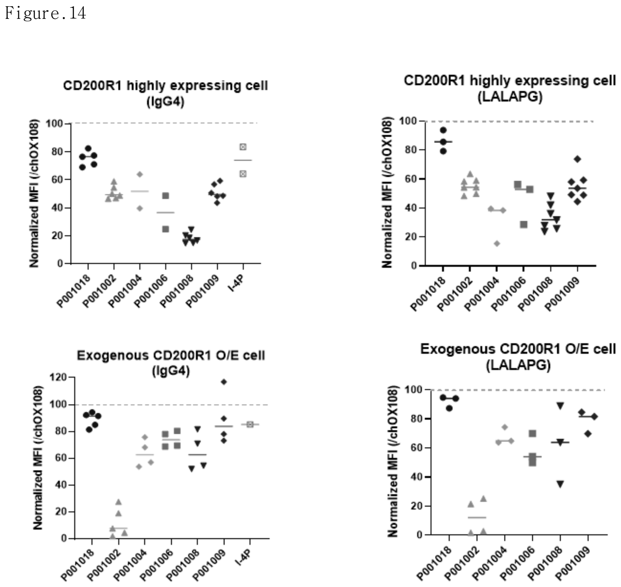

As shown in FIG. 14, with the exception of P001002, the remaining five active substances exhibited excellent binding ability regardless of isotype. P001009, which exhibited the highest binding activity among the antibodies discovered using phage display technology, exhibited the highest efficacy.

### Example 14: Affinity improvement and sequence identification of anti-CD200R1 antibody

Mutation was performed to improve the affinity of the antibody obtained in Example 13, enhance cross-species binding, and remove unwanted PTM sites. Specifically, affinity improvement was performed based on the P001009 antibody sequence, which exhibited excellent efficacy in Example 13. The CDR, VH, and VL sequences of the affinity-improved antibody are shown in Tables 26 to 29.

**[Table 26]**

| | CDR H1 | | CDR H2 | | CDR H3 | |
|---|---|---|---|---|---|---|
| | Sequence | SEQ ID NO. | Sequence | SEQ ID NO. | Sequence | SEQ ID NO. |
| P001009v1 | SYDMS | 7 | WISHGGGSIYYADSVKG | 16 | DAPAYSAPLFDY | 30 |
| P001009v2 | SYDMS | 7 | WISHGGGSIYYADSVKG | 16 | DAPAYSAPLFDY | 30 |
| P001009v3 | SYDMS | 7 | WISHGGGSIYYADSVKG | 16 | DAPAYSAPLFDY | 30 |
| P001009v4 | SYDMS | 7 | WISHGGGSIYYADSVKG | 16 | DAPAYSAPLFDY | 30 |

**[Table 27]**

| | CDR L1 | | CDR L2 | | CDR L3 | |
|---|---|---|---|---|---|---|
| | Sequence | SEQ ID NO. | Sequence | SEQ ID NO. | Sequence | SEQ ID NO. |
| P001009v1 | SGSSSNIGNNAVN | 43 | YDKNRPS | 194 | AAWDDRLSGYV | 195 |
| P001009v2 | SGSSSNIGNNAVN | 43 | YDKNRPS | 194 | AAWDRSLSGYV | 196 |
| P001009v3 | SGSSSNIGSNAVN | 192 | YDSNRPS | 54 | AAWDDSLSGYV | 66 |
| P001009v4 | SGSSSNIGNKAVN | 193 | YDSNRPS | 54 | AAWDDSLSGYV | 66 |

**[Table 28]**

| | VH Sequence | SEQ ID NO. |
|---|---|---|
| P001009v1 | EVQLLESGGGLVQPGGSLRLSCAASGFTFSSYDMSWVRQAPGKGL EWVSWISHGGGSIYYADSVKGRFTISRDNSKNTLYLQMNSLRAED TAVYYCARDAPAYSAPLFDYWGQGTLVTVSS | 79 |
| P001009v2 | EVQLLESGGGLVQPGGSLRLSCAASGFTFSSYDMSWVRQAPGKGL EWVSWISHGGGSIYYADSVKGRFTISRDNSKNTLYLQMNSLRAED TAVYYCARDAPAYSAPLFDYWGQGTLVTVSS | 79 |
| P001009v3 | EVQLLESGGGLVQPGGSLRLSCAASGFTFSSYDMSWVRQAPGKGL EWVSWISHGGGSIYYADSVKGRFTISRDNSKNTLYLQMNSLRAED TAVYYCARDAPAYSAPLFDYWGQGTLVTVSS | 79 |
| P001009v4 | EVQLLESGGGLVQPGGSLRLSCAASGFTFSSYDMSWVRQAPGKGL EWVSWISHGGGSIYYADSVKGRFTISRDNSKNTLYLQMNSLRAED TAVYYCARDAPAYSAPLFDYWGQGTLVTVSS | 79 |

**[Table 29]**

| | VL Sequence | SEQ ID NO. |
|---|---|---|
| P001009v1 | QSVLTQPPSASGTPGQRVTISCSGSSSNIGNNAVNWYQQLPGTAP KLLIYYDKNRPSGVPDRFSGSKSGTSASLAISGLRSEDEADYYCA AWDDRLSGYVFGGGTKLTVL | 197 |
| P001009v2 | QSVLTQPPSASGTPGQRVTISCSGSSSNIGNNAVNWYQQLPGTAP KLLIYYDKNRPSGVPDRFSGSKSGTSASLAISGLRSEDEADYYCA AWDRSLSGYVFGGGTKLTVL | 198 |
| P001009v3 | QSVLTQPPSASGTPGQRVTISCSGSSSNIGSNAVNWYQQLPGTAP KLLIYYDSNRPSGVPDRFSGSKSGTSASLAISGLRSEDEADYYCA AWDDSLSGYVFGGGTKLTVL | 199 |
| P001009v4 | QSVLTQPPSASGTPGQRVTISCSGSSSNIGNKAVNWYQQLPGTAP KLLIYYDSNRPSGVPDRFSGSKSGTSASLAISGLRSEDEADYYCA AWDDSLSGYVFGGGTKLTVL | 200 |

| | | |
|---|---|---|
| * Bold/underline indicates mutation location | | |

First, sequence motifs in the antibody sequence that were at risk of deamidation and isomerization were identified during and after production under stressed conditions. The locations of these motifs, numbered according to Kabat Numbering, are as follows: LC N30 (P019001v3), LC N31 (P019001v4), LC D51, LC S52 (P019001v1, P019001v2), LC D92, LC D93 (P019001v2), and LC S94 (P019001v1).

It is known that substitution of N or D in sequences prone to deamidation and isomerization may significantly impair antibody affinity (Journal of Pharmaceutical Sciences, 105(2), 512-518).

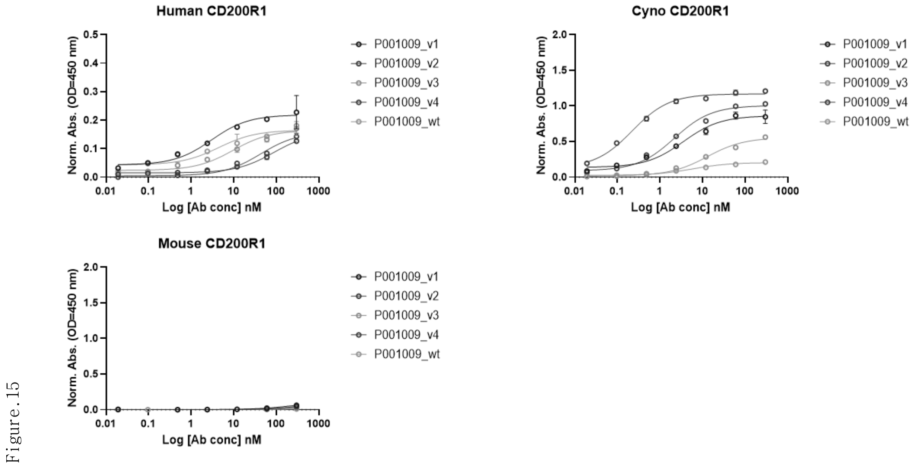

Therefore, the LC D51 and LC D92 sequences were not substituted during the optimization process. However, since LC N30 and LC N31 can play an important role in the specific binding of antibodies as paratopes, the sequences were substituted and optimized. Through rational design, LC S52 and LC S94 were substituted with K and R, respectively, to derive P001009v1, and LC S52 and LC D93 were substituted with K and R, respectively, to derive P001009v2. Further, P001009v3, in which LC N30 was substituted with S, and P001009v4, in which LC N31 was substituted with K, were derived and produced, and the binding ability to human, mouse, and monkey CD200R1 was analyzed by ELISA (FIG. 15). As shown in FIG. 15, the binding ability to human and monkey CD200R1 of P019001v1 was improved compared to the conventional P019001. P019001v2 exhibited significantly improved binding ability to monkey CD200R1 compared to P019001, but reduced binding ability to human CD200R1. P019001v3 and P019001v4 also exhibited improved monkey CD200R1 binding, but reduced human CD200R1 binding. Both P019001 and variants thereof had very weak binding ability to mouse CD200R1 at concentrations up to 300 nM.

### Example 15: Analysis of CD200R1-specific binding ability of anti-CD200R1 antibodies (SPR)

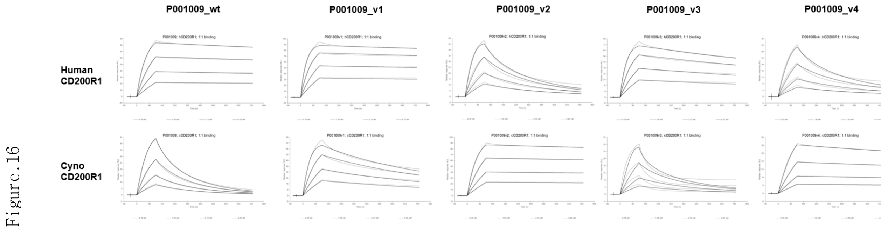

The CD200R1-specific binding ability of the antibodies (P001009_wt, P001009_v1, P001009_v2, P001009_v3, P001009_v4) selected in Example 14 was analyzed using surface plasma resonance (SPR). For the experiment, 30 µg/mL of an anti-histidine antibody was conjugated to a CM5 biacore chip via an amine bond while allowing to flow at 10 µl/min (10,000 RU). Then, 0.25 µg/mL of human or cynomolgus CD200R1 with dual His-tags was captured onto the completed chip for 60 seconds, and the binding ability of each antibody was analyzed. All experiments were performed by association of antibodies for 120 seconds and dissociation of antibodies for 600 seconds. The resulting graphs were fitted using a 1:1 Langmuir binding kinetic model (FIG. 16), and the kinetics of each antibody were calculated. The kinetics of the parent and modified antibodies with human CD200R1 are shown in Table 30 below. The kinetics of the parent and modified antibodies with cynomolgus CD200R1 are shown in Table 31 below.

**[Table 30]**

| Target | Name | Ka (1/Ms) | Kd (1/s) | KD (M) |
|---|---|---|---|---|
| | P001009 WT | 2.81e+6 | 1.34e-4 | 4.75e-11 |
| hCD200R1 -his | P001009v1 | 4.77e+6 | 1.00e-4 | 2.10e-11 |
| | P001009v2 | 7.52e+6 | 6.54e-3 | 8.70e-10 |
| | P001009v3 | 4.16e+6 | 4.52e-4 | 1.09e-10 |
| | P001009v4 | 6.74e+6 | 7.00e-3 | 1.04e-9 |

**[Table 31]**

| Target | Name | Ka (1/Ms) | Kd (1/s) | KD (M) |
|---|---|---|---|---|
| | P001009 WT | 5.63e+6 | 1.17e-2 | 2.08e-9 |
| cCD200R1-his | P001009v1 | 5.23e+6 | 1.38e-3 | 2.64e-10 |
| | P001009v2 | 2.82e+6 | 8.08e-5 | 2.86e-11 |
| | P001009v3 | 6.14e+9 | 7.09e0 | 1.15e-9 |
| | P001009v4 | 3.71e+6 | 3.05e-4 | 8.22e-11 |

### Example 16: Specific binding ability of anti-CD200R1 antibodies to cell surface-expressed CD200R1

Although the antigen binding ability of the anti-CD200R1 antibodies selected in examples described above was determined, it was necessary to determine whether or not the anti-CD200R1 antibodies exhibited superior binding ability to cell lines with high expression of CD200R1. Binding ability was determined in HH, an endogenous T cell line with high CD200R1 expression, and two additional overexpressing cell lines: U937-CD200R overexpressing cells, generated by intranuclear injection of human CD200R1 cDNA into the monocytic cell line U937 (CRL-1593.2) (Source: UMC Utrecht, the Netherlands) and Jurkat-CD200R overexpressing cells, were also cells engineered using a similar method.

All cell lines were maintained in RPMI 1640 medium supplemented with 10% heat-inactivated FBS and 1% penicillin/streptomycin (P/S) and cell binding was determined in the same manner as in Example 12. Two additional wells were treated with 10 µl of mouse IgG1K (BD, #555751) and 5 µl of commercial CD200R1 antibody (Biolegend, #329308) to determine CD200R1 expression on the cell line surface for cell quality control.

Specific binding ability was determined using P001009 WT and antibody-optimized mutants, P001009v1, P001009v2, P001009v3, and P001009v4. The antibodies were serially diluted 10-fold from a maximum concentration of 100 nM to 10 nM, 1 nM, 100 pM, 10 pM, and 1 pM, and were added to the pre-seeded cells. After reaction for 1 hour at 4°C, the cells were treated with Alexa-647 goat anti-human IgG (H+L) cross-adsorbed secondary antibody (Invitrogen, #A21445) and incubated for an additional 30 minutes at 4°C. After washing twice with FACS buffer, flow cytometry was performed using a Novocyte Flow cytometer (Agilent). Using GraphPad Prism software, the EC₅₀ was determined using a nonlinear graph model of log(agonist) vs. response - variable slope, as shown in Table 32.

**[Table 32]**

| mAb | Cell binding affinity EC50 (nM) | | |
|---|---|---|---|
| | Jurkat-CD200R cells | U937-CD200R cells | HH cells |
| Positive control | 43.48 ± 0.97 | 152.10 ± 19.12 | 82.73 ± 2.01 |
| P001009 WT | 0.22 ± 0.03 | 0.41 ± 0.06 | 0.19 ± 0.05 |
| P001009v1 | 0.17 ± 0.03 | 0.30 ± 0.07 | 0.16 ± 0.06 |
| P001009v2 | 3.08 ± 0.32 | - | - |
| P001009v3 | 0.33 ± 0.05 | - | - |
| P001009v4 | 2.37 ± 0.08 | - | - |

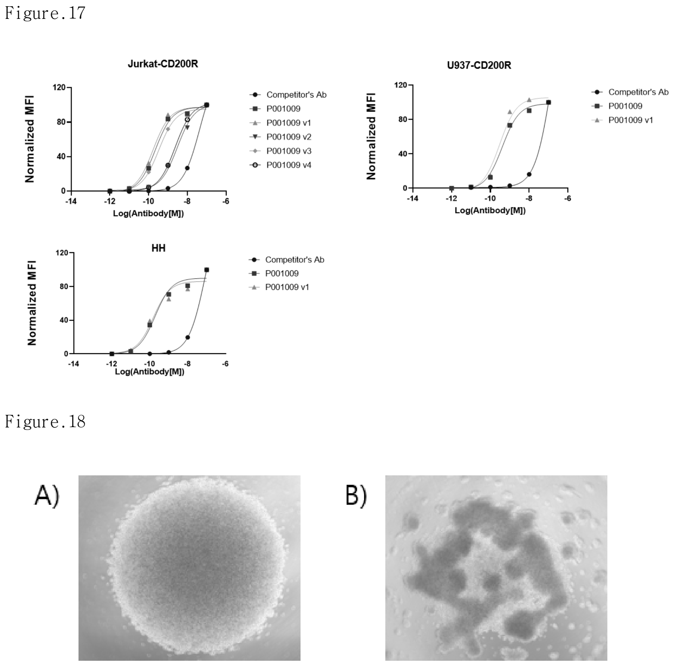

P001009v1 exhibited the highest binding ability to Jurkat-CD200R1. Further, only P001009v1 exhibited binding ability to U937-CD200R1 and HH cell lines. All candidates exhibited excellent binding ability of 10⁻¹⁰ M to receptors expressed on various cells (FIG. 17).

### Example 17: Confirmation of anti-inflammatory effect of anti-CD200R1 antibodies

To determine the binding ability of the candidates to U937-CD200R1-overexpressing cell lines and verify the efficacy thereof, the anti-inflammatory effect of anti-CD200R1 antibodies was analyzed. First, 5 x 10⁶ U937-CD200R1 cells were added to 12 ml of medium, treated with 30 ng/ml of Phorbol 12-myristate 13-acetate (Sigma Aldrich, #P1585), and reacted at 37°C in 5% CO2 for 24 hours. After reaction, the medium was replaced with RPMI 1640 medium containing only 10% heat-inactivated FBS, and 5 x 10⁶ cells were maintained at 37°C in 5% CO₂ for 24 hours.

Cells were seeded on duplicate onto 96-well round-bottom plates (SPL, #34296) at 1 x 10⁵ cells/50 µl /well. P001009 WT, P001009v1, and the previously published CD200R antibody LY3454738 (US 2020/0087395, I-4P, Eli Lilly) as a positive control were used in this experiment. The drug was serially diluted 10-fold from a maximum concentration of 100 nM and treated to the cells at concentrations of 10 nM, 1 nM, 100 pM, 10 pM, and 1 pM, respectively. Additional cells were treated with 100 nM of hIgG4 kappa isotype control (Crownvivo, #C0004) as a negative control. Untreated cells were seeded in the same amount of medium (RPMI 1640 containing 10% heat-inactivated FBS) and incubated at 37°C, 5% CO₂ for 1 hour. To induce an inflammatory response in drug-treated cells, each well was treated with 10 µl of lipopolysaccharide from Salmonella typhosa (Sigma Aldrich, #L7895) at a concentration of 1 µg/ml. One well was left untreated to assess the induction of inflammatory cytokines. After thorough mixing, the cells were incubated at 37°C and 5% CO2 for 24 hours.

After centrifugation at 1100 g for 2 minutes, images of the pellets before and after LPS treatment were captured using a microscope (Carl Zeiss, AXIO VERT.A1). After LPS treatment, the pellet appeared as scattered debris (FIG. 18B) rather than uniform spheroids (FIG. 18A). This was observed under a microscope. The supernatant was collected and analyzed for inflammatory cytokines such as TNF-α and IL-6 using an EnVision^{®} multimode plate reader (PerkinElmer). A bead-based cytokine assay was performed using the AlphaLISA immunoassay on the EnVision^{®} instrument. TNF-α (human) AlphaLISA Detection Kit, 500 assay points (PerkinElmer, #AL208C) was used for TNF-α, and the IL-6 (human) AlphaLISA Detection Kit, 500 assay points (PerkinElmer, #AL223C) was used for IL-6. Cytokine levels were determined depending on the concentration of each antibody.

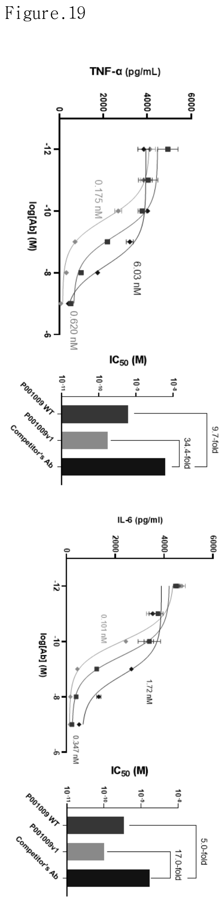

Table 33 shows the results of the TNF-α inhibitory activity of three drugs, and Table 34 shows the results of the IL-6 inhibitory activity (FIG. 19). The IC₅₀ calculated by measuring cytokine levels at different drug concentrations are shown in Table 35.

**[Table 33]**

| log [Ab] (M) | TNF-α (pg/ml) | | | |
|---|---|---|---|---|
| | hIgG4 | P001009 WT | P001009v1 | Positive control |
| -7 | 5086.7±150. 4 | 619.6+12.3 | 14 6. 7±23.8 | 472.4±26.9 |
| -8 | - | 1259.1±29.9 | 609.5±25.6 | 1043.1±54.2 |
| -9 | - | 2035.8±108.9 | 1039.8±54.7 | 1480.6±23.5 |
| -10 | - | 4148.8±208.7 | 3352.5±189.8 | 3758.8±205.7 |
| -11 | - | 5038.4±7.7 | 4753.2±96.1 | 4945.9±380.0 |
| -12 | - | 5128.6±667.6 | 4082.1±964.6 | 5486.5±222.3 |

**[Table 34]**

| log [Ab] (M) | IL-6 (pg/ml) | | | |
|---|---|---|---|---|
| | hIgG4 | P001009 WT | P001009v1 | Positive control |
| -7 | 3509.7±153. 9 | 231.7±24.1 | 153.7±8.8 | 523.1±6.4 |
| -8 | - | 401.5±9.6 | 165.1±8.4 | 1332±59.1 |
| -9 | - | 1245.6±49.9 | 454.2±45 | 2668.1±57.4 |
| -10 | - | 3398.1±325.5 | 2439±56.8 | 3379.2±134.9 |
| -11 | - | 3751.0±144.5 | 3517.7±166.3 | 3545.8±109.7 |
| -12 | - | 4512.7±74.1 | 4730.2±99.8 | 4526.4±95.9 |

**[Table 35]**

| MAb | IC50 (nM) | |
|---|---|---|
| | TNF-α | IL-6 |
| Positive control | 6.03 | 1.72 |
| P001009 WT | 0.62 | 0.35 |
| P001009v1 | 0.18 | 0.10 |

When treated with the negative control, hIgG4, at the highest concentration, TNF-α and IL-6 levels were high, reaching 5 ng/ml and 3.5 ng/ml, respectively. However, these levels were suppressed when treated with the corresponding drugs. Among them, P001009v1 maintained low inflammatory cytokine levels even at low nanomolar (nM) drug concentrations, indicating that the antibody of the present invention exhibits an excellent anti-inflammatory effect.

### Example 18: Confirmation of Dok2 protein phosphorylation effect of anti-CD200R1 antibody

The Dok2 adaptor protein was phosphorylated by the CD200-CD200R1 interaction to inhibit mast cell degranulation and inflammatory cytokine secretion through downstream signaling. The Dok2 protein gene, like CD200R1, was found to have a high heritability in the genomes of atopic dermatitis patients (Mucha et al., 2020). Based on this, a drug evaluation platform was established.

Jurkat-CD200R1 cells were maintained as shown in Example 17 and were then seeded in triplicate onto CulturePlate-384, a White Opaque 384-well microplate (PerkinElmer, #6007688) at a density of 8,000 cells/well (32 µl). AssayComplete Cell Plating 0 Reagent (DiscoverX, #93-0563R0A) was used for cell plating and antibody dilution. The seeded cells were incubated at 37°C and 5% CO₂ for 30 minutes.

During the reaction, an Fc capture antibody, anti-human IgG (Fc-specific) antibody produced in goat (Sigma Aldrich, #I2136, 2.3 mg/ml), was diluted in AssayComplete media and incubated at 37°C, 5% CO₂ for 30 minutes. After incubation of both the plate containing the cells and the Fc capture antibody, the cells were treated with the Fc capture antibody at a concentration of 50 µg/ml per well. The reaction was incubated for 3 hours at 37°C, 5% CO2. After cross-reactivity with the Fc capture antibody, the cells were treated with a total of three antibodies, as shown in Example 17. The cells were diluted two-fold from a maximum concentration of 50 nM to 25 nM, 12.5 nM, 6.25 nM, 3.12 nM, 1.56 nM, 0.78 nM, 0.39 nM, 0.20 nM, 0.098 nM, and 0.049 nM. After treatment, the result was reacted at room temperature for an additional 2 hours.

The PathHunter Detection kit (DiscoverX, #93-0001) was used to confirm the response of the treated antibodies to the Jurkat-CD200R1 reporter cell line. Final data were analyzed using an Infinite M200 Pro Plate reader (Tecan) to measure the luminescence from phosphorylated Dok2. Random luminescence unit (RLU) is an arbitrary unit representing luminescence, calculated by converting the signal detected by a cross-linked Fc capture antibody. Table 36 shows RLU for each drug treatment concentration, and Table 37 shows the EC50 representing the degree of signal transduction by the Dok2 protein.

**[Table 36]**

| Antibody dose (nM) | RLU (a.u.) | | |
|---|---|---|---|
| | P001009 WT | P001009v1 | Positive control |
| 50 | 37120 ± 14299 | 32676 ± 5379 | 25594 ± 3521 |
| 25 | 50858 ± 8029 | 53185± 13281 | 22714 ± 3610 |
| 12.5 | 57733 ± 6385 | 45830 ± 6990 | 12950 ± 1492 |
| 6.25 | 56115 ± 6866 | 48879 ± 1315 | 8932 ± 683 |
| 3.13 | 41203 ± 4810 | 29729 ± 3825 | 9472 ± 1492 |
| 1.56 | 33140 ± 5780 | 32863 ± 4207 | 9100 ± 1827 |
| 0.78 | 25264 ± 1484 | 28644 ± 4755 | 8902 ± 399 |
| 0.39 | 10861 ± 917 | 11340 ± 509 | 8964 ± 426 |
| 0.20 | 15184 ± 2826 | 17866 ± 1925 | 8002 ± 451 |
| 0.10 | 10359 ± 1227 | 10677 ± 2258 | 8399 ± 421 |
| 0.05 | 10432 ± 2166 | 9521 ± 1128 | 9258 ± 2086 |

**[Table 37]**

| mAb | EC50 (nM) |
|---|---|
| Positive control | > 10⁴ |
| P001009 WT | 1.57 |
| P001009v1 | 1.28 |

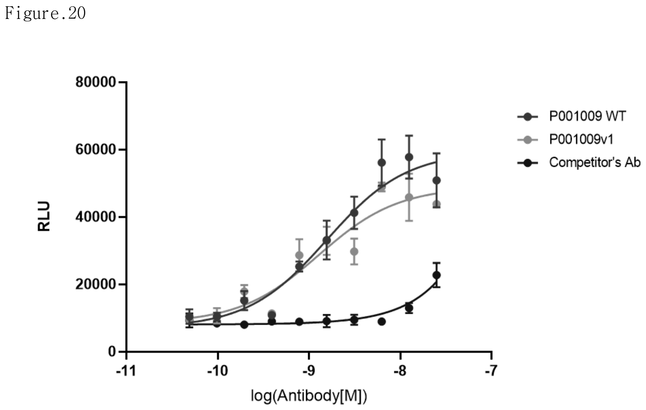

As shown in FIG. 20, the positive control group had no reactivity, but the P001009 WT and P001009v1 antibodies exhibited luminescence due to phosphoDok2, indicating downstream immune signaling is efficiently activated.

### Example 19: Confirmation of Type 1 and Type 2 inflammatory cytokine inhibition effect of anti-CD200R1 antibody in nasal polyp patient specimens

Specimens from patients with chronic rhinosinusitis with nasal polyps were treated with a drug to determine the efficacy of anti-CD200R1.

The specimens were finely chopped and minced, and then additionally processed twice using the *m_lung_02* protocol on a gentleMACS (Miltenyi Biotec) to obtain tissue fragments. After centrifugation at 1500 rpm for 3 minutes to ensure that all tissues formed a pellet, RPMI media + 10% FBS was aliquoted in the pellet at a 1:3 ratio. The pellet was thoroughly mixed with the medium and dispensed into a 96-well u-bottom plate, 100 µl per well, in triplicate for each drug. To establish the screening assay, results were obtained first using dexamethasone (a steroid-based positive control drug), followed by treatment with the anti-CD200R1 antibody (P001009v1) and other drugs used for the corresponding disease. All antibody-based drugs were treated at 100 nM, while steroid drugs such as dexamethasone were treated at 20 µM.

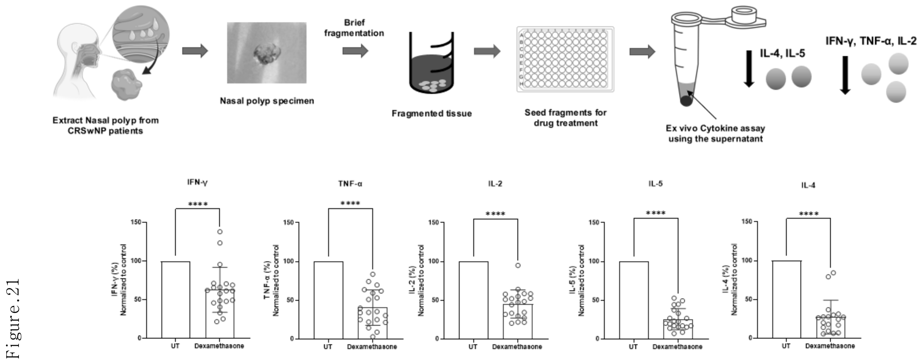

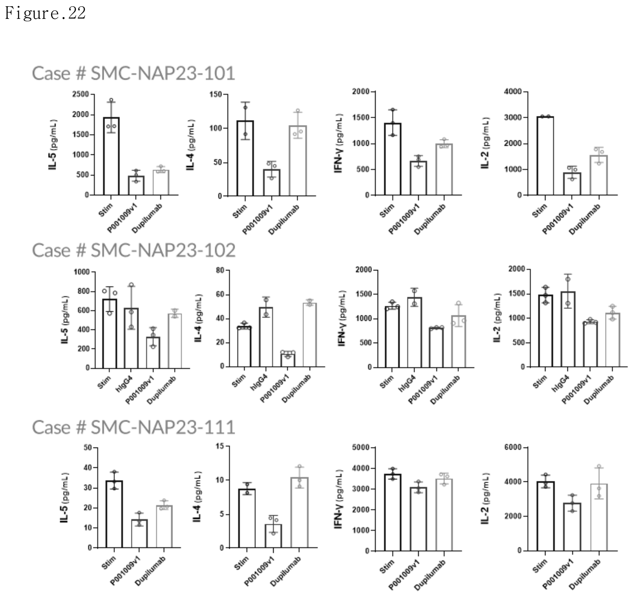

After drug treatment, the cells were incubated at 37°C for 1 hour. To increase overall cytokine levels, 4 µl of a 10-fold diluted cell stimulation cocktail 500X (Invitrogen #00-4970-03) was added on each well and mixed. After reaction for 24 hours at 37°C, the supernatant was collected by centrifugation. Using the human Th1/Th2 cytokine cytometric bead array (CBA) kit (BD, #550749), cytokine levels in the supernatant were measured and the degree of drug responsiveness of each sample was determined. Among the 10 patient samples tested, three exhibited resistance to DUPIXENT^{®}, a drug used as a conventional therapeutic agent for atopic dermatitis and allergic diseases, **showing no suppression** of Th2 cytokine levels (IL-5, IL-4) associated with allergic reactivity. In the same sample, the anti-CD200R1 antibody of the present invention exhibited excellent immunosuppressive activity (FIGS. 21 and 22).

### Example 20: In vivo confirmation of Inhibition of Inflammation of anti-CD200R1 antibody

An *in vivo* multiple sclerosis animal model was constructed and the anti-inflammatory response of the anti-CD200R1 antibody of the present invention (P0001009v1) was determined. The multiple sclerosis animal model was constructed using 10-week-old female C57BL/6J mice and the Hooke kit (EK-2110, Hooke Laboratories, USA). The MOG35-55/CFA emulsion from the Hooke kit was injected subcutaneously into the back and neck of the experimental animals at a volume of 100 µL. Two hours later, pertussis toxin (PTX) was primarily administered intraperitoneally, and 24 hours later, pertussis toxin (PTX) was intraperitoneally administered again thereto. Mice were weighed once daily and scored according to symptoms of multiple sclerosis. Scoring was as follows:
0: Asymptomatic;
0.5: Paralysis of the tip of the tail;
1.0: Paralysis of the entire tail;
1.5: When the mouse was loaded on a stainless steel cage top to cover the cage and was allowed to walk, one hind leg intermittently dropped;
2.0: When the mouse was lifted upside down by the tail, the hind leg did not fully extend outward;
2.5: When the mouse walked on the stainless steel top, the hind leg continued to drop, but the mouse could barely pull the leg up;
3.0: When the mouse walked on the stainless steel top, the mouse could not pull the hind leg up, and trembled the foot;
3.5: Complete paralysis of hind legs, complete immobility;
4.0: Forelimbs weakened, unable to grasp the stainless steel top;
4.5: Death or severe weight loss (30%) inevitably causes euthanasia.

To determine these scores, the animals were placed on a wire mesh screen or were picked up by the tail in an inverted position to assess symptoms. Administration of the anti-CD200R1 antibody was initiated when at least 50% of the total animals exhibited symptoms. The control group received PBS, while the treatment groups received 5 mg/kg and 30 mg/kg intravenously, twice weekly for a total of four doses. Statistical analysis was performed after the experiment using a two-way ANOVA with repeated measures.

The anti-CD200R1 antibody-treated group maintained a score of approximately 1.0 after repeated administration, showing no progression of multiple sclerosis symptoms, whereas the negative control group maintained an average score of 2.5 or higher, confirming the therapeutic effect of the antibody against multiple sclerosis.

### Example 21: Confirmation of anti-CD200R1 antibody inhibition of inflammation in in vivo contact dermatitis animal model

An *in vivo* contact dermatitis animal model was established to evaluate the anti-inflammatory efficacy of the anti-CD200R1 antibody. Ten-week-old female C57BL/6 mice were used for the contact dermatitis model. The animals were anesthetized and the abdominal hair thereof was removed. The following day, the shaved abdominal area was sensitized with 100 µL of 3% oxazolone in ethanol. Seven days after sensitization, P001009v1 was administered intravenously at a dose of 20 mg/kg. Four hours later, the thickness of both ears was measured. Then, both ears were sensitized with 2% oxazolone in 10 µL of ethanol. The thickness of both ears was measured again at 24 and 48 hours, and changes in ear edema thickness were recorded. Statistical analysis was performed using one-way ANOVA.

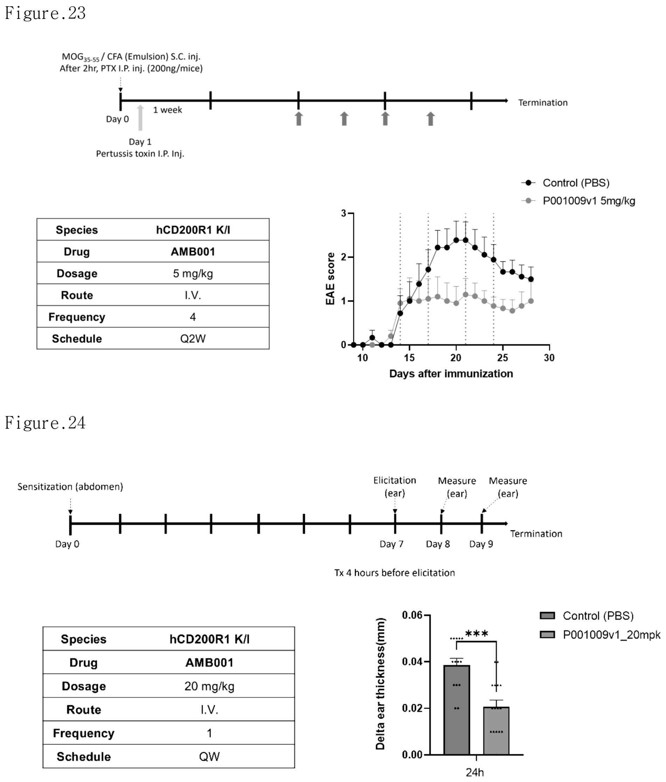

As shown in FIG. 24, antibody P001009v1 of the present invention exhibited a significant anti-inflammatory response compared to the control group. This efficacy behavior was maintained even after 48 hours.

### [Industrial Applicability]

The anti-CD200R1 antibody or antigen-binding fragment thereof of the present invention exhibits superior binding affinity to CD200R1 while non-competitively binding to human CD200 compared to conventional anti-CD200R1 antibodies. In particular, the anti-CD200R1 antibody or antigen-binding fragment thereof of the present invention exhibits excellent immunosuppressive and anti-inflammatory effects and thus is useful for the prevention or treatment of immune or inflammatory diseases.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

### [Sequence Listing Free Text]

An electronic file is attached.

## Claims

1. An antibody specifically binding to CD200R1 or an antigen-binding fragment thereof comprising:
a heavy chain CDR1 including at least one amino acid sequence selected from the group consisting of SEQ ID NOs: 1 to 8, SEQ ID NOs: 100 to 103, and SEQ ID NO: 158;
a heavy chain CDR2 including at least one amino acid sequence selected from the group consisting of SEQ ID NOs: 9 to 21, SEQ ID NOs: 104 to 109, and SEQ ID NO: 159;
a heavy chain CDR3 including at least one amino acid sequence selected from the group consisting of SEQ ID NOs: 22 to 35, SEQ ID NOs: 110 to 115, SEQ ID NO: 160, and SEQ ID NOs: 172 and 173;
a light chain CDR1 including at least one amino acid sequence selected from the group consisting of SEQ ID NOs: 36 to 47, SEQ ID NOs: 116 to 122, SEQ ID NO: 161, SEQ ID NO: 192, and SEQ ID NO: 193;
a light chain CDR2 including at least one amino acid sequence selected from the group consisting of SEQ ID NOs: 48 to 58, SEQ ID NOs: 123 to 127, SEQ ID NO: 162, and SEQ ID NO: 194; and
a light chain CDR3 including at least one amino acid sequence selected from the group consisting of SEQ ID NOs: 59 to 70, SEQ ID NOs: 128 to 134, SEQ ID NO: 163, SEQ ID NOs: 174 to 183, SEQ ID NO: 195, and SEQ ID NO: 196.

2. The antibody or antigen-binding fragment thereof according to claim 1, wherein the antibody or antigen-binding fragment thereof comprises:
a heavy chain variable region including at least one amino acid sequence selected from the group consisting of SEQ ID NOs: 71 to 85, SEQ ID NOs: 135 to 141, SEQ ID NOs: 149 to 153, SEQ ID NO: 184, and SEQ ID NO: 185.

3. The antibody or antigen-binding fragment thereof according to claim 1, wherein the antibody or antigen-binding fragment thereof comprises:
a light chain variable region comprising at least one amino acid sequence selected from the group consisting of SEQ ID NOs: 86 to 99, SEQ ID NOs: 142 to 148, SEQ ID NOs: 154 to 157, SEQ ID NOs: 186 to 191, and SEQ ID NOs: 197 to 200.

4. The antibody or antigen-binding fragment thereof according to claim 1, wherein the antibody or antigen-binding fragment thereof comprises a heavy chain variable region and a light chain variable region selected from the group consisting of:
- a heavy chain variable region of SEQ ID NO: 71 and a light chain variable region of SEQ ID NO: 86;
- a heavy chain variable region of SEQ ID NO: 72 and a light chain variable region of SEQ ID NO: 87;
- a heavy chain variable region of SEQ ID NO: 73 and a light chain variable region of SEQ ID NO: 88;
- a heavy chain variable region of SEQ ID NO: 74 and a light chain variable region of SEQ ID NO: 89;
- a heavy chain variable region of SEQ ID NO: 75 and a light chain variable region of SEQ ID NO: 90;
- a heavy chain variable region of SEQ ID NO: 76 and a light chain variable region of SEQ ID NO: 91;
- a heavy chain variable region of SEQ ID NO: 77 and a light chain variable region of SEQ ID NO: 92;
- a heavy chain variable region of SEQ ID NO: 78 and a light chain variable region of SEQ ID NO: 93;
- a heavy chain variable region of SEQ ID NO: 79 and a light chain variable region of SEQ ID NO: 94;
- a heavy chain variable region of SEQ ID NO: 80 and a light chain variable region of SEQ ID NO: 95;
- a heavy chain variable region of SEQ ID NO: 81 and a light chain variable region of SEQ ID NO: 96;
- a heavy chain variable region of SEQ ID NO: 82 and a light chain variable region of SEQ ID NO: 97;
- a heavy chain variable region of SEQ ID NO: 83 and a light chain variable region of SEQ ID NO: 98;
- a heavy chain variable region of SEQ ID NO: 84 and a light chain variable region of SEQ ID NO: 94;
- a heavy chain variable region of SEQ ID NO: 85 and a light chain variable region of SEQ ID NO: 99;
- a heavy chain variable region of SEQ ID NO: 135 and a light chain variable region of SEQ ID NO: 142;
- a heavy chain variable region of SEQ ID NO: 136 and a light chain variable region of SEQ ID NO: 143;
- a heavy chain variable region of SEQ ID NO: 137 and a light chain variable region of SEQ ID NO: 144;
- a heavy chain variable region of SEQ ID NO: 138 and a light chain variable region of SEQ ID NO: 145;
- a heavy chain variable region of SEQ ID NO: 139 and a light chain variable region of SEQ ID NO: 146;
- a heavy chain variable region of SEQ ID NO: 140 and a light chain variable region of SEQ ID NO: 147;
- a heavy chain variable region of SEQ ID NO: 141 and a light chain variable region of SEQ ID NO: 148;
- a heavy chain variable region of SEQ ID NO: 150 and a light chain variable region of SEQ ID NO: 155;
- a heavy chain variable region of SEQ ID NO: 151 and a light chain variable region of SEQ ID NO: 155;
- a heavy chain variable region of SEQ ID NO: 152 and a light chain variable region of SEQ ID NO: 157;
- a heavy chain variable region of SEQ ID NO: 153 and a light chain variable region of SEQ ID NO: 157;
- a heavy chain variable region of SEQ ID NO: 151 and a light chain variable region of SEQ ID NO: 157;
- a heavy chain variable region of SEQ ID NO: 152 and a light chain variable region of SEQ ID NO: 155;
- a heavy chain variable region of SEQ ID NO: 184 and a light chain variable region of SEQ ID NO: 155;
- a heavy chain variable region of SEQ ID NO: 150 and a light chain variable region of SEQ ID NO: 186;
- a heavy chain variable region of SEQ ID NO: 150 and a light chain variable region of SEQ ID NO: 187;
- a heavy chain variable region of SEQ ID NO: 150 and a light chain variable region of SEQ ID NO: 188;
- a heavy chain variable region of SEQ ID NO: 150 and a light chain variable region of SEQ ID NO: 189;
- a heavy chain variable region of SEQ ID NO: 185 and a light chain variable region of SEQ ID NO: 155;
- a heavy chain variable region of SEQ ID NO: 150 and a light chain variable region of SEQ ID NO: 190;
- a heavy chain variable region of SEQ ID NO: 185 and a light chain variable region of SEQ ID NO: 186;
- a heavy chain variable region of SEQ ID NO: 185 and a light chain variable region of SEQ ID NO: 187;
- a heavy chain variable region of SEQ ID NO: 150 and a light chain variable region of SEQ ID NO: 191;
- a heavy chain variable region of SEQ ID NO: 185 and a light chain variable region of SEQ ID NO: 190;
- a heavy chain variable region of SEQ ID NO: 185 and a light chain variable region of SEQ ID NO: 191;
- a heavy chain variable region of SEQ ID NO: 79 and a light chain variable region of SEQ ID NO: 197;
- a heavy chain variable region of SEQ ID NO: 79 and a light chain variable region of SEQ ID NO: 198;
- a heavy chain variable region of SEQ ID NO: 79 and a light chain variable region of SEQ ID NO: 199; or
- a heavy chain variable region of SEQ ID NO: 79 and a light chain variable region of SEQ ID NO: 200.

5. The antibody or antigen-binding fragment thereof according to claim 1, wherein the antibody or antigen-binding fragment thereof comprises a single-chain Fv (scFv), a single-chain antibody, Fab, F(ab'), or a disulfide-linked Fv (sdFv).

6. The antibody or antigen-binding fragment thereof according to claim 5, wherein the scFv comprises a heavy chain variable region and a light chain variable region linked to each other via a linker,
the heavy chain variable region comprising at least one amino acid sequence selected from the group consisting of SEQ ID NOs: 71 to 85, SEQ ID NOs: 135 to 141, SEQ ID NOs: 149 to 153, SEQ ID NO: 184, and SEQ ID NO: 185, and the light chain variable region comprising at least one amino acid sequence selected from the group consisting of SEQ ID NOs: 86 to 99, SEQ ID NOs: 142 to 148, SEQ ID NOs: 154 to 157, SEQ ID NOs: 186 to 191, and SEQ ID NOs: 197 to 200.

7. A nucleic acid encoding the antibody or antigen-binding fragment thereof according to any one of claims 1 to 6.

8. A recombinant expression vector comprising the nucleic acid according to claim 7.

9. A host cell transfected with the recombinant expression vector according to claim 8.

10. A method of producing an antibody specifically binding to CD200R1 or an antigen-binding fragment thereof, the method comprising:
culturing the host cell according to claim 9 to produce an antibody; and
isolating and purifying the produced antibody.

11. A bispecific/multispecific antibody comprising the antibody or antigen-binding fragment thereof according to any one of claims 1 to 6.

12. A chimeric antigen receptor (CAR) comprising:
an extracellular domain including the antigen-binding fragment according to any one of claims 1 to 6;
a transmembrane domain; and
an intracellular signaling domain.

13. An immune cell comprising the chimeric antigen receptor (CAR) according to claim 12.

14. The immune cell according to claim 13, wherein the immune cell is a regulatory T cell.

15. A composition for use in preventing or treating an immune or inflammatory disease comprising the antibody or antigen-binding fragment thereof according to any one of claims 1 to 6, the bispecific or multispecific antibody comprising the antibody or antigen-binding fragment thereof according to any one of claims 1 to 6, the chimeric antigen receptor comprising the antibody or antigen-binding fragment thereof according to any one of claims 1 to 6, and/or the immune cell comprising the chimeric antigen receptor.
